# EUROPEAN PATENT APPLICATION

(11) **EP 2 471 450 A1**
(43) Date of publication of application: **04.07.2012**
(21) Application number: 12159219.0
(22) Date of filing: 13.10.2006
(51) Int. Cl.: A61B 5/0428, H04B 13/00, A61N 1/375, A61N 1/362, A61N 1/05, A61N 1/365, A61N 1/368, A61N 1/39, A61N 1/372

(54) **Leadless cardiac pacemaker and system**

(30) Priority: 14.10.2005 US 726706 P; 24.10.2005 US 729671 P; 16.11.2005 US 737296 P; 26.11.2005 US 739901 P; 10.12.2005 US 749017 P; 24.01.2006 US 761531 P; 24.01.2006 US 761740 P
(62) Divisional of application: 06836350.6
(71) Applicant: Nanostim, Inc., Sunnyvale, CA 94085 (US)
(72) Inventor: Jacobson, Peter, M., Chanhassen, MN Minnesota 55317 (US)
(74) Representative: Zijlstra, Robert Wiebo Johan

(57) **Abstract**

In a cardiac pacing system, a leadless cardiac pacemaker is configured for implantation in electrical contact with a cardiac chamber and configured for leadless pacing.

## Description

### BACKGROUND

Cardiac pacing electrically stimulates the heart when the heart's natural pacemaker and/or conduction system fails to provide synchronized atrial and ventricular contractions at appropriate rates and intervals for a patient's needs. Such bradycardia pacing provides relief from symptoms and even life support for hundreds of thousands of patients. Cardiac pacing may also give electrical overdrive stimulation intended to suppress or convert tachyarrhythmias, again supplying relief from symptoms and preventing or terminating arrhythmias that could lead to sudden cardiac death.

Cardiac pacing is usually performed by a pulse generator implanted subcutaneously or sub-muscularly in or near a patient's pectoral region. The generator usually connects to the proximal end of one or more implanted leads, the distal end of which contains one or more electrodes for positioning adjacent to the inside or outside wall of a cardiac chamber. The leads have an insulated electrical conductor or conductors for connecting the pulse generator to electrodes in the heart. Such electrode leads typically have lengths of 50 to 70 centimeters.

A conventional pulse generator may be connected to more than one electrode-lead. For example, atrio-ventricular pacing, also commonly called dual-chamber pacing, involves a single pulse generator connected to one electrode-lead usually placed in the right atrium and a second electrode-lead usually placed in the right ventricle. Such a system can electrically sense heartbeat signals and deliver pacing pulses separately in each chamber. In typical use, the dual-chamber pacing system paces the atrium if no atrial heartbeat is sensed since a predetermined time, and then paces the ventricle if no ventricular heartbeat is sensed within a predetermined time after the natural or paced atrial beat. Such pulse generators can also alter the timing of atrial and ventricular pacing pulses when sensing a ventricular beat that is not preceded by an atrial beat within a predetermined time; that is, a ventricular ectopic beat or premature ventricular contraction. Consequently, dual-chamber pacing involves pacing and sensing in an atrium and a ventricle, and internal communication element so that an event in either chamber can affect timing of pacing pulses in the other chamber.

Recently, left-ventricular cardiac pacing has been practiced to ameliorate heart failure; a practice termed cardiac resynchronization therapy (CRT). CRT has been practiced with electrode-leads and a pulse generator, either an implantable cardioverter-defibrillator (CRT-D) or an otherwise conventional pacemaker (CRT-P). The left-ventricular pacing conventionally uses an electrode in contact with cardiac muscle in that chamber. The corresponding electrode-lead is usually placed endocardially in a transvenous manner through the coronary sinus vein, or epicardially. Left-ventricular pacing is usually practiced together with right-atrial and right-ventricular pacing with a single implanted pulse generator connected to three electrode-leads. CRT pulse generators can independently vary the time between an atrial event and right-ventricular pacing, and the time between an atrial event and left-ventricular pacing, so that the left ventricular pacing pulse can precede, follow, or occur at the same time as the right-ventricular pacing pulse. Similarly to dual-chamber pacing, systems with left-ventricular pacing also change atrial and ventricular pacing timing in response to premature ventricular contractions. Consequently, CRT-D or CRT-P involves pacing in an atrium and in two ventricles, sensing in the atrium and at least one ventricle, and an internal communication element so that an event in the atrium can affect timing of pacing pulses in each ventricle, and an internal communication element so that an event in at least one ventricle can affect timing of pacing pulses in the atrium and the other ventricle.

Pulse generator parameters are usually interrogated and modified by a programming device outside the body, via a loosely-coupled transformer with one inductance within the body and another outside, or via electromagnetic radiation with one antenna within the body and another outside.

Although more than one hundred thousand conventional cardiac pacing systems are implanted annually, several well-known difficulties exist. Although tens of thousands of dual-chamber and CRT systems are implanted annually, several problems are known.

In some conditions, left-ventricular cardiac pacing can be practiced to ameliorate heart failure; a practice termed cardiac resynchronization therapy (CRT). CRT has been practiced with electrode-leads and a pulse generator, either an implantable cardioverter-defibrillator (CRT-D) or an otherwise conventional pacemaker (CRT-P). The left-ventricular pacing conventionally uses an electrode in contact with cardiac muscle in that chamber. The corresponding electrode-lead is usually placed endocardially in a transvenous manner through the coronary sinus vein, or epicardially. Although tens of thousands of left-ventricular electrode leads are implanted annually for use with separate pulse generators for CRT-D or CRT-P, various well-known difficulties are present.

In other applications and conditions, A conventional pulse generator may be connected to more than one electrode-lead. For example, atrio-ventricular pacing, also commonly called dual-chamber pacing, involves a single pulse generator connected to one electrode-lead usually placed in the right atrium and a second electrode-lead usually placed in the right ventricle. Such a system can electrically sense heartbeat signals and deliver pacing pulses separately in each chamber. In typical use, the dual-chamber pacing system paces the atrium if no atrial heartbeat is sensed since a predetermined time, and then paces the ventricle if no ventricular heartbeat is sensed within a predetermined time after the natural or paced atrial beat. Such pulse generators can also alter the timing of atrial and ventricular pacing pulses when sensing a ventricular beat that is not preceded by an atrial beat within a predetermined time; that is, a ventricular ectopic beat or premature ventricular contraction. Consequently, dual-chamber pacing involves pacing and sensing in an atrium and a ventricle, and internal communication element so that an event in either chamber can affect timing of pacing pulses in the other chamber.

Recently, left-ventricular cardiac pacing has been practiced to ameliorate heart failure; a practice termed cardiac resynchronization therapy (CRT). CRT has been practiced with electrode-leads and a pulse generator, either an implantable cardioverter-defibrillator (CRT-D) or an otherwise conventional pacemaker (CRT-P). The left-ventricular pacing conventionally uses an electrode in contact with cardiac muscle in that chamber. The corresponding electrode-lead is usually placed endocardially in a transvenous manner through the coronary sinus vein, or epicardially. Left-ventricular pacing is usually practiced together with right-atrial and right-ventricular pacing with a single implanted pulse generator connected to three electrode-leads. CRT pulse generators can independently vary the time between an atrial event and right-ventricular pacing, and the time between an atrial event and left-ventricular pacing, so that the left ventricular pacing pulse can precede, follow, or occur at the same time as the right-ventricular pacing pulse. Similarly to dual-chamber pacing, systems with left-ventricular pacing also change atrial and ventricular pacing timing in response to premature ventricular contractions. Consequently, CRT-D involves pacing in an atrium and in two ventricles, sensing in the atrium and at least one ventricle, and an internal communication element so that an event in the atrium can affect timing of pacing pulses in each ventricle, and an internal communication element so that an event in at least one ventricle can affect timing of pacing pulses in the atrium and the other ventricle. Although more than one hundred thousand ICD and CRT-D systems are implanted annually, several problems are known.

A conventional pulse generator has an interface for connection to and disconnection from the electrode leads that carry signals to and from the heart. Usually at least one male connector molding has at least one additional terminal pin not required for defibrillation functions at the proximal end of the electrode lead. The at least one male connector mates with at least one corresponding female connector molding and terminal block within the connector molding at the pulse generator. Usually a setscrew is threaded in at least one terminal block per electrode lead to secure the connection electrically and mechanically. One or more O-rings usually are also supplied to help maintain electrical isolation between the connector moldings. A setscrew cap or slotted cover is typically included to provide electrical insulation of the setscrew. The complex connection between connectors and leads provides multiple opportunities for malfunction.

Subcutaneous ICDs that do not use endocardial, transvenous or epicardial lead wires, can deliver defibrillation using subcutaneous electrodes. However, pacing the heart from subcutaneous electrodes results in diaphragmatic stimulation which is uncomfortable to the patient if used in long-term therapy. Therefore pacing therapies such as bradycardia pacing therapy, anti-tachycardia therapy, atrial overdrive pacing for the prevention of arrhythmias, dual chamber pacing for atrio-ventricular synchronization and CRT therapies are inappropriate.

In a subset of pacing devices, known pulse generators can include various sensors for estimating metabolic demand, to enable an increase in pacing rate proportional and appropriate for the level of exercise. The function is usually known as rate-responsive pacing. For example, an accelerometer can measure body motion and indicate activity level. A pressure transducer in the heart can sense the timing between opening and closing of various cardiac valves, or can give a measure of intracardiac pressure directly, both of which change with changing stroke volume. Stroke volume increases with increased activity level. A temperature sensor can detect changes in a patient's blood temperature, which varies based on activity level. The pacemaker can increase rate proportional to a detected increase in activity. Although more than one hundred thousand rate-responsive pacemakers are implanted annually, various well-known difficulties are present.

Although more than five hundred thousand pacemakers are implanted annually, various well-known difficulties are present.

The pulse generator, when located subcutaneously, presents a bulge in the skin that patients can find unsightly or unpleasant. Patients can manipulate or "twiddle" the device. Even without persistent twiddling, subcutaneous pulse generators can exhibit erosion, extrusion, infection, and disconnection, insulation damage, or conductor breakage at the wire leads. Although sub-muscular or abdominal placement can address some of these concerns, a more difficult surgical procedure is involved for implantation and adjustment, which can prolong patient recovery.

A conventional pulse generator, whether pectoral or abdominal, has an interface for connection to and disconnection from the electrode leads that carry signals to and from the heart. Usually at least one male connector molding has at least one terminal pin at the proximal end of the electrode lead. The at least one male connector mates with at least one corresponding female connector molding and terminal block within the connector molding at the pulse generator. Usually a setscrew is threaded in at least one terminal block per electrode lead to secure the connection electrically and mechanically. One or more O-rings usually are also supplied to help maintain electrical isolation between the connector moldings. A setscrew cap or slotted cover is typically included to provide electrical insulation of the setscrew. The complex connection between connectors and leads provides multiple opportunities for malfunction.

For example, failure to introduce the lead pin completely into the terminal block can prevent proper connection between the generator and electrode.

Failure to insert a screwdriver correctly through the setscrew slot, causing damage to the slot and subsequent insulation failure.

Failure to engage the screwdriver correctly in the setscrew can cause damage to the setscrew and preventing proper connection.

Failure to tighten the setscrew adequately also can prevent proper connection between the generator and electrode, however over-tightening of the setscrew can cause damage to the setscrew, terminal block, or lead pin, and prevent disconnection if necessary for maintenance.

Fluid leakage between the lead and generator connector moldings, or at the setscrew cover, can prevent proper electrical isolation.

Insulation or conductor breakage at a mechanical stress concentration point where the lead leaves the generator can also cause failure.

Inadvertent mechanical damage to the attachment of the connector molding to the generator can result in leakage or even detachment of the molding.

Inadvertent mechanical damage to the attachment of the connector molding to the lead body, or of the terminal pin to the lead conductor, can result in leakage, an open-circuit condition, or even detachment of the terminal pin and/or molding.

The lead body can be cut inadvertently during surgery by a tool, or cut after surgery by repeated stress on a ligature used to hold the lead body in position. Repeated movement for hundreds of millions of cardiac cycles can cause lead conductor breakage or insulation damage anywhere along the lead body.

Although leads are available commercially in various lengths, in some conditions excess lead length in a patient exists and is to be managed. Usually the excess lead is coiled near the pulse generator. Repeated abrasion between the lead body and the generator due to lead coiling can result in insulation damage to the lead.

Friction of the lead against the clavicle and the first rib, known as subclavian crush, can result in damage to the lead.

For CRT-D or CRT-P, multiple leads are implanted in the same patient and sometimes in the same vessel. Abrasion between the leads for hundreds of millions of cardiac cycles can cause insulation breakdown or even conductor failure.

In some applications, for example dual-chamber pacing and CRT, multiple leads are implanted in the same patient and sometimes in the same vessel. Abrasion between these leads for hundreds of millions of cardiac cycles can cause insulation breakdown or even conductor failure.

Data stored in memory of implanted pulse generators is typically made available to a physician or other personnel for collection and/or analysis. For example, information is sought regarding system performance and trouble-shooting relating to the device, lead system, and/or patient in an acute, clinical setting. The information is generally supplied via a telemetry capability between the external programmer and the implanted device. In addition, an external programmer can be used to adjust parameters of multi-function implantable medical devices, such as pacing rate, pulse amplitude, sensed signal gain, and pulse timing and coordination.

Typically, an external programmer used during a telemetry procedure is positioned remotely from the patient. A programming head of the programmer such as a wand or other external device, containing an antenna or coil, is connected to the remainder of the programmer via a stretchable coil cable and is positioned over the patient's implanted device site for programming or telemetry interrogation of the implanted device.

Communication between the implanted pulse generator and external programmer uses a telemetry coil or antenna and associated circuitry in the pulse generator where complexity increases the size and cost of the devices. Moreover, power necessary from the pulse generator battery for communication typically exceeds power for pacing by one or more orders of magnitude, introducing a requirement for battery power capability that can prevent selecting the most optimal battery construction for the otherwise low-power requirements of pacing.

Therefore, minimization of power consumption by the implanted medical device is a design and operational consideration. To facilitate power consumption management, transmitter and receiver circuitry can be powered down when not in use but are to be awakened when desired to enable communication. Awakening can occur periodically, in which the implantable device checks for a communication signal at regular intervals. The awakening process can otherwise be achieved by using electromagnetic energy coupled to the receiving antenna or coil to facilitate the wake up function. Awakening techniques result in a complicated telemetry protocol, which generally results in longer linkup times. In addition, the awakening techniques employ a relatively large antenna or coil, which is undesirable and inconsistent with a physically compact implanted medical device.

In addition to power reduction and small size, another design criterion for implanted medical devices is accurate communication of data. Communication often occurs in environments such as hospitals and doctors' offices, which can be noisy due to the presence of other electronic and electromagnetic sources. To achieve robustness ofthe link, bandwidth is generally kept low, with small packet sizes. To assure that data are transmitted accurately, the antenna or coil in the implantable device is typically positioned to maximize signal strength, both transmitted and received.

### SUMMARY

According to an embodiment of a cardiac pacing system, a leadless cardiac pacemaker is configured for implantation in electrical contact with a cardiac chamber and configured for leadless pacing.

### BRIEF DESCRIPTION OF THE DRAWINGS

Embodiments of the invention relating to both structure and method of operation may best be understood by referring to the following description and accompanying drawings, in which similar reference characters denote similar elements throughout the several views:
**FIGURE 1A** is a pictorial diagram showing an embodiment of a leadless cardiac pacemaker;
**FIGURE 1B** is a pictorial diagram showing an embodiment of a cardiac pacing system that includes a leadless cardiac pacemaker which is triggered by conductive communication or, in some embodiments, a cardiac pacing system that includes a rate-responsive leadless cardiac pacemaker;
**FIGURE 1C** is a pictorial diagram showing an embodiment of a cardiac pacing system including multiple leadless cardiac pacemakers that can be used in combination for multi-chamber cardiac pacing using conductive communication or can be used for performing cardiac pacing in conjunction with an implantable cardioverter-defibrillator (ICD);
**FIGURE 1D** is a schematic block diagram showing interconnection of operating elements of an embodiment of a biostimulator or leadless cardiac pacemaker, including a pacemaker that can be used in the multi-chamber cardiac pacing system or can be used in cardiac pacing system including one or more leadless cardiac pacemakers and an implantable cardioverter-defibrillator (ICD);
**FIGURE 1E** is a schematic block diagram showing interconnection of operating elements of an embodiment of the illustrative rate-responsive leadless cardiac pacemaker;
**FIGURE 2** is a pictorial diagram showing the physical location of some elements of an embodiment of a leadless biostimulator that can be used as part of a multi-chamber cardiac pacing system;
**FIGURE 3** is a pictorial diagram that depicts the physical location of some elements in an alternative embodiment of a leadless biostimulator that can be used as part of a multi-chamber cardiac pacing system;
**FIGURE 4** is a time waveform graph illustrating a conventional pacing pulse;
**FIGURE 5** is a time waveform graph depicting a pacing pulse adapted for communication as implemented for an embodiment of the illustrative pacing system;
**FIGURE 6** is a time waveform graph showing a sample pulse waveform using off-time variation for communication;
**FIGUREs 7A** through **7D** are schematic flow charts depicting an embodiments of a method for communicating in an implantable device; and
**FIGUREs 8A** and **8B** are schematic flow charts showing another embodiment of a method for communicating in a cardiac pacing system;
**FIGURE 9** is a state-mechanical representation illustrating an embodiment of a technique for operation of an atrial leadless cardiac pacemaker in a multi-chamber cardiac pacing system;
**FIGURE 10** is a state-mechanical representation illustrating an embodiment of a technique for operation of a right-ventricular leadless cardiac pacemaker in a multi-chamber cardiac pacing system;
**FIGURE 11** is a state-mechanical representation illustrating an embodiment of a technique for operation of a left-ventricular leadless cardiac pacemaker in a multi-chamber cardiac pacing system;
**FIGUREs 12A** and **12B** are schematic flow charts that depict embodiments of methods for operating an atrial leadless cardiac pacemaker in multi-chamber cardiac pacing;
**FIGUREs 13A** and **13B** are schematic flow charts that depict embodiments of methods for operating an right-ventricular leadless cardiac pacemaker in multi-chamber cardiac pacing;
**FIGUREs 14A** and **14B** are schematic flow charts that depict embodiments of methods for operating a left-ventricular leadless cardiac pacemaker in multi-chamber cardiac pacing;
**FIGURE 15** is a state-mechanical representation illustrating an embodiment of a technique for operation of an atrial leadless cardiac pacemaker in a multi-chamber cardiac pacing system;
**FIGURE 16** is a state-mechanical representation illustrating an embodiment of a technique for operation of a right-ventricular leadless cardiac pacemaker in a multi-chamber cardiac pacing system;
**FIGURE 17** is a state-mechanical representation illustrating an embodiment of a technique for operation of a left-ventricular leadless cardiac pacemaker in a multi-chamber cardiac pacing system;
**FIGUREs 18A** and **18B** are schematic flow charts that depict embodiments of methods for operating an atrial leadless cardiac pacemaker in a cardiac pacing system including an implantable cardioverter-defibrillator (ICD) and one or more leadless cardiac pacemakers;
**FIGUREs 19A** and **19B** are schematic flow charts that depict embodiments of methods for operating a right-ventricular leadless cardiac pacemaker in a cardiac pacing system including an implantable cardioverter-defibrillator (ICD) and one or more leadless cardiac pacemakers;
**FIGUREs 20A** and **20B** are schematic flow charts that depict embodiments of methods for operating a left-ventricular leadless cardiac pacemaker in a cardiac pacing system including an implantable cardioverter-defibrillator (ICD) and one or more leadless cardiac pacemakers;
**FIGURE 21** is a schematic flow chart depicting an embodiment of a method for operating an activity sensor in a rate-responsive cardiac pacemaker;
**FIGURE 22** is a schematic flow chart showing an embodiment of a method for communicating information for setting control parameters for an activity sensor in a cardiac pacing system;
**FIGURE 23A** is a pictorial diagram showing an embodiment of a leadless cardiac pacemaker with active fixation for use with a sheath catheter;
**FIGURE 23B** is a schematic block diagram depicting an embodiment of a leadless cardiac pacemaker with passive fixation for use with a sheath catheter;
**FIGURE 24** is a pictorial diagram that illustrates an embodiment of a delivery catheter comprising a sheath and stylet;
**FIGURE 25A** is a pictorial diagram showing an embodiment of a leadless cardiac pacemaker with active fixation for use with a sheath catheter;
**FIGURE 25B** is a pictorial diagram showing an embodiment of a leadless cardiac pacemaker with a soluble cap over the passive fixation for use with a sheath-less catheter;
**FIGURE 26** is a pictorial diagram that illustrates an embodiment of a delivery catheter for usage in delivery in implantable device using a sheath-less approach; and
**FIGURE 27** is a pictorial diagram illustrating a cross-sectional view of an embodiment of a lumen assembly and stylet delivery catheter;
**FIGUREs 28A** through **28E** are flow charts depicting embodiments of methods for implanting a biostimulation device in patient body tissue;
**FIGUREs 29A** and **29B** are pictorial diagrams showing embodiments of biostimulator systems comprising two leadless cardiac pacemakers secured to internal and to exterior surfaces of the heart, respectively, and an external programmer and two surface electrodes;
**FIGURE 30** is a schematic block diagram depicting an embodiment of an external programmer that can be used in a biostimulator system and adapted to communicate via conductive techniques;
**FIGURE 31** is a time waveform graph showing a sample of modulated communication transmitted from an external programmer to a system of one or more leadless cardiac pacemakers; and
**FIGUREs 32A** through **32E** are schematic flow charts depicting techniques that can be used in various embodiments of methods for communicating in an implantable biostimulator system.

### DETAILED DESCRIPTION

A leadless biostimulator is adapted for conducted communication.

In some embodiments of a leadless biostimulator, a leadless cardiac pacemaker can communicate by conducted communication, representing a substantial departure from the conventional pacing systems. For example, an illustrative cardiac pacing system can perform cardiac pacing that has many of the advantages of conventional cardiac pacemakers while extending performance, functionality, and operating characteristics with one or more of several improvements.

In a particular embodiment of a cardiac pacing system, cardiac pacing is provided without a pulse generator located in the pectoral region or abdomen, without an electrode-lead separate from the pulse generator, without a communication coil or antenna, and without an additional requirement on battery power for transmitted communication.

An embodiment of a cardiac pacing system configured to attain these characteristics comprises a leadless cardiac pacemaker that is substantially enclosed in a hermetic housing suitable for placement on or attachment to the inside or outside of a cardiac chamber. The pacemaker has at least two electrodes located within, on, or near the housing, for delivering pacing pulses to muscle of the cardiac chamber and optionally for sensing electrical activity from the muscle, and for bidirectional communication with at least one other device within or outside the body. The housing contains a primary battery to provide power for pacing, sensing, and communication, for example bidirectional communication. The housing can optionally contain circuits for sensing cardiac activity from the electrodes. The housing contains circuits for receiving information from at least one other device via the electrodes and contains circuits for generating pacing pulses for delivery via the electrodes. The housing can optionally contain circuits for transmitting information to at least one other device via the electrodes and can optionally contain circuits for monitoring device health. The housing contains circuits for controlling these operations in a predetermined manner.

In accordance with some embodiments, a cardiac pacemaker is adapted for implantation in the human body. In a particular embodiment, a leadless cardiac pacemaker can be adapted for implantation adjacent to the inside or outside wall of a cardiac chamber, using two or more electrodes located within, on, or within two centimeters of the housing of the pacemaker, for pacing the cardiac chamber upon receiving a triggering signal from at least one other device within the body.

For example, some embodiments of a leadless pacemaker can be configured for implantation adjacent to the inside or outside wall of a cardiac chamber without the need for a connection between the pulse generator and an electrode-lead, and without the need for a lead body.

Other example embodiments provide communication between the implanted leadless pulse generator and a device internal or external to the body, using conducted communication via the same electrodes used for pacing, without the need for an antenna or telemetry coil.

Some example embodiments can provide communication between the implanted leadless pacemaker pulse generator and a device internal or external to the body, with power requirements similar to those for cardiac pacing, to enable optimization of battery performance. In an illustrative embodiment, outgoing telemetry can be adapted to use no additional energy other than the energy contained in the pacing pulse. The telemetry function can be supplied via conducted communication using pacing and sensing electrodes as the operative structures for transmission and reception.

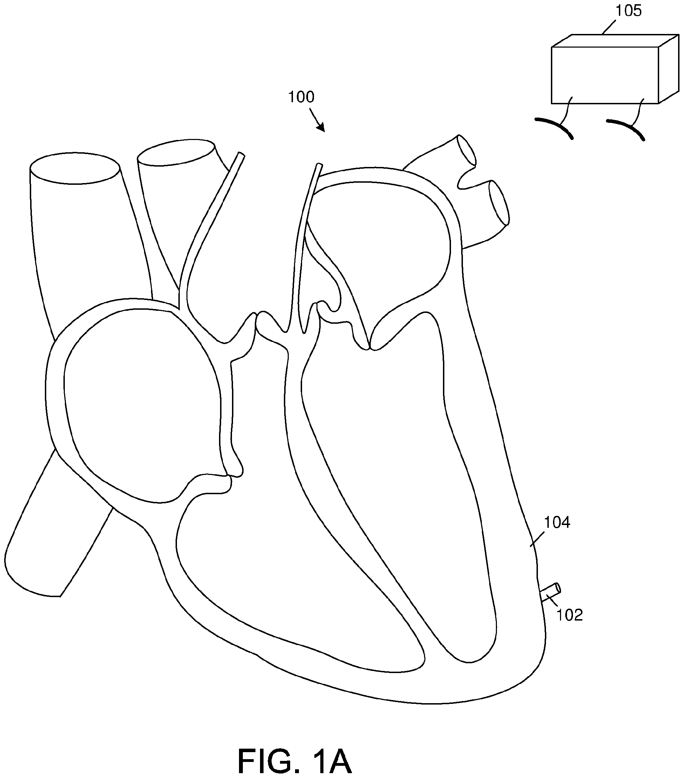

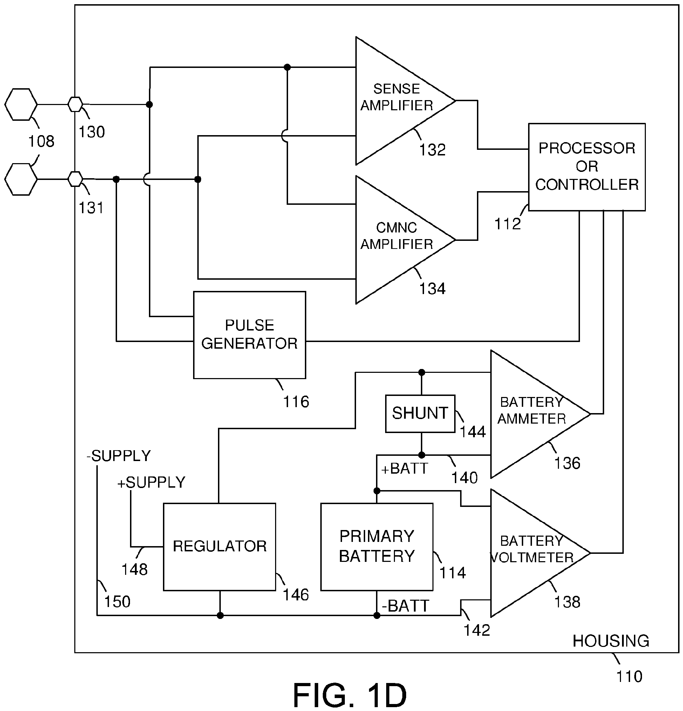

Referring to **FIGUREs 1A** and **1D****,** a pictorial view that is not shown to scale and a schematic block diagram respectively depict an embodiment of a leadless biostimulator **102,** for example cardiac pacemaker **102,** that generates pacing pulses and derives operating power from an internal source. The leadless cardiac pacemaker **102** comprises a housing **110,** two or more electrodes **108** coupled to the housing **110,** and a pulse generator **116** hermetically contained within the housing **110** and electrically coupled to the electrodes **108.** The pulse generator **116** generates and delivers electrical pulses via the electrodes **108.** A processor **112** is hermetically contained within the housing **110** and is communicatively coupled to the pulse generator **116** and the electrodes **108.** The processor **112** controls electrical pulse delivery according to programmed instructions. A power supply is hermetically contained within the housing **110** and coupled to the pulse generator **116.** The power supply supplies all energy for operations and electrical pulse generation as a source internal to the housing **110.** In the illustrative embodiment, the power supply includes a primary battery with an energy density of at least **3** watt-hours/cubic centimeter (W·h/cc).

In various embodiments, the electrodes **108** can be formed integrally to the housing 110 or may be coupled but separated by a distance, for example up to 2 cm, from the housing 110 as is typical for a screw-in electrode.

The processor **112** communicates with a device external to the pacemaker, for example typically an external programmer or another implanted device **105,** by conducted communication signals transmitted via the electrodes **108.** Communication is typically bidirectional although some implementations may include only one-way communication, either to or from the pacemaker **102.** The processor **112** controls electrical pulse delivery based on one or more programmable parameters and can be programmed by conducted communication signals transmitted over the electrodes **108.**

The pulse generator **116** can selectively generate and deliver electrical energy in a stimulation pulse to two or more of the electrodes **108** to cause a contraction of a patient's heart in response to control signals from the processor **112.** In some implementations, the pulse generator **116** can generate and deliver electrical energy with the stimulation pulse interrupted by one or more notches that convey information to a device **105** external to the pacemaker **102.** The processor **112** can encode the information to be conveyed on a pacing pulse. For example, the processor **112** can communicate control signals to the pulse generator **116** specifying characteristics of the notch or notches which define the conveyed information.

In a typical embodiment, the pulse generator **116** can generate and deliver electrical energy with the stimulation pulse interrupted by one or more notches that convey information to a device **105** external to the pacemaker **102.** For example, the conveyed information can be programmable parameter settings, event counts, power-supply voltage, power-supply current, and other data. The notches can be any suitable width. One example of a suitable notch width is approximately 15 microseconds.

In some embodiments, the pulse generator **116** generates and delivers the stimulation pulse interrupted by a notch or notches that are timed to occur within selected timing windows. The selected timing windows can be separated by any suitable spacing. One example of a suitable spacing between timing windows is approximately **100** microseconds.

In other embodiments or in some conditions, the pulse generator **116** can be configured for generating and delivering electrical energy in a series of stimulation pulses with time between the stimulation pulses selectively varied to convey information to a device **105** external to the pacemaker **102.** The variation of time between pulses can be controlled to any suitable variation. One example of a suitable allowable variation is less than a total of approximately 10 milliseconds.

In some pacemaker implementations, a battery ammeter **136** can be coupled between the primary battery **114** and the processor **112** for indicating battery current drain and indirectly indicating device health for usage by the processor **112.** Some implementations can have a battery voltmeter **138** coupled between the primary battery **114** and the processor **112** for indicating battery voltage for usage by the processor **112.**

In some implementations, the leadless cardiac pacemaker **102** can also comprise a regulator circuit **146** which is electrically connected between the power supply and pacemaker circuitry. The regulator circuit **146** regulates the voltage supply for powering pacemaker circuitry.

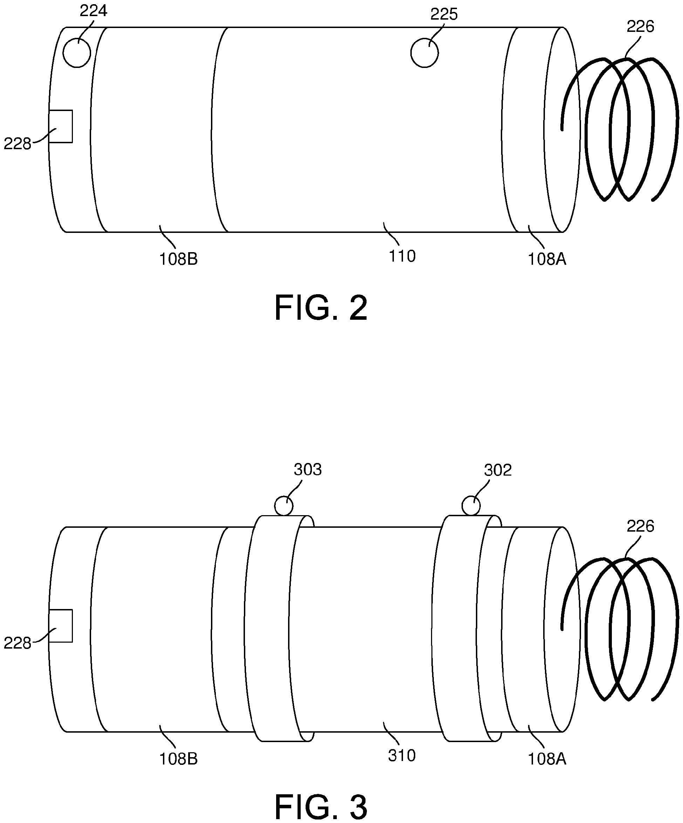

Referring to **FIGUREs 2** and **3****,** pictorial diagrams show embodiments of leadless cardiac pacemakers **102.** In the illustrative embodiments, the pacemaker **102** has a cylindrical housing **110, 310** and the electrodes comprise annular electrodes **108A, 108B** and located at extremities of the housing.

As shown in **FIGURE 2****,** the housing **110** is constructed from a ceramic material and the electrodes **108A, 108B** can be deposited on the ceramic material.

In contrast, **FIGURE 3** depicts a pacemaker **102** comprising a housing **310** constructed from titanium or stainless steel and coated over part of an exterior surface with a silicone rubber, polyurethane insulating material, or other biocompatible insulating material. The housing **310** constructed from titanium or stainless steel can function as one of the electrodes.

The leadless cardiac pacemaker **102** can further comprise a tissue connector **224, 225, 226, 302, 303** adapted to affix the housing **110, 310** to cardiac muscle **104.** In various embodiments, the tissue connector can be a helix **226** configured to rotatingly advance into the cardiac muscle **104,** one or more members pierced with a hole **224, 225** for passing a suture, one or more tines, and other structures.

Referring again to **FIGURE 1D****,** another embodiment of a leadless cardiac pacemaker **102** comprises a housing **110,** two or more electrodes **108** formed integrally to the housing **110** or at a distance of up to 2 centimeters from the housing **100,** and a pulse generator **116** hermetically contained within the housing **110** and electrically coupled to the electrodes **108.** The pulse generator **116** generates and delivers electrical pulses to the electrodes **108.** The pacemaker 102 further comprises one or more amplifiers **132, 134** hermetically contained within the housing **110** and electrically coupled to the electrodes **108.** The amplifiers **132, 134** amplify signals received from the electrodes **108.** A processor **112** is hermetically contained within the housing **110** and communicatively coupled to the pulse generator 116, the amplifiers **132, 134,** and the electrodes **108.** The processor **112** receives amplifier output signals from the amplifiers **132, 134** and controls electrical pulse delivery according to programmed instructions. A power supply is hermetically contained within the housing **110** and coupled to the pulse generator 116. The power supply supplies energy for operations and electrical pulse generation as a source internal to the housing **110.**

An amplifier can be a cardiac sensing amplifier **132** that detects signals associated with a cardiac contraction from at least two of the electrodes **108** and sends a contraction output signal to the processor **112** in response to the cardiac contraction.

Another amplifier can be a communication amplifier **134** that detects an incoming communication signal from at least one device **105** external to the pacemaker from two or more of the electrodes **108** and sends a communication output signal to the processor **112** in response to the communication signal conveying information from the external device **105.** The conveyed information can be programmable parameter settings. The communication amplifier **134** amplifies signals in any suitable frequency range. For example, the communication amplifier **134** can be configured to amplify signals in a frequency range from approximately 10 kHz to 100 kHz.

In a particular cardiac pacing system **100** embodiment, a leadless cardiac pacemaker **102** is configured for implantation in electrical contact with a cardiac chamber **104** and configured for leadless pacing, and powered by a battery **114** contained within a volume of less than one cubic centimeter.

In accordance with another embodiment of a leadless cardiac pacemaker **102,** the pacemaker **102** comprises a housing **110,** multiple electrodes **108** formed integrally to the housing **110** or coupled at a short distance of up to about 2 centimeters for example for usage of a screw-in electrode, and a pulse generator **116** hermetically contained within the housing **110** and electrically coupled to the electrodes **108.** The pulse generator **116** generates and delivers electrical pulses to the electrodes **108** and causes cardiac contractions. The pulse generator **116** also conveys information to at least one device **105** external to the pacemaker **102** by conductive communication via the electrodes **108.** The pacemaker **102** further comprises a plurality of amplifiers **132, 134** hermetically contained within the housing **110** and electrically coupled to the electrodes **108.** The amplifier **132** amplifies signals received from the electrodes **108** for detecting cardiac contractions. The amplifier **134** receives information from the external device **105.** A processor **112** is hermetically contained within the housing **110** and communicatively coupled to the pulse generator **116,** the amplifiers **132, 134,** and the electrodes **108.** The processor **112** receives amplifier output signals from the amplifiers **132, 134,** controls communications, and controls electrical pulse delivery according to programmed instructions. A power supply is hermetically contained within the housing **110** and coupled to the pulse generator **116.** The power supply supplies energy for operations, communication, and electrical pulse generation as a source internal to the housing **110.**

The pulse generator **116** is configured to consume a suitable electrical power. For example, the pacemaker **102** can be constructed so that the pulse generator **116** and rate limiter (not shown) consume a power of approximately 25 microwatts or less averaged over one cardiac cycle. The illustrative power consumption can be attained by limiting the recharging current of a pacing tank capacitor, for example.

The amplifiers **132, 134** can be configured to consume a suitable electrical power. For example, the pacemaker **102** can be constructed so that the amplifiers **132, 134** consume an electrical power of 30 microwatts or less.

The power supply is configured to consume and supply a suitable electrical power. For example, the power supply can be configured to consume a maximum electrical power of no more than 2 microwatts and configured to supply a minimum electrical power of approximately 64 microwatts.

The processor **112** can be configured to consume a suitable electrical power. For example, the pacemaker **102** can be constructed so that the processor **112** consumes a maximum electrical power of no more than 5 microwatts averaged over one cardiac cycle.

Referring again to **FIGURE 1D****,** another embodiment of a leadless cardiac pacemaker **102** comprises a housing **110,** a plurality of electrodes **108** formed integrally to the housing **110** or coupled at a distance of up to two centimeters, and a pulse generator **116** hermetically contained within the housing **110** and electrically coupled to the electrodes **108.** The pulse generator **116** generates and delivers electrical pulses to the electrodes **108** powered from a source contained entirely within the housing **110.** The pacemaker **102** further comprises a logic **112** hermetically contained within the housing **110** and communicatively coupled to the pulse generator **116** and the electrodes **108.** The logic **112** controls electrical pulse delivery according by logic execution of program instructions. A battery **114** powers the pacemaker **102** and has a volume of less than one cubic centimeter and a minimum lifetime of five years.

In accordance with another embodiment of a cardiac pacing system **100,** a leadless cardiac pacemaker **102** comprises a housing **110,** a plurality of electrodes **108** formed integrally to the housing **110** or coupled at a short distance, and a pulse generator 116 hermetically contained within the housing **110** and electrically coupled to the electrodes **108.** The pulse generator **116** generates and delivers electrical pulses to the electrodes **108.** A processor **112** is hermetically contained within the housing **110** and communicatively coupled to the pulse generator **116** and the electrodes **108.** The processor **112** can control electrical pulse delivery and can communicate with one or more devices **105** external to the pacemaker **102** by conductive communication via the electrodes **108.**

The processor **112** can be configured to control electrical pulse delivery according to one or more programmable parameters. The processor **112** is programmable by conducted communication signals transmitted via the electrodes **108.**

The processor **112** communicates via bidirectional communication with one or more devices **105** external to the pacemaker **102** by conducted communication signals transmitted via the electrodes **108.**

A leadless biostimulator can be triggered by conducted communication. For example, in a particular application, a leadless cardiac pacemaker can be triggered by conducted communication for pacing the left ventricle for CRT.

In some embodiments of a leadless biostimulator, a leadless left-ventricular cardiac pacemaker can be triggered by conducted communication, representing a substantial departure from the conventional CRT-D or CRT-P systems. For example, an illustrative cardiac pacing system can perform cardiac pacing, and in particular left-ventricular cardiac pacing for CRT-D or CRT-P, that has many of the advantages of conventional cardiac pacemakers while extending performance, functionality, and operating characteristics with one or more of several improvements.

In a particular embodiment of a cardiac pacing system, a left-ventricular cardiac pacemaker is configured for CRT-D or CRT-P operation without a left-ventricular electrode-lead connected to a separate pulse generator, without a communication coil or antenna, and without an additional requirement on battery power for transmitted communication.

An embodiment of a cardiac pacing system configured to attain these characteristics comprises a leadless cardiac pacemaker that is triggered by conducted communication and is substantially enclosed in a hermetic housing suitable for placement on or attachment to the inside or outside of a cardiac chamber, in particular the left ventricle. The leadless pacemaker has at least two electrodes located within, on, or near the housing, for delivering pacing pulses to muscle of the cardiac chamber and optionally for sensing electrical activity from the muscle, for receiving triggering signals from an implanted pulse generator, and optionally for bidirectional communication with at least one other device within or outside the body. The housing contains a primary battery to provide power for pacing, for receiving triggering signals, optionally for sensing, and optionally for bidirectional communication. The housing can optionally contain circuits for sensing cardiac activity from the electrodes. The housing contains circuits for receiving information from at least one other device via the electrodes and contains circuits for generating pacing pulses for delivery via the electrodes. The housing can optionally contain circuits for transmitting information to at least one other device via the electrodes and can optionally contain circuits for monitoring device health. The housing contains circuits for controlling these operations in a predetermined manner.

The conducted signal that triggers pacing in the leadless cardiac pacemaker can be any signal from a separate implanted pulse generator contained within the body and used with at least one electrode-lead. For example, the conducted signal can be a right-ventricular pacing pulse or atrial pacing pulse delivered by the implanted pulse generator. The implanted pulse generator may or may not include cardioversion and defibrillation functions so that a physician can use the leadless cardiac pacemaker to add left-ventricular pacing for CRT to an existing cardiac pacemaker or implantable cardioverter defibrillator. In some embodiments, a left-ventricular implanted leadless cardiac pacemaker can operate as a slave that is triggered by the atrial pacing pulse or right-ventricular pacing pulse of the separate pulse generator used for right-ventricular and/or atrial pacing.

In accordance with some embodiments, a cardiac pacemaker is adapted for implantation in the human body. In a particular embodiment, a leadless cardiac pacemaker can be adapted for implantation adjacent to the inside or outside wall of a cardiac chamber, using two or more electrodes located within, on, or within two centimeters of the housing of the pacemaker, for pacing the cardiac chamber upon receiving a triggering signal from at least one other device within the body.

In some embodiments, the cardiac pacing system can be configured for left-ventricular pacing in cardiac resynchronization therapy (CRT).

For example, some embodiments of a leadless pacemaker can be configured for implantation adjacent to the inside or outside wall of a cardiac chamber, in particular the left ventricle, without the need for a connection between the pulse generator and an electrode-lead, and without the need for a lead body.

In some examples, left-ventricular pacing can be triggered by conducted communication from another implanted pulse generator which is also implanted within the body such as by a right-ventricular pacing pulse or atrial pacing pulse from the other implanted pulse generator.

Other example embodiments optionally provide communication between the implanted leadless pacemaker and a programmer outside the body, or between the implanted leadless pacemaker and another pulse generator implanted within the body, using conducted communication via the same electrodes used for pacing, without the need for an antenna or telemetry coil.

Some example embodiments can to provide communication between the implanted leadless pacemaker and a programmer outside the body, or between the implanted leadless pacemaker and another pulse generator implanted within the body, with power requirements for the implanted leadless pacemaker similar to those for cardiac pacing, enabling optimization of battery performance.

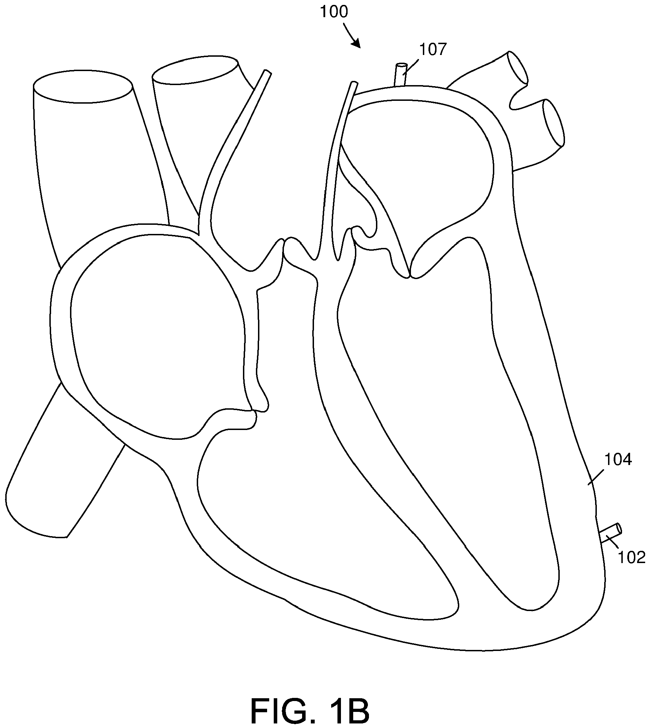

Referring to **FIGUREs 1B** and **1D****,** a pictorial view which is not shown to scale and a schematic block diagram respectively depict an embodiment of a cardiac pacing system **100** that comprises a leadless cardiac pacemaker **102** configured for implantation in electrical contact with a left ventricular cardiac chamber **104** and for leadless triggered left-ventricular pacing for cardiac resynchronization therapy (CRT) in response to conducted signals from a pulse generator **107.**

In a particular arrangement, the leadless cardiac pacemaker **102** can be configured for leadless triggered left-ventricular pacing in response to conducted signals from one or more implanted leadless or electrode-lead pulse generators **107.** The system **100** can also include the one or more implanted leadless or electrode-lead pulse generators **107** configured to conduct signals to the leadless cardiac pacemaker that trigger left-ventricular pacing.

In some arrangements, the cardiac pacing system **100** can include or be used with pulse generators **107** such as a cardioverter-defibrillator (CRT-D) or a conventional pacemaker (CRT-P). For example, the leadless cardiac pacemaker **102** can be configured for operation as a left-ventricular pacemaker for cardiac resynchronization therapy using a cardioverter-defibrillator (CRT-D) or cardiac resynchronization therapy using an otherwise conventional pacemaker (CRT-P), in response to wireless conducted signals from at least one implanted leadless or electrode-lead pulse generator **107.** The wireless conducted signals are conducted pacing and/or cardiac signals.

The cardiac pacing system **100** can be implemented in various arrangements for multiple therapeutic uses. For example, the leadless cardiac pacemaker **102** can be configured for leadless triggered left-ventricular pacing in response to conducted signals selected from signals from a separate implanted pulse generator, signals from one or more electrode-leads of a separate implanted pulse generator, a right-ventricular pacing pulse delivered by an implanted pulse generator, an atrial pacing pulse delivered by an implanted pulse generator, a signal delivered in combination with a cardioversion function, and a signal delivered in combination with a defibrillation function.

In one example application, the leadless cardiac pacemaker **102** can be operative as a "slave" left-ventricular leadless cardiac pacemaker triggered by an atrial pacing pulse or right-ventricular pacing pulse of the pulse generator operative for right-ventricular and/or atrial pacing.

In another example, application, the leadless pacemaker **102** can be configured for left-ventricular pacing triggered by conducted communication from a pulse generator **107** which is implanted within the body. Left-ventricular pacing can be triggered by a right-ventricular pacing pulse or atrial pacing pulse delivered by the pulse generator **107.**

The leadless cardiac pacemaker **102** has two or more electrodes **108** abutting or adjacent to a housing **110** and configured for delivering pacing pulses and operative as an incoming communication channel for receiving triggering signals from the pulse generator **107.** The triggering information can be an electrical potential difference resulting from a right-ventricular pacing pulse or an atrial pacing pulse of an implanted pulse generator and electrode-lead system.

The illustrative cardiac pacing system **100** further comprises a controller **112** coupled to the electrodes **108** adapted to examine triggering information validity. For a valid condition, the controller **112** can activate delivery of a pacing pulse following a predetermined delay of zero or more milliseconds.

The incoming communication channel can communicate information such as pacing rate, pulse duration, sensing threshold, delay intervals, refractory time intervals, stimulation pulse amplitudes, and parameters commonly programmed from an external programmer in a pacemaker.

The electrodes **108** can also be used as an outgoing communication channel for communicating information such as programmable parameter settings, pacing and sensing event counts, battery voltage, battery current, information commonly displayed by external programmers used with pacemakers, and echoed information from the incoming channel to confirm correct programming.

In one example control technique, the controller **112** can monitor electrical signals on the electrodes **108** and examine the potential difference resulting from a pacing pulse. The controller **112** can also decode information encoded in the pacing pulse and evaluate the decoded information for pacing pulse signature validation.

In another example, the controller **112** can monitor electrical signals on the electrodes 108 and examine output pulse duration from an implanted pulse generator **107** for usage as a signature for determining triggering information validity. For a signature arriving within predetermined limits, the controller **112** can activate delivery of a pacing pulse following a predetermined delay of zero or more milliseconds. The predetermined delay can be determined from information sources such as information preset at manufacture, information that is programmed via an external programmer, and information attained by adaptive monitoring and conformance to duration of a triggering signal.

In another example, the controller **112** can monitor electrical signals on the electrodes 108 and examine information or parameters such as output pulse amplitude, duration, and rate from an implanted pulse generator for usage as a signature for determining triggering information validity. For a signature that arrives within predetermined limits, the controller **112** can activate delivery of a pacing pulse following a predetermined delay of zero or more milliseconds.

In other embodiments, a controller **112** coupled to the two electrodes **108** can also be adapted to trigger delivery of a left-ventricular pacing pulse from an atrial pacing pulse of an implanted pulse generator for cardiac resynchronization therapy (CRT) after a selected atrio-ventricular delay of 50 to 300 milliseconds. The controller **112** can vary the atrio-ventricular delay according to time since a last delivered left-ventricular pacing pulse whereby a shorter atrio-ventricular delay is selected for a higher atrial rate.

Also referring to **FIGURE 1D****,** the block diagram illustrates an embodiment of a cardiac pacing system **100** comprising a leadless cardiac pacemaker **102** configured for implantation in electrical contact with a cardiac chamber **104.** The pacemaker **102** receives and evaluates triggering information from an implanted pulse generator **107** via an electrical signal conducted from an atrial or ventricular pacing pulse delivered by the implanted pulse generator **107.**

The pacing system **100** can further comprise one or more implanted leadless or electrode-lead pulse generators **107** which are configured to conduct signals that trigger left-ventricular pacing to the leadless cardiac pacemaker by direct conduction using modulated signals at a frequency in a range from approximately 10 kHz to 100 kHz.

The leadless cardiac pacemaker **102** can be configured for retriggering by the implanted pulse generator **107** whereby the leadless cardiac pacemaker **102** generates a pacing pulse after a predetermined time with no received triggering signal and the predetermined time is preset slightly longer than a pacing interval of the implanted pulse generator **107,** enabling the leadless cardiac pacemaker **102** to operate as a synchronized redundant pacemaker.

The pacemaker **102** can trigger delivery of an atrial pacing pulse in response to sensing of an atrial heartbeat in sinus rhythm and in response to detection of sinus rhythm below a selected rate for atrial demand pacing.

The controller **112** can be adapted to limit synchronous pacing pulse delivery rate to a selected maximum rate.

In some embodiments, multiple leadless cardiac pacemakers **102** can be included in the system **100** and configured for implantation in electrical contact with at least one cardiac chamber **104** and distributed epicardially. The multiple leadless cardiac pacemakers **102** can respond with pacing activity that is timed from an initial triggering pulse for generating simultaneous pulses for defibrillation or cardioversion therapy.

Referring again to **FIGURE 1D****,** a schematic block diagram depicts a generic embodiment of a biostimulation system **100** comprising a biostimulator **102** configured for implantation in electrical contact with a biological tissue **104** and configured for receiving and evaluating triggering information from an implanted pulse generator **107** via an electrical signal conducted from an stimulation pulse delivered by the implanted pulse generator.

The incoming communication channel serves to receive triggering information for the leadless cardiac pacemaker. In a most simple expected manner, the triggering information can comprise an electrical potential difference appearing on the electrodes **108** of the leadless cardiac pacemaker **102** resulting from a right-ventricular pacing pulse or an atrial pacing pulse of another pulse generator **107** and electrode-lead system implanted in the body. When the leadless cardiac pacemaker receives the triggering information via electrodes **108** and circuits **134,** controlling or processing circuits **112** examine the validity of the triggering information. If the information is determined to be valid, the controller **112** instructs the pulse generator **116** to deliver a pacing pulse, optionally after a predetermined delay.

Referring to **FIGURE 2****,** a schematic pictorial view shows an embodiment of the leadless cardiac pacemaker **102** that can be used in the cardiac pacing system **100.** The leadless cardiac pacemaker **102** comprises a hermetic housing **110** configured for placement on or attachment to the inside or outside of a cardiac chamber **104.** Two or more electrodes **108** abut or are adjacent to the housing **110.** The electrodes **108** are configured for delivering pacing pulses and receiving triggering signals from the pulse generator **107.** The electrodes **108** can also sense electrical activity from cardiac chamber muscle.

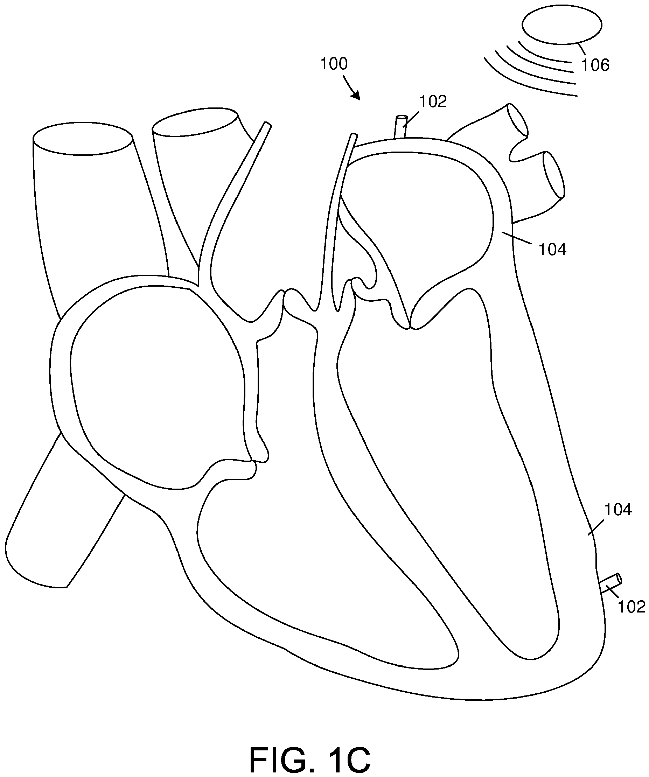

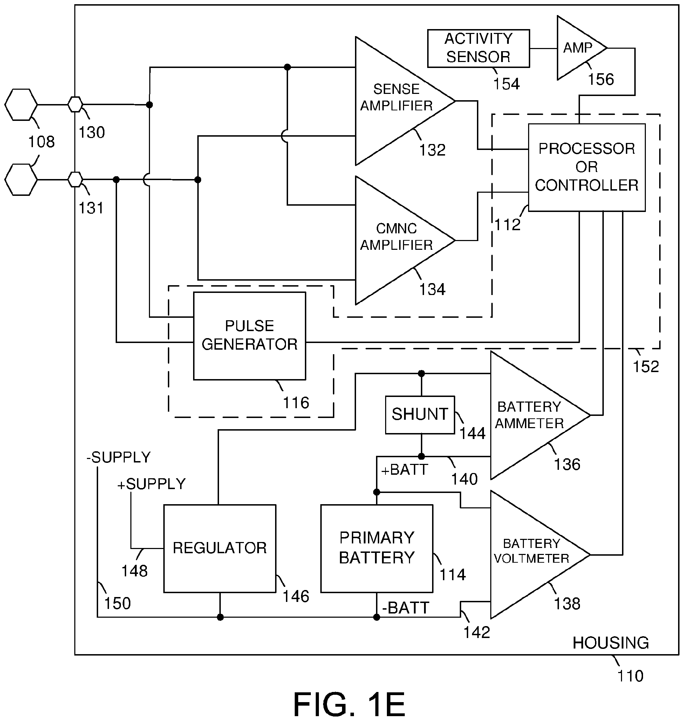

The illustrative leadless pulse generator **102** as shown in **FIGUREs 1****,** **2,** and **3** can use the duration T0 of the output pulse of another pulse generator **107** implanted within the body as a "signature" to aid the processor **112** in determining the validity of triggering information received via electrodes **108** and receiving circuits **134.** When such a signature arrives with duration within predetermined limits, processor 112 recognizes the signature as a valid triggering signal, and instructs pulse generator **116** to generate a pacing pulse, optionally after a delay.

The predetermined limits for evaluating duration of the received triggering information can be stored in the depicted leadless pulse generator **102** in various manners. For example, limits can be preset at manufacture, programmed using a programmer outside the body, or can be "learned" by the leadless pulse generator **102.** For example, if the leadless pulse generator **102** detects a predetermined number of pulses via electrodes **108,** and receiving circuits **134,** each of such pulses with substantially the same duration, such as within 10 microseconds between pulses, and each is separated from the others by an interval characteristic of cardiac pacing, such as 400 to 1200 milliseconds, then the leadless pulse generator can use the maximum and minimum measured durations to establish limits for validity of received triggering signals.

In addition, the leadless pacemaker **102** can use the amplitude of the output pulse of another pulse generator to validate the pacing pulse, since pacing amplitudes tend to be higher magnitude than other electrical signals in the body.

In the illustrative leadless pacemaker **102,** the other triggering pulse generator **107** implanted in the body generates a pacing pulse to trigger each pacing pulse generated by the leadless pacemaker **102.** In a simple embodiment, the other triggering pulse generator **107** provides right ventricular pacing pulses as needed, and the left-ventricular leadless pulse pacemaker **102** generates a triggered pacing pulse to the left ventricle substantially synchronously with the detected right-ventricular pacing pulse, or after a predetermined delay. The predetermined delay can be a few tens of milliseconds typically and can be programmed from a programmer outside the body or communicated from the other pulse generator **107** implanted within the body.

Conditions can be sufficient or even advantageous in certain applications of CRT to pace the left ventricle a few tens of milliseconds before pacing the right ventricle, or to dispense altogether with right-ventricular pacing and provide only left-ventricular pacing. The leadless pacemaker **102** can use triggering information from the atrial pacing pulse of the other pulse generator **107** implanted within the body to provide CRT by delaying the left-ventricular pacing pulse typically by 50 to 300 milliseconds from the received atrial pacing pulse. The leadless pacemaker **102** can vary the atrio-ventricular delay according to the time since the last delivered left-ventricular pacing pulse, in general providing shorter atrio-ventricular delays for higher atrial rates. To realize the illustrative operation, the other pulse generator **107** implanted within the body provides triggered atrial pacing, defined as delivery of an atrial pacing pulse after sensing an atrial heartbeat in sinus rhythm, or when sinus rhythm falls below a predetermined rate and the atrial pacemaker paces the atrium on demand.

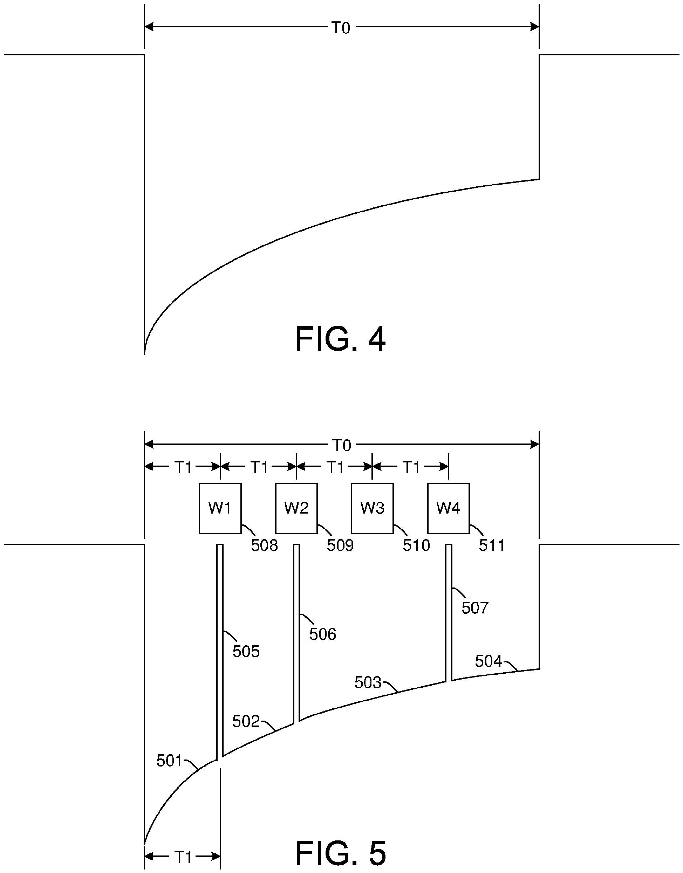

As described hereinbefore, the leadless pulse generator **102** can evaluate information received on the incoming channel to determine validity for triggering pacing pulses. The coding scheme illustrated in **FIGURE 5** can be used to provide a more distinctive signature for the other implanted pulse generator **107.** In the depicted embodiment, the other implanted pulse generator **107** encodes an output pulse in the manner illustrated in **FIGURE 5** and the leadless pulse generator **102** uses the additional data encoded in the manner shown in **FIGURE 5** to evaluate the received information to determine whether or not the pulse corresponds to the atrial or ventricular pacing pulse of the other implanted pulse generator **107.**

To ensure the leadless cardiac pacemaker functions correctly, a specific minimum internal supply voltage is maintained. When pacing tank capacitor charging occurs, the supply voltage can drop from a pre-charging level which can become more significant when the battery nears an end-of-life condition and has reduced current sourcing capability. Therefore, a leadless cardiac pacemaker can be constructed with a capability to stop charging the pacing tank capacitor when the supply voltage drops below a specified level. When charging ceases, the supply voltage returns to the value prior to the beginning of tank capacitor charging.

In another technique, the charge current can be lowered to prevent the supply voltage from dropping below the specified level. However, lowering the charge current can create difficulty in ensuring pacing rate or pacing pulse amplitude are maintained, since the lower charge current can extend the time for the pacing tank capacitor to reach a target voltage level.

Referring again to **FIGURE 1D****,** the circuit **132** for receiving communication via electrodes **108** receives the triggering information as described and can also optionally receive other communication information, either from the other implanted pulse generator **107** or from a programmer outside the body. This other communication could be coded with a pulse-position scheme as described in **FIGURE 5** or could otherwise be a pulse-modulated or frequency-modulated carrier signal, preferably from 10 kHz to 100 kHz.

The illustrative leadless pacemaker **102** could otherwise receive triggering information from the other pulse generator **107** implanted within the body via a pulse-modulated or frequency-modulated carrier signal, instead of via the pacing pulses of the other pulse generator **107.**

As in conventional pacemakers, the leadless pacemaker **102** can include in controller **112** a capability to limit the rate of delivering synchronous pacing pulses, typically to 150 pulses per minute or less. An independent hardware rate limiter can also be used to prevent rapid pacing in case of hardware or software failure, as in conventional pacemakers.

In the CRT application described herein, the leadless cardiac pacemaker **102** can provide left-ventricular pacing for amelioration of heart failure but typically does not provide beat-to-beat life support, as with a conventional pacemaker or implantable cardioverter-defibrillator. Consequently certain functions considered essential in a conventional cardiac pacemaker can be optional in the illustrative application. Features such as sensing cardiac activity, communication from or to an external programmer, and monitoring device health, while potentially beneficial, are not essential to the operation ofthe pacemaker **102.**

In an example of a configuration for a cardiac pacing system **102,** multiple leadless pacemakers **102** can be distributed around the heart epicardially, endocardially, or in a combination of epicardially and endocardially, and operate in a coordinated manner based on timing from an initial triggering pulse to generate pulses simultaneously, thereby providing defibrillation or cardioversion therapy.

A slight modification of a stored program in processor **112** enables the leadless cardiac pacemaker **102** to be retriggered rather than triggered by a pacing pulse from another pulse generator **107** implanted in the body so that the leadless cardiac pacemaker **102** generates a pacing pulse after a predetermined time with no received triggering signal. The predetermined time can be preset slightly longer than the pacing interval of the other implanted pulse generator **107** so that the leadless pulse generator **102** serves as a synchronized redundant pacemaker. Synchronized redundant pacing is useful in pacemaker-dependent patients to ensure continued pacing in the event of failure of one implanted pulse generator. Use of two such leadless triggered cardiac pacemakers, one with a slightly longer retriggering interval than the other, would ensure continued pacing if either device fails.

Referring again to **FIGURE 1B****,** in accordance with another embodiment a cardiac pacing system **100** comprises a leadless cardiac pacemaker **102** that is configured for implantation in electrical contact with a cardiac chamber **104** and configured for delivering a pacing pulse and encoding outgoing communication in the pacing pulse whereby a power requirement for the outgoing communication adds nothing above the power requirement for delivering the pacing pulse.

In a specific embodiment, the outgoing communication power requirement does not exceed approximately 25 microwatts.

In another particular embodiment of a cardiac pacing system **100,** a leadless cardiac pacemaker **102** can be configured for implantation in electrical contact with a left ventricular cardiac chamber and configured for leadless triggered left-ventricular pacing in response to conducted signals from a pulse generator **107** and powered by a battery **114** contained within a volume of less than one cubic centimeter.

The illustrative cardiac pacing system **100** enables left-ventricular pacing for cardiac resynchronization therapy, employing a leadless triggered pacemaker **102** for the left ventricle, in conjunction with another implanted pulse generator **107** with at least one electrode-lead or leadless pulse generator.

The depicted leadless cardiac pacemaker **102** can receive and evaluate triggering information from another implanted pulse generator **107** via an electrical signal conducted from the other pulse generator's atrial or ventricular pacing pulse.

The illustrative cardiac pacing system **100** enables encoding optional outgoing communication in the pacing pulse, so that the total power consumption for outgoing communication and pacing does not exceed the power consumption for pacing alone. Thus, power consumption for outgoing communication is effectively zero because outgoing communication uses the same power already used to create a pacing pulse.

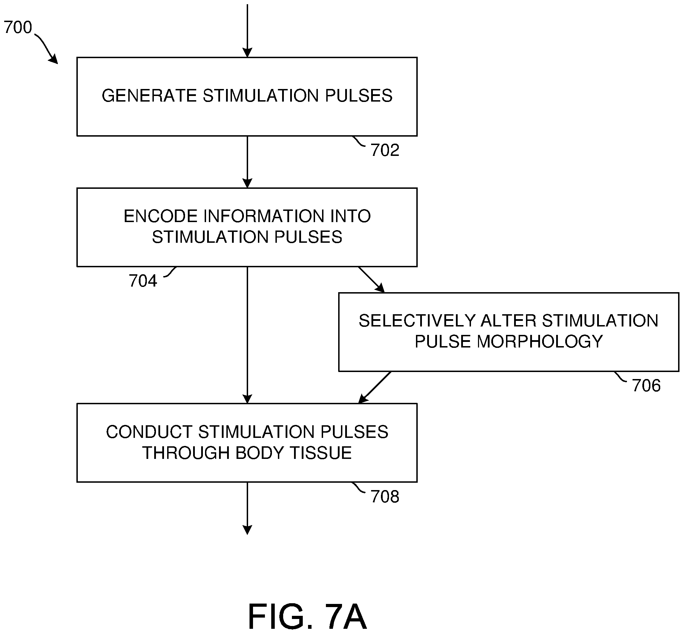

Referring to **FIGURE 7A** in combination with **FIGURE 5****,** a schematic flow chart depicts an embodiment of a method **700** for communicating in an implantable device. Stimulation pulses are generated **702** by an implanted biostimulator. Information can be encoded **704** onto the generated stimulation pulses by the implanted biostimulator by selective alteration **706** of stimulation pulse morphology that is benign to therapeutic effect and energy cost of the stimulation pulse. The stimulation pulses are conducted **708** through body tissue via electrodes for antenna-less and telemetry coil-less communication.

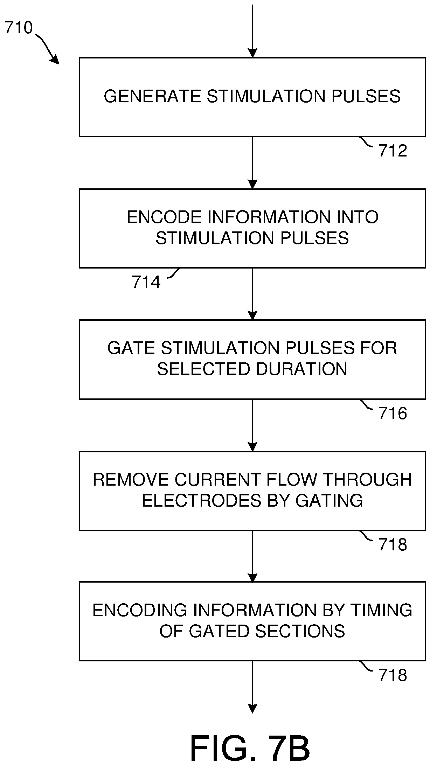

Referring to **FIGURE 7B****,** a flow chart depicts operations of another embodiment of a method **710** for communicating in an implantable device. Stimulation pulses are generated **712** on stimulating electrodes of an implanted biostimulator. Information can be encoded **714** onto generated stimulation pulses by gating **716** the stimulation pulses for selected durations at selected timed sections in the stimulation pulses whereby gating removes **718** current flow through the stimulating electrodes and timing of the gated sections encodes **719** the information.

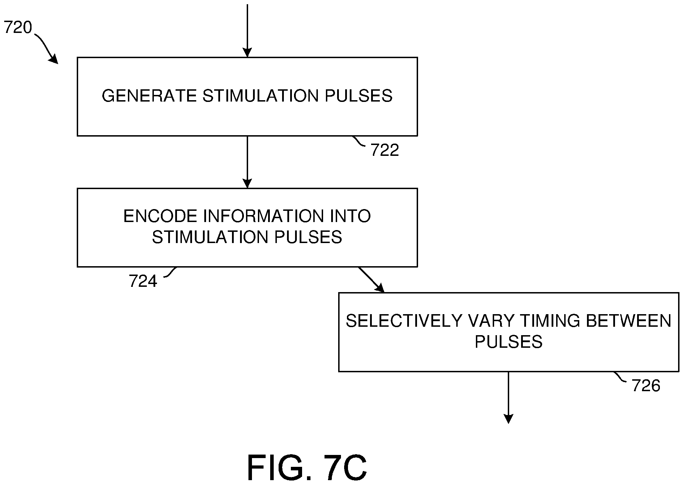

Referring to **FIGURE 7C****,** a flow chart depicts an embodiment of a communication method **720** for usage in an implantable device. Stimulation pulses are generated **722** on stimulating electrodes of an implanted biostimulator. Information is encoded **724** onto generated stimulation pulses by selectively varying **726** timing between consecutive stimulation pulses.

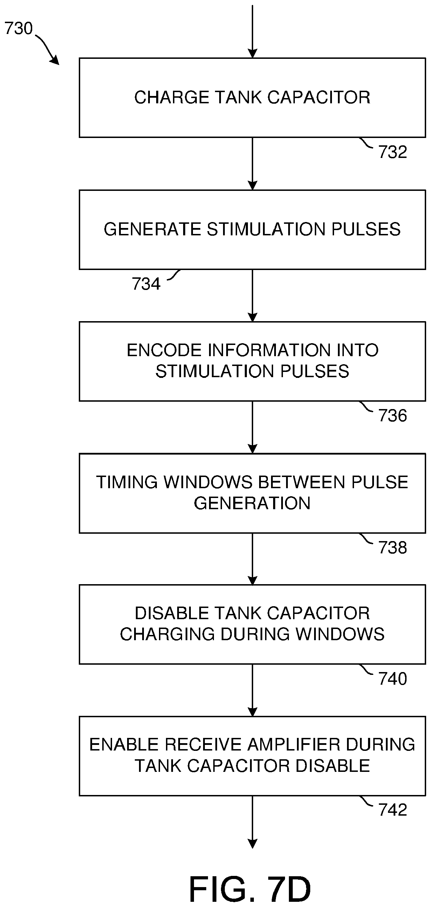

Referring to **FIGURE 7D****,** a flow chart depicts another embodiment of a communication method **730** for usage in an implantable device. A tank capacitor is charged **732** in preparation for stimulation pulse generation and stimulation pulses are generated **734** on stimulating electrodes of an implanted biostimulator. Information is encoded **736** onto generated stimulation pulses one or more windows timed **738** between pulse generation. Charging of the tank capacitor can be disabled **740** during the one or more timed windows with a receive amplifier enabled **742** in the implanted biostimulator while the tank capacitor is disabled so that operation of the communications amplifier and charging of the pacing tank capacitor are made mutually exclusive.

In conventional implantable devices, a communication amplifier and a sensing amplifier both continuously consume power, for example constantly requiring on the order of 25 microwatts and 5 microwatts respectively from the battery. In some embodiments of the implantable cardiac pacemaker described herein, operation of the communications amplifier and charging of the pacing tank capacitor can be made mutually exclusive. For example, after the pacing pulse, charging of the pacing tank capacitor can be suspended by an appropriate time window, for example 10 milliseconds. During the window, the communication amplifier can be enabled and ready to receive commands and information from an external programmer or another implantable device. Thus, the 25 microwatts used by the communications amplifier is mutually exclusive from the 25 microwatts consumed by charging the pacing tank capacitor, enabling the total power consumption of the pacemaker to drop to 39 microwatts.

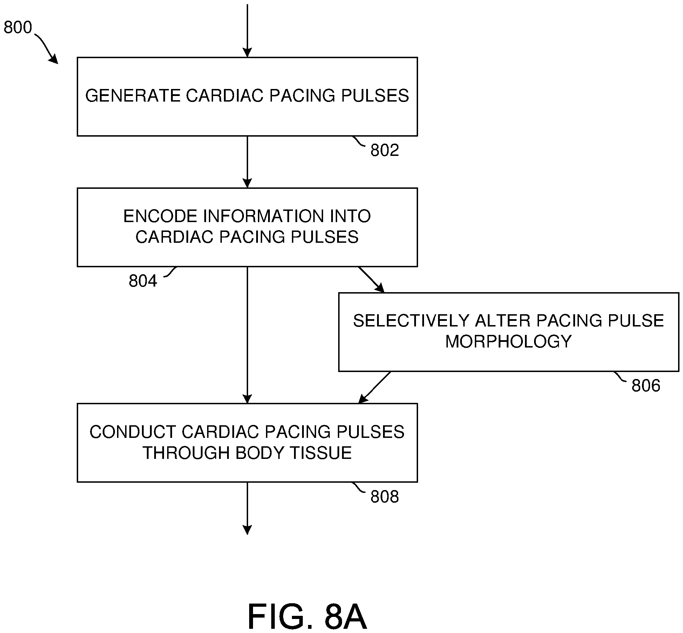

Referring to **FIGURE 8A** in combination with **FIGURE 5****,** a schematic flow chart depicts an embodiment of a method **800** for communicating in a cardiac pacing system. Cardiac pacing pulses are generated **802** by an implanted leadless cardiac pacemaker. Information can be encoded **804** onto the generated cardiac pacing pulses by the implanted leadless cardiac pacemaker by selective alteration **806** of pacing pulse morphology that is benign to therapeutic effect and energy cost of the pacing pulse. The cardiac pacing pulses are conducted **808** into body tissue via electrodes for antenna-less and telemetry coil-less communication.

In some embodiments, the information that is encoded onto generated cardiac pacing pulses at the implanted leadless cardiac pacemaker comprises pacemaker state information, battery voltage, lead impedance, sensed electrocardiogram amplitude, pacemaker current drain, and programmed parameters.

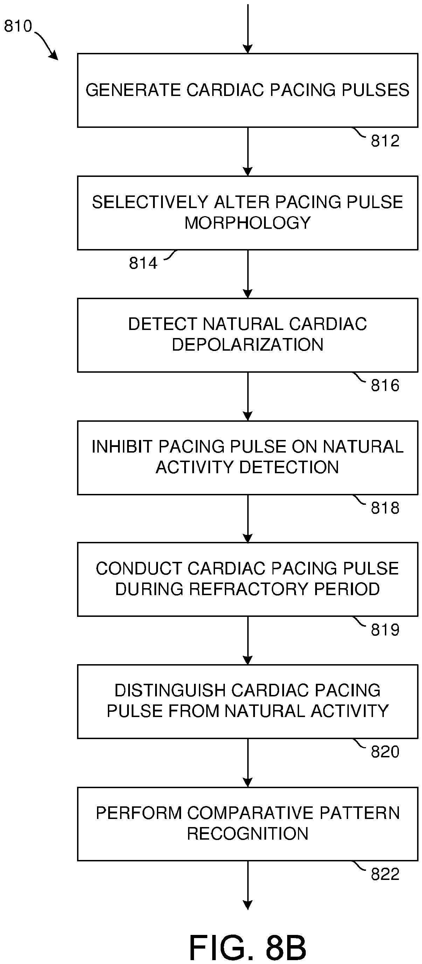

**FIGURE 8B** illustrates an embodiment ofthe method **810** wherein information is encoded **812** onto generated cardiac pacing pulses at the implanted leadless cardiac pacemaker by selective alteration **814** of pacing pulse morphology that is benign to therapeutic effect and energy cost of the pacing pulse. The implanted leadless cardiac pacemaker detects **816** a natural cardiac depolarization and inhibits **818** cardiac pacing pulse delivery with delay for delivery during a refractory period following the natural cardiac depolarization before conducting **819** the cardiac pacing pulses into body tissue via electrodes for antenna-less and telemetry coil-less communication.

In some embodiments, a generated cardiac pacing pulse is distinguished **820** from a natural cardiac depolarization in an electrocardiogram by comparative pattern recognition **822** of a pacing pulse and an R-wave produced during a cardiac cycle.

A system of leadless cardiac pacemakers with low-power conducted communication enables dual-chamber pacing, CRT-P, or other multi-chamber pacing.

Various embodiments of a system of leadless cardiac pacemakers with conducted communication for multi-chamber pacing are disclosed that can implement, for example, dual-chamber pacing or three-chamber pacing for cardiac resynchronization therapy. The individual leadless cardiac pacemakers can be substantially enclosed in a hermetic housing suitable for placement on or attachment to the inside or outside of a cardiac chamber. The pacemaker can have at least two electrodes located within, on, or near the housing, for delivering pacing pulses to and sensing electrical activity from the muscle of the cardiac chamber, and for bidirectional communication with at least one other co-implanted leadless cardiac pacemaker and optionally with another device outside the body. The housing can contain a primary battery to provide power for pacing, sensing, and communication. The housing can also contain circuits for sensing cardiac activity from the electrodes, receiving information from at least one other device via the electrodes, generating pacing pulses for delivery via the electrodes, transmitting information to at least one other device via the electrodes, monitoring device health, and controlling these operations in a predetermined manner.

A cardiac pacing system includes two or more leadless cardiac pacemakers to enable improved performance in comparison to conventional multi-chamber cardiac pacing arrangements.

In some embodiments, a cardiac pacing system comprises two or more leadless pacemakers for implantation adjacent to the inside or outside wall of a cardiac chamber, without the need for a connection between the pulse generator and electrode lead that can be connected or disconnected during implantation and repair procedures, and without the need for a lead body.

In some embodiments of a cardiac pacing system, communication between implanted leadless cardiac pacemakers and optionally between an implanted leadless cardiac pacemaker and a device external to the body, uses conducted communication via the same electrodes used for pacing, without the need for an antenna or telemetry coil.

Some embodiments and/or arrangements can implement communication between an implanted leadless cardiac pacemaker and a device or devices internal or external to the body, with power requirements similar to those for cardiac pacing, to enable optimization of battery performance. For example, transmission from the leadless cardiac pacemaker adds no power while reception adds a limited amount of power, such as about 25 microwatts.

Referring to **FIGURE 1C****,** a pictorial diagram, which is not to scale, shows an embodiment of a cardiac pacing system **100** including multiple leadless cardiac pacemakers **102** that can be used in combination for multi-chamber cardiac pacing and can communicate via conductive communication. **FIGURE 1D** is a schematic block diagram showing an embodiment of a leadless cardiac pacemaker **102** that can be a component of the cardiac pacing system **100.** In the system **100,** multiple leadless cardiac pacemakers **102** are individually configured for implantation in electrical contact with multiple cardiac chambers **104** and arranged in combination for multi-chamber cardiac pacing. The individual leadless cardiac pacemakers **102** comprise two or more leadless electrodes **108** configured for delivering cardiac pacing pulses, sensing evoked and/or natural cardiac electrical signals, and communicating bidirectionally among the leadless cardiac pacemakers.

The illustrative cardiac pacing system **100** enables extended performance in comparison to conventional dual-chamber cardiac pacing and cardiac resynchronization therapy (CRT-P). The depicted cardiac pacing system **100** can be configured for dual-chamber, CRT-P, and other multi-chamber cardiac pacing schemes.

Individual pacemakers of the multiple leadless cardiac pacemakers **102** can comprise a hermetic housing **110** configured for placement on or attachment to the inside or outside of the cardiac chambers **104.** The two or more leadless electrodes **108** proximal to the housing **110** can be configured for bidirectional communication with one or more other devices **106** within or outside the body.

The cardiac pacing system **100** can perform multi-chamber cardiac pacing in the absence of a pulse generator located in the pectoral region or abdomen of a patient, in the absence of an electrode-lead separate from the pulse generator, in the absence of a communication coil or antenna, and without imposing an additional requirement on battery power for communication of pacing pulse delivery.

The cardiac pacing system **100** attains improved performance through usage of at least two leadless cardiac pacemakers **102.** An individual leadless cardiac pacemaker **102** can be substantially enclosed in a hermetic housing **110** which is suitable for placement on or attachment to the inside or outside of a cardiac chamber **104.** The pacemaker **102** has at least two electrodes **108** located within, on, or near the housing **110,** for delivering pacing pulses to and sensing electrical activity from the muscle of the cardiac chamber **104,** and for bidirectional communication with at least one other leadless cardiac pacemaker within the body, and possibly for bidirectional communication with at least one other device **106** outside the body. The illustrative housing **110** contains a primary battery **114** to supply power for pacing, sensing, and communication. The depicted housing **110** also contains circuits for sensing cardiac activity from the electrodes, receiving information from at least one other device via the electrodes **108,** generating pacing pulses for delivery via the electrodes **108,** transmitting information to at least one other device via the electrodes **108,** optionally monitoring device health, and controlling the operations in a predetermined manner.

**FIGURE 1D** depicts a single leadless cardiac pacemaker **102** and shows the pacemaker's functional elements substantially enclosed in a hermetic housing **110.** The pacemaker **102** has at least two electrodes **108** located within, on, or near the housing **110,** for delivering pacing pulses to and sensing electrical activity from the muscle of the cardiac chamber, and for bidirectional communication with at least one other device within or outside the body. Hermetic feedthroughs **130, 131** conduct electrode signals through the housing **110.** The housing **110** contains a primary battery **114** to supply power for pacing, sensing, and communication. The housing **110** also contains circuits **132** for sensing cardiac activity from the electrodes **108,** circuits **134** for receiving information from at least one other device via the electrodes **108,** and a pulse generator **116** for generating pacing pulses for delivery via the electrodes **108** and also for transmitting information to at least one other device via the electrodes **108.** The housing **110** can further contain circuits for monitoring device health, for example a battery current monitor **136** and a battery voltage monitor **138,** and can contain circuits **112** for controlling operations in a predetermined manner.

Individual pacemakers **102** of the leadless cardiac pacemaker plurality can be configured to intercommunicate and to communicate with a non-implanted programmer via the electrodes **108** that are also used for delivering pacing pulses. Accordingly, the pacemakers **102** can be configured for antenna-less and telemetry coil-less communication. The individual pacemakers **102** can also intercommunicate among multiple pacemakers and communicate with a non-implanted programmer via communication that has outgoing communication power requirements essentially met by power consumed in cardiac pacing.

The two or more leadless electrodes **108** can be configured to communicate bidirectionally among the multiple leadless cardiac pacemakers to coordinate pacing pulse delivery using messages that identify an event at an individual pacemaker originating the message. A pacemaker or pacemakers that receive the message react as directed by the message depending on the message origin or location. In some embodiments or conditions, the two or more leadless electrodes **108** can be configured to communicate bidirectionally among the multiple leadless cardiac pacemakers and transmit data including designated codes for events detected or created by an individual pacemaker. Individual pacemakers can be configured to issue a unique code corresponding to an event type and a location of the sending pacemaker.

Information communicated on the incoming communication channel can include but is not limited to pacing rate, pulse duration, sensing threshold, and other parameters commonly programmed externally in conventional pacemakers. Information communicated on the outgoing communication channel can include but is not limited to programmable parameter settings, pacing and sensing event counts, battery voltage, battery current, device health, and other information commonly displayed by external programmers used with conventional pacemakers. The outgoing communication channel can also echo information from the incoming channel, to confirm correct programming.

Moreover, information communicated on the incoming channel can also include a message from another leadless cardiac pacemaker signifying that the other leadless cardiac pacemaker has sensed a heartbeat or has delivered a pacing pulse, and identifies the location of the other pacemaker. Similarly, information communicated on the outgoing channel can also include a message to another leadless cardiac pacemaker or pacemakers that the sending leadless cardiac pacemaker has sensed a heartbeat or has delivered a pacing pulse at the location of the sending pacemaker.

For example, in some embodiments an individual pacemaker **102** of the multiple leadless cardiac pacemakers can be configured to deliver a coded pacing pulse with a code assigned according to pacemaker location and configured to transmit a message to one or more other leadless cardiac pacemakers via the coded pacing pulse wherein the code identifies the individual pacemaker originating an event. The pacemaker or pacemakers receiving the message are adapted to respond to the message in a predetermined manner depending on type and location of the event.

In some embodiments and in predetermined conditions, an individual pacemaker **102** of the multiple leadless cardiac pacemakers can be configured to communicate to one or more other implanted pacemakers indication of the occurrence of a sensed heartbeat at the individual pacemaker location via generation of a coded pacing pulse triggered by the sensed heartbeat in a natural refractory period following the sensed heartbeat.

Multiple leadless cardiac pacemakers **102** can be configured for co-implantation in a single patient and multiple-chamber pacing, for example by defining logic internal to the pacemakers **102** at manufacture, by programming using an external programmer, or the like. Bidirectional communication among the multiple leadless cardiac pacemakers can be arranged to communicate notification of a sensed heartbeat or delivered pacing pulse event and encoding type and location of the event to another implanted pacemaker or pacemakers. The pacemaker or pacemakers receiving the communication decode the information and respond depending on location of the receiving pacemaker and predetermined system functionality.

Also shown in **FIGURE 1D****,** the primary battery **114** has positive terminal **140** and negative terminal **142.** A suitable primary battery has an energy density of at least 3 W_{·}h/cc, a power output of 70 microwatts, a volume less than 1 cubic centimeter, and a lifetime greater than 5 years.

One suitable primary battery uses beta-voltaic technology, licensed to BetaBatt Inc. of Houston, Texas, USA, and developed under a trade name DEC^{™} Cell, in which a silicon wafer captures electrons emitted by a radioactive gas such as tritium. The wafer is etched in a three-dimensional surface to capture more electrons. The battery is sealed in a hermetic package which entirely contains the low-energy particles emitted by tritium, rendering the battery safe for long-term human implant from a radiological-health standpoint. Tritium has a half-life of 12.3 years so that the technology is more than adequate to meet a design goal of a lifetime exceeding 5 years.

Current from the positive terminal **140** of primary battery **114** flows through a shunt **144** to a regulator circuit **146** to create a positive voltage supply **148** suitable for powering the remaining circuitry of the pacemaker **102.** The shunt **144** enables the battery current monitor **136** to provide the processor **112** with an indication of battery current drain and indirectly of device health.

The illustrative power supply can be a primary battery **114** such as a beta-voltaic converter that obtains electrical energy from radioactivity. In some embodiments, the power supply can be selected as a primary battery **114** that has a volume less than approximately 1 cubic centimeter.

In an illustrative embodiment, the primary battery **114** can be selected to source no more than 70 microwatts instantaneously since a higher consumption may cause the voltage across the battery terminals to collapse. Accordingly in one illustrative embodiment the circuits depicted in **FIGURE 1D** can be designed to consume no more than a total of 64 microwatts. The design avoids usage of a large filtering capacitor for the power supply or other accumulators such as a supercapacitor or rechargeable secondary cell to supply peak power exceeding the maximum instantaneous power capability of the battery, components that would add volume and cost.

In various embodiments, the system can manage power consumption to draw limited power from the battery, thereby reducing device volume. Each circuit in the system can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit is throttled to recharge the tank capacitor at constant power from the battery.

Implantable systems that communicate via long distance radio-frequency (RF) schemes, for example Medical Implant Communication Service (MICS) transceivers, which exhibit a peak power specification on the order of 10 milliwatts, and other RF or inductive telemetry schemes are unable to operate without use of an additional accumulator. Moreover, even with the added accumulator, sustained operation would ultimately cause the voltage across the battery to collapse.

Referring to **FIGURE 2****,** a schematic pictorial view shows an embodiment of the leadless cardiac pacemaker 102 that can be used with at least one other pacemaker in the cardiac pacing system **100.** The leadless cardiac pacemaker **102** comprises a hermetic housing **110** configured for placement on or attachment to the inside or outside of a cardiac chamber **104.** Two or more electrodes **108** abut or are adjacent to the housing **110.** The electrodes **108** are configured for delivering pacing pulses and receiving triggering signals from the other pulse generator **106.** The electrodes **108** can also sense electrical activity from cardiac chamber muscle.

Furthermore, the electrodes **108** are adapted for bidirectional communication with at least one other device within or outside the body. For example, the leadless pacemaker **102** can be configured to communicate with a non-implanted programmer or one or more implanted pulse generators via the same electrodes **108** that are used for delivering pacing pulses. The illustrative leadless pacemaker **102** is adapted for antenna-less and telemetry coil-less communication. Usage of the electrodes **108** for communication enables the leadless pacemaker **102** to communicate with a non-implanted programmer or one or more implanted pulse generators via communication that adds nothing to power requirements in addition to power requirements for cardiac pacing. For example, transmission from the leadless cardiac pacemaker **102** adds no power while reception adds on the order of 25 microwatts.

The illustrative example avoids usage of radiofrequency (RF) communication to send pacing instructions to remote electrodes on a beat-to-beat basis to cause the remote electrodes to emit a pacing pulse. RF communication involves use of an antenna and modulation/demodulation unit in the remote electrode, which increase implant size significantly. Also, communication of pacing instructions on a beat-to-beat basis increases power requirements for the main body and the remote electrode. In contrast, the illustrative system and stimulator do not require beat-to-beat communication with any controlling main body.

The illustrative leadless pacemaker **102** includes an internal power source that can supply all energy for operations and pulse generation. In contrast, some conventional implanted pulse generators have remote pacing electrodes that receive some or all energy from an energy source through an RF induction technique, an energy transfer scheme that employs a large loop antenna on the remote electrode that increases size significantly. In addition, energy transfer with the RF induction technique is inefficient and is associated with a significant increase in battery size of the energy source. In contrast, the illustrative leadless pacemaker **102** uses an internal battery and does not require energy to be drawn from outside sources. Also in the conventional system, the energy source receives sensing information by RF communication from the remote electrodes and sends pacing instructions to the electrodes on a beat-to-beat basis in a configuration that uses an addressing scheme in which the identity of specific remote pacing electrodes is stored in the energy source memory. The conventional method can also be inefficient due to overhead for transmitting an identification number from/to a generic pacing electrode at implant and/or during sensing. The illustrative leadless pacemaker **102** avoids such overhead through a structure in which pulse generation functionality is independent within a single implantable body.

Another conventional technology uses a system of addressable remote electrodes that stimulate body tissue without requiring a main body to send commands for individual stimulations. The remote electrodes are specified to be of a size and shape suitable for injection rather than for endocardial implantation. A controller can set operating parameters and send the parameters to remote electrodes by addressable communication, enabling the remote electrodes function relatively autonomously while incurring some overhead to controller operations. However, the remote electrodes do not sense or monitor cardiac information and rely on the main body to provide sensing functionality. In contrast, the illustrative leadless pacemaker **102** combines pacing and sensing of intrinsic cardiac activity in a single implantable body.

In some embodiments, the controller **112** in one leadless cardiac pacemaker **102** can access signals on the electrodes **108** and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller **112** can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

The illustrative leadless pacemaker **102** has one or more structures that enable fixture to tissue, for example suture holes **224, 225** or a helix **226.** The affixing structures enable implantation of the leadless pacemaker **102** directly to the cardiac muscle and with ligatures in procedures where the exterior surface of the heart can be accessed.

Also shown in **FIGURE 2****,** a cylindrical hermetic housing **110** is shown with annular electrodes **108** at housing extremities. In the illustrative embodiment, the housing **110** can be composed of alumina ceramic which provides insulation between the electrodes. The electrodes **108** are deposited on the ceramic, and are platinum or platinum-iridium.

Several techniques and structures can be used for attaching the housing **110** to the interior or exterior wall of cardiac chamber muscle **104.**

A helix **226** and slot **228** enable insertion of the device endocardially or epicardially through a guiding catheter. A screwdriver stylet can be used to rotate the housing **110** and force the helix **226** into muscle **104,** thus affixing the electrode **108A** in contact with stimulable tissue. Electrode **108B** serves as an indifferent electrode for sensing and pacing. The helix **226** may be coated for electrical insulation, and a steroid-eluting matrix may be included near the helix to minimize fibrotic reaction, as is known in conventional pacing electrode-leads.

In other configurations, suture holes **224** and **225** can be used to affix the device directly to cardiac muscle with ligatures, during procedures where the exterior surface of the heart is exposed.

Other attachment structures used with conventional cardiac electrode-leads including tines or barbs for grasping trabeculae in the interior of the ventricle, atrium, or coronary sinus may also be used in conjunction with or instead of the illustrative attachment structures.

Referring to **FIGURE 3****,** a pictorial view shows another embodiment of a single leadless cardiac pacemaker **102** that can be used in a cardiac pacing system **100** with at least one other pacemaker. The leadless cardiac pacemaker **102** includes a cylindrical metal housing **310** with an annular electrode **108A** and a second electrode **108B.** Housing **310** can be constructed from titanium or stainless steel. Electrode **108A** can be constructed using a platinum or platinum-iridium wire and a ceramic or glass feed-thru to provide electrical isolation from the metal housing. The housing can be coated with a biocompatible polymer such as medical grade silicone or polyurethane except for the region outlined by electrode **108B.** The distance between electrodes **108A** and **108B** should be approximately 1 cm to optimize sensing amplitudes and pacing thresholds. A helix **226** and slot **228** can be used for insertion ofthe device endocardially or epicardially through a guiding catheter. In addition, suture sleeves **302** and **303** made from silicone can be used to affix to the device directly to cardiac muscle with ligatures, for example in an epicardial or other application.

Referring to **FIGURE 4****,** a typical output-pulse waveform for a conventional pacemaker is shown. The approximately-exponential decay is due to discharge of a capacitor in the pacemaker through the approximately-resistive load presented by the electrodes and leads. Typically the generator output is capacitor-coupled to one electrode to ensure net charge balance. The pulse duration is shown as T0 and is typically 500 microseconds.

When the depicted leadless pacemaker **102** is used in combination with at least one other pacemaker or other pulse generator in the cardiac pacing system **100** and is generating a pacing pulse but is not optionally sending data for communication, the pacing waveform of the leadless pacemaker **102** can also resemble the conventional pacing pulse shown in **FIGURE 4****.**

Referring to **FIGURE 5****,** a time waveform graph depicts an embodiment of an output-pacing pulse waveform adapted for communication. The output-pulse waveform of the illustrative leadless pacemaker **102** is shown during a time when the pacemaker **102** is optionally sending data for communication and also delivering a pacing pulse, using the same pulse generator **116** and electrodes **108** for both functions.

**FIGURE 5** shows that the pulse generator **102** has divided the output pulse into shorter pulses **501, 502, 503, 504;** separated by notches **505, 506,** and **507.** The pulse generator **102** times the notches **505, 506,** and **507** to fall in timing windows W1, W2, and W4 designated **508, 509,** and **511** respectively. Note that the pacemaker **102** does not form a notch in timing window W3 designated **510.** The timing windows are each shown separated by a time T1, approximately 100 microseconds in the example.

As controlled by processor **112,** pulse generator **116** selectively generates or does not generate a notch in each timing window **508, 509, 510,** and **511** so that the device **102** encodes four bits of information in the pacing pulse. A similar scheme with more timing windows can send more or fewer bits per pacing pulse. The width of the notches is small, for example approximately 15 microseconds, so that the delivered charge and overall pulse width, specifically the sum of the widths of the shorter pulses, in the pacing pulse is substantially unchanged from that shown in **FIGURE 4****.** Accordingly, the pulse shown in **FIGURE 5** can have approximately the same pacing effectiveness as that shown in **FIGURE 4****,** according to the law of Lapique which is well known in the art of electrical stimulation.

In the leadless cardiac pacemaker **102,** a technique can be used to conserve power when detecting information carried on pacing pulses from other implanted devices. The leadless cardiac pacemaker **102** can have multiple gain settings on the receiving or sensing amplifier **132,** for example using a low-gain setting for normal operation. The low-gain setting could be insufficiently sensitive to decode gated information on a pacing pulse accurately, but could detect whether the pacing pulse is present. If an edge of a pacing pulse is detected during low-gain operation, the amplifier **132** can be switched quickly to the high-gain setting, enabling the detailed encoded data to be detected and decoded accurately. Once the pacing pulse has ended, the receiving amplifier **132** can be set back to low-gain. In the illustrative technique, a useful condition is that the receiving amplifier **132** shift to the more accurate high-gain quickly when called upon. To allow a maximum amount of time for shifting to occur, the encoded data can be placed at the end of the pacing pulse.

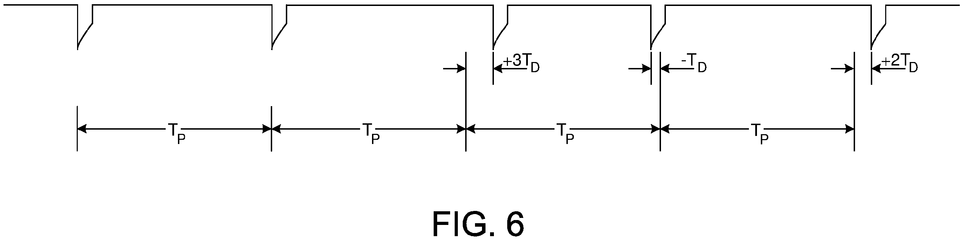

As an alternative or in addition to using notches in the stimulation pulse, the pulses can be generated with varying off-times, specifically times between pulses during which no stimulation occurs. The variation of off-times can be small, for example less than 10 milliseconds total, and can impart information based on the difference between a specific pulse's off-time and a preprogrammed off-time based on desired heart rate. For example, the device can impart four bits of information with each pulse by defining 16 off-times centered around the preprogrammed off-time. **FIGURE 6** is a graph showing a sample pulse generator output which incorporates a varying off-time scheme. In the figure, time Tₚ represents the preprogrammed pulse timing. Time T_{d} is the delta time associated with a single bit resolution for the data sent by the pulse generator. The number of T_{d} time increments before or after the moment specified by T_{P} gives the specific data element transmitted. The receiver of the pulse generator's communication has advance information of the time Tₚ. The communication scheme is primarily applicable to overdrive pacing in which time Tₚ is not dynamically changing or altered based on detected beats.

An aspect of proper functionality for a cardiac pacemaker is maintenance of a specified minimum internal supply voltage. When pacing tank capacitor charging occurs, the supply voltage can drop from a pre-charging level, a drop that can become more significant when the battery nears an end-of-life condition and has reduced current sourcing capability. Accordingly, in some implementations the leadless cardiac pacemaker **102** can be configured to stop charging the pacing tank capacitor when the supply voltage drops below a specified level. Accordingly, the processor **112** can be configured to control recharging of the tank capacitor so that recharging is discontinued when battery terminal voltage falls below a predetermined value, ensuring sufficient voltage for powering the leadless cardiac pacemaker circuitry. When charging ceases, the supply voltage returns to the value before charging began. In other implementations, the charge current can be lowered to prevent the supply voltage from dropping below the specified level, possibly creating difficulty in ensuring the same pacing rate or pacing pulse amplitude since the lower charge current results in the pacing tank taking longer to reach the desired voltage level.

**FIGURE 5** depicts a technique in which information is encoded in notches in the pacing pulse. **FIGURE 6** shows a technique of conveying information by modulating the off-time between pacing pulses. Alternatively or in addition to the two illustrative coding schemes, overall pacing pulse width can be used to impart information. For example, a paced atrial beat may exhibit a pulse width of 500 microseconds and an intrinsic atrial contraction can be identified by reducing the pulse width by 30 microseconds. Information can be encoded by the absolute pacing pulse width or relative shift in pulse width. Variations in pacing pulse width can be relatively small and have no impact on pacing effectiveness.

In some embodiments, a pacemaker **102** can use the leadless electrodes **108** to communicate bidirectionally among multiple leadless cardiac pacemakers and transmit data including designated codes for events detected or created by an individual pacemaker wherein the codes encode information using pacing pulse width.

The illustrative scheme for transmitting data does not significantly increase the current consumption of the pacemaker. For example, the pacemaker could transmit data continuously in a loop, with no consumption penalty.

The illustrative schemes for transmitting data enable assignment of designated codes to events detected or caused by a leadless cardiac pacemaker, such as sensing a heartbeat or delivering a pacing pulse at the location of the pacemaker that senses the event. Individual leadless cardiac pacemakers **102** in a system **100** can be configured, either at manufacture or with an external programmer as described above, to issue a unique code corresponding to the type of event and location of the leadless cardiac pacemaker. By delivery of a coded pacing pulse with a code assigned according to the pacemaker location, a leadless cardiac pacemaker can transmit a message to any and all other leadless cardiac pacemakers implanted in the same patient, where the code signifies the origin of the event. Each other leadless cardiac pacemaker can react appropriately to the conveyed information in a predetermined manner encoded in the internal processor **112,** as a function of the type and location of the event coded in the received pulse. A leadless cardiac pacemaker **102** can thus communicate to any and all other co-implanted leadless cardiac pacemakers the occurrence of a sensed heartbeat at the originating pacemaker's location by generating a coded pacing pulse triggered by the sensed event. Triggered pacing occurs in the natural refractory period following the heartbeat and therefore has no effect on the chamber where the leadless cardiac pacemaker is located.

Referring again to **FIGURE 1D****,** the circuit **132** for receiving communication via electrodes **108** receives the triggering information as described and can also optionally receive other communication information, either from the other implanted pulse generator **106** or from a programmer outside the body. This other communication could be coded with a pulse-position scheme as described in **FIGURE 5** or could otherwise be a pulse-modulated or frequency-modulated carrier signal, preferably from 10 kHz to 100 kHz. The illustrative scheme of a modulated carrier is applicable not only to intercommunication among multiple implanted pacemakers but also is applicable to communication from an external programmer or, in some configurations, a co-implanted ICD.

The illustrative leadless pacemaker **102** could otherwise receive triggering information from the other pulse generator **106** implanted within the body via a pulse-modulated or frequency-modulated carrier signal, instead of via the pacing pulses of the other pulse generator **106 .**

With regard to operating power requirements in the leadless cardiac pacemaker **102,** for purposes of analysis, a pacing pulse of 5 volts and 5 milliamps amplitude with duration of 500 microseconds and a period of 500 milliseconds has a power requirement of 25 microwatts.

In an example embodiment of the leadless pacemaker **102,** the processor **112** typically includes a timer with a slow clock that times a period of approximately 10 milliseconds and an instruction-execution clock that times a period of approximately 1 microsecond. The processor 112 typically operates the instruction-execution clock only briefly in response to events originating with the timer, communication amplifier **134,** or cardiac sensing amplifier **132.** At other times, only the slow clock and timer operate so that the power requirement of the processor 112 is no more than 5 microwatts.

For a pacemaker that operates with the aforementioned slow clock, the instantaneous power consumption specification, even for a commercially-available micropower microprocessor, would exceed the battery's power capabilities and would require an additional filter capacitor across the battery to prevent a drop of battery voltage below the voltage necessary to operate the circuit. The filter capacitor would add avoidable cost, volume, and potentially lower reliability.

For example, a microprocessor consuming only 100 microamps would require a filter capacitor of 5 microfarads to maintain a voltage drop of less than 0.1 volt, even if the processor operates for only 5 milliseconds. To avoid the necessity for such a filter capacitor, an illustrative embodiment of a processor can operate from a lower frequency clock to avoid the high instantaneous power consumption, or the processor can be implemented using dedicated hardware state machines to supply a lower instantaneous peak power specification.

In a pacemaker **102,** the cardiac sensing amplifier operates with a power consumption of no more than 5 microwatts. A communication amplifier that operates at a suitable frequency for usage in an implantable device, for example approximately 100 kHz, has a power consumption of no more than 25 microwatts in some embodiments. The battery ammeter **136** and battery voltmeter **138** operate at a power consumption of no more than 1 microwatt each. The pulse generator **116** typically includes an independent rate limiter with a power consumption of no more than 2 microwatts.

In conventional implantable devices, a communication amplifier and a sensing amplifier both continuously consume power, for example constantly requiring on the order of 25 microwatts and 5 microwatts respectively from the battery. In some embodiments of the implantable biostimulator described herein, operation of the communications amplifier and charging of the pacing tank capacitor can be made mutually exclusive. For example, after the pacing pulse, charging of the pacing tank capacitor can be suspended by an appropriate time window, for example 10 milliseconds. During the window, the communication amplifier can be enabled and ready to receive commands and information from an external programmer or another implantable device. Thus, the 25 microwatts used by the communications amplifier is mutually exclusive from the 25 microwatts consumed by charging the pacing tank capacitor, enabling the total power consumption of the biostimulator to drop to 39 microwatts.

The total power consumption of the pacemaker is thus 64 microwatts, less than the disclosed 70-microwatt battery output.

Improvement attained by the illustrative cardiac pacing system **100** and cardiac pacemaker **102** is apparent.

The illustrative cardiac pacing system **100** enables encoding outgoing communication in the pacing pulse, so that the total power consumption for outgoing communication and pacing does not exceed the power consumption for pacing alone. Thus, the power consumption for outgoing communication is effectively zero because outgoing communication uses the same power already used to create a pacing pulse.

The illustrative cardiac pacemaker **102** can have sensing and processing circuitry that consumes no more than 25 microwatts as in conventional pacemakers.

The described leadless cardiac pacemaker **102** can have an incoming communication amplifier for receiving communication which consumes no more than 25 microwatts.

Furthermore, the cardiac pacemaker **102** can have a primary battery, for example a beta-voltaic primary battery that produces sufficient power, for example 100 microwatts, in a suitably compact volume, for example less than one cubic centimeter. In addition, the leadless cardiac pacemaker **102** can have a primary battery that exhibits an energy density of at least 3W·h/cc.

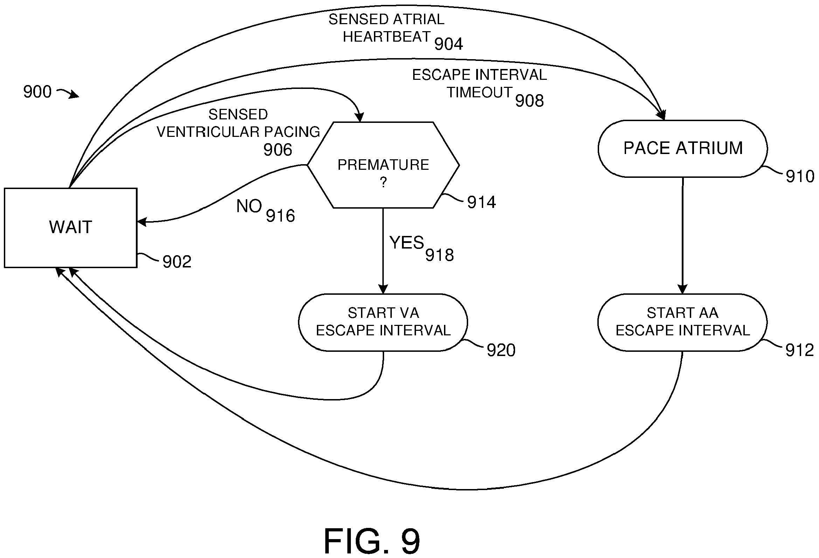

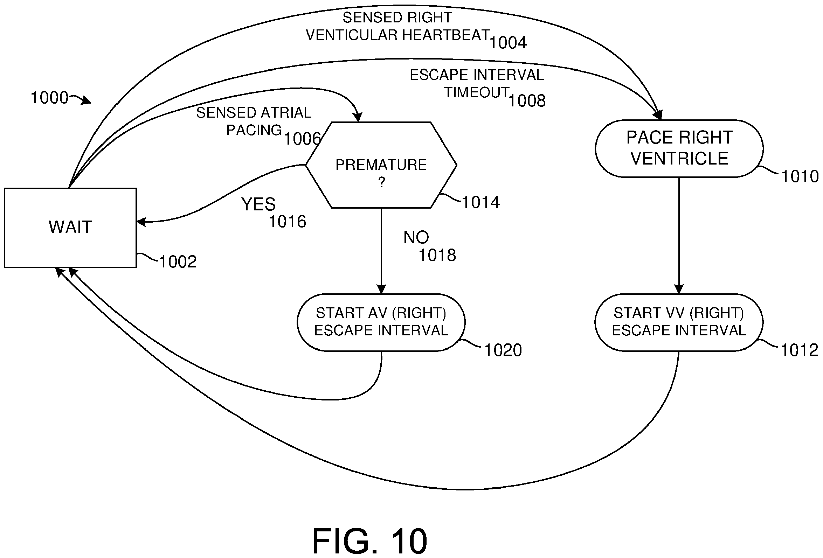

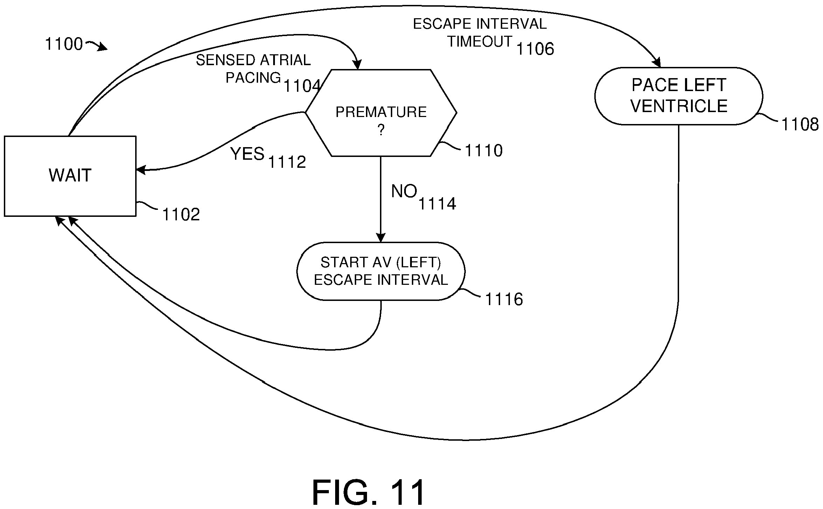

In an illustrative application of the cardiac pacing system **100,** multiple leadless cardiac pacemakers **102** can be co-implanted in a single patient to provide a system for dual-chamber pacing, CRT-P, or any other multi-chamber pacing application. Each leadless cardiac pacemaker in the system can use the illustrative communication structures to communicate the occurrence of a sensed heartbeat or a delivered pacing pulse at the location of sensing or delivery, and a communication code can be assigned to each combination of event type and location. Each leadless cardiac pacemaker can receive the transmitted information, and the code of the information can signify that a paced or sensed event has occurred at another location and indicate the location of occurrence. The receiving leadless cardiac pacemaker's processor **112** can decode the information and respond appropriately, depending on the location of the receiving pacemaker and the desired function of the system. **FIGUREs 9** and **10** are state diagrams that illustrate application of illustrative combined control operations in an atrial and right-ventricular leadless cardiac pacemaker respectively, to implement a simple dual-chamber pacing system when co-implanted. **FIGURE 11** is a state diagram that illustrates inclusion of a left-ventricular leadless cardiac pacemaker to form a CRT-P system.

In various embodiments, each leadless cardiac pacemaker may also encode other information destined for co-implanted leadless cardiac pacemakers, besides markers of paced or sensed events.

For clarity of illustration, descriptions of the atrial, right ventricular, and left-ventricular leadless cardiac pacemakers in respective **FIGUREs 9****,** **10****,** and **11** show only basic functions of each pacemaker. Other functions such as refractory periods, fallback mode switching, algorithms to prevent pacemaker-mediated tachycardia, and the like, can be added to the leadless cardiac pacemakers and to the system in combination. Also for clarity, functions for communication with an external programmer are not shown and are shown elsewhere herein.

Referring to **FIGURE 9****,** a state-mechanical representation shows operation of a leadless cardiac pacemaker for implantation adjacent to atrial cardiac muscle. As explained above, a leadless cardiac pacemaker can be configured for operation in a particular location and system either at manufacture or by an external programmer. Similarly, all individual pacemakers of the multiple pacemaker system can be configured for operation in a particular location and a particular functionality at manufacture and/or at programming by an external programmer wherein "configuring" means defining logic such as a state machine and pulse codes used by the leadless cardiac pacemaker.

In a cardiac pacing system, the multiple leadless cardiac pacemakers can comprise an atrial leadless cardiac pacemaker implanted in electrical contact to an atrial cardiac chamber. The atrial leadless cardiac pacemaker can be configured or programmed to perform several control operations **900** in combination with one or more other pacemakers. In a wait state **902** the atrial leadless cardiac pacemaker waits for an earliest occurring event of multiple events including a sensed atrial heartbeat **904,** a communication of an event sensed on the at least two leadless electrodes encoding a pacing pulse marking a heartbeat **906** at a ventricular leadless cardiac pacemaker, or timeout of an interval timed locally in the atrial leadless cardiac pacemaker shown as escape interval timeout **908.** The atrial pacemaker responds to a sensed atrial heartbeat **904** by generating **910** an atrial pacing pulse that signals to one or more other pacemakers that an atrial heartbeat has occurred, encoding the atrial pacing pulse with a code signifying an atrial location and a sensed event type. The atrial pacing pulse can be encoded using the technique shown in **FIGURE 5** with a unique code signifying the location in the atrium. After pacing the atrium, the atrial cardiac pacemaker times **912** a predetermined atrial-to-atrial (AA) escape interval. Accordingly, the atrial leadless cardiac pacemaker restarts timing **912** for a predetermined escape interval, called the AA (atrial to atrial) escape interval, which is the time until the next atrial pacing pulse if no other event intervenes. The atrial leadless cardiac pacemaker then re-enters the Wait state **902.** The atrial pacemaker also responds to timeout of a first occurring escape interval **908** by delivering an atrial pacing pulse **910,** causing an atrial heartbeat with the atrial pacing pulse encoding paced type and atrial location of an atrial heartbeat event. When the atrial escape interval times out, shown as transition **908,** the atrial leadless cardiac pacemaker delivers an atrial pacing pulse. Because no other atrial heartbeat has occurred during the duration of the escape interval, the atrial pacing pulse does not fall in the atria's natural refractory period and therefore should effectively pace the atrium, causing an atrial heartbeat. The atrial pacing pulse, coded in the manner shown in **FIGURE 5****,** also signals to any and all other co-implanted leadless cardiac pacemakers that an atrial heartbeat has occurred. If functionality is enhanced for a more complex system, the atrial leadless cardiac pacemaker can use a different code to signify synchronous pacing triggered by an atrial sensed event in comparison to the code used to signify atrial pacing at the end of an escape interval. However, in the simple example shown in **FIGUREs 9** and **10****,** the same code can be used for all atrial pacing pulses. In fact, for the simple dual-chamber pacing system described in **FIGUREs 9** and **10** encoding may be omitted because each leadless cardiac pacemaker can conclude that any detected pacing pulse, which is not generated locally, must have originated with the other co-implanted leadless cardiac pacemaker. After generating the atrial pacing pulse **910,** the atrial leadless cardiac pacemaker starts timing an atrial (AA) escape interval at action **912,** and then returns to the wait state **902.**

The atrial leadless cardiac pacemaker can further operate in response to another pacemaker. The atrial pacemaker can detect **906** a signal originating from a co-implanted ventricular leadless cardiac pacemaker. The atrial pacemaker can examine the elapsed amount of the atrial-to-atrial (AA) escape time interval since a most recent atrial heartbeat and determine 914 whether the signal originating from the co-implanted ventricular leadless cardiac pacemaker is premature. Thus, if the atrial leadless cardiac pacemaker detects a signal originating from a co-implanted ventricular leadless cardiac pacemaker, shown as sensed ventricular pacing **906,** then the atrial device examines the amount of the escape interval elapsed since the last atrial heartbeat at decision point 914 to determine whether the ventricular event is "premature", meaning too late to be physiologically associated with the last atrial heartbeat and in effect premature with respect to the next atrial heartbeat. In the absence 916 of a premature signal, the atrial pacemaker waits 902 for an event with no effect on atrial pacing. In contrast if the signal is premature **918,** the pacemaker restarts **920** a ventricle-to-atrium (VA) escape interval that is shorter than the atrial-to-atrial (AA) escape interval and is representative of a typical time from a ventricular beat to a next atrial beat in sinus rhythm, specifically the atrial interval minus the atrio-ventricular conduction time. After starting **920** the VA interval, the atrial leadless cardiac pacemaker returns to wait state **902,** whereby a ventricular premature beat can be said to "recycle" the atrial pacemaker. The pacemaker responds to timeout of the atrial-to-atrial (AA) escape interval **908** by delivering an atrial pacing pulse **910,** causing an atrial heartbeat. The atrial pacing pulse encodes the paced type and atrial location of an atrial heartbeat event.

The atrial leadless cardiac pacemaker can be further configured to time a prolonged post-ventricular atrial refractory period (PVARP) after recycling in presence of the premature signal, thereby preventing pacemaker-mediated tachycardia (PMT). Otherwise, if a received ventricular pacing signal evaluated at decision point **914** is not found to be premature, then the atrial leadless cardiac pacemaker follows transition **916** and re-enters the wait state **902** without recycling, thus without any effect on the timing of the next atrial pacing pulse.

Referring to **FIGURE 10****,** a state-mechanical representation depicts operation of a leadless cardiac pacemaker for implantation adjacent to right-ventricular cardiac muscle. The leadless cardiac pacemaker can be configured for operation in a particular location and system either at manufacture or by an external programmer. A system comprising multiple leadless cardiac pacemakers can include a right-ventricular leadless cardiac pacemaker implanted in electrical contact to a right-ventricular cardiac chamber. The right-ventricular leadless cardiac pacemaker can be configured to perform actions **1000** for coordinated pacing in combination with the other pacemakers. The right-ventricular leadless cardiac pacemaker waits **1002** for the earliest occurring event of multiple events including a sensed right-ventricular heartbeat **1004,** a sensed communication of a pacing pulse **1006** marking a heartbeat at an atrial leadless cardiac pacemaker, and timeout **1008** of an escape interval. Generally, the sensed communication of a pacing pulse **1006** can be any suitable sensed communication of an event originating at another co-implanted leadless cardiac pacemaker, in the illustrative embodiment a pacing pulse marking a heartbeat at an atrial leadless cardiac pacemaker shown as sensed atrial pacing. The escape interval timeout **1008** can be any suitable timeout of an interval timed locally in the right-ventricular leadless cardiac pacemaker.

The right-ventricular leadless cardiac pacemaker responds to the sensed right-ventricular heartbeat **1004** by generating **1010** a right-ventricular pacing pulse that signals to at least one other pacemaker of the multiple cardiac pacemakers that a right-ventricular heartbeat has occurred. Thus, when a sensed right-ventricular heartbeat occurs **1004,** the right-ventricular leadless cardiac pacemaker generates **1010** a right-ventricular pacing pulse, not to pace the heart but rather to signal to another leadless cardiac pacemaker or pacemakers that a right-ventricular heartbeat has occurred. The right-ventricular pacing pulse can be encoded with a code signifying the right-ventricular location and a sensed event type. The right-ventricular pacing pulse is coded in the manner shown in **FIGURE 5** with a unique code signifying the location in the right ventricle. Upon right-ventricular pacing pulse generation **1010,** the right-ventricular leadless cardiac pacemaker can time **1012** a predetermined right ventricular-to-right ventricular (VV) escape interval. The right-ventricular leadless cardiac pacemaker restarts **1012** timing of a predetermined escape interval, called the VV (right-ventricular to right-ventricular) escape interval, which is the time until the next right-ventricular pacing pulse if no other event intervenes. The VV escape interval is started after delivering a ventricular pacing pulse subsequent to various events including an atrial-ventricular (AV) delay, a ventricular-to-ventricular (VV) delay, or a ventricular sensed event.

The right-ventricular leadless cardiac pacemaker can further be configured to set the ventricular-to-ventricular (VV) escape interval longer than a predetermined atrial-to-atrial (AA) escape interval to enable backup ventricular pacing at a low rate corresponding to the VV escape interval in case of failure of a triggered signal from a co-implanted atrial leadless cardiac pacemaker. Typically, the VV (right) escape interval is longer than the AA interval depicted in **FIGURE 9****,** so that the system supports backup ventricular pacing at a relatively low rate in case of failure of the co-implanted atrial leadless cardiac pacemaker. In normal operation of the system, timeout of the VV interval never occurs. The right-ventricular leadless cardiac pacemaker then re-enters the Wait state **1002.**

The right-ventricular leadless cardiac pacemaker can respond to timeout of a first occurring escape interval **1008** by delivering **1010** a right ventricular pacing pulse, causing a right ventricular heartbeat. The right ventricular pacing pulse can encode information including paced type and right-ventricular location of a right ventricular heartbeat event.

When the right-ventricular escape interval times out **1008,** the right-ventricular leadless cardiac pacemaker delivers **1010** a right-ventricular pacing pulse. Because no other right-ventricular heartbeat has occurred during the duration of the VV escape interval, the pacing pulse **1010** does not fall in the ventricles' natural refractory period and therefore should effectively pace the ventricles, causing a ventricular heartbeat. The right-ventricular pacing pulse, coded in the manner shown in **FIGURE 5****,** also signals to any and all other co-implanted leadless cardiac pacemakers that a right-ventricular heartbeat has occurred. If useful for the function of a more complex system, the right-ventricular leadless cardiac pacemaker can use a different code to signify synchronous pacing triggered by a right-ventricular sensed event in comparison to the code used to signify right-ventricular pacing at the end of a VV escape interval. However, in the simple example shown in **FIGUREs 9** and **10****,** the same code can be used for all right-ventricular pacing pulses. In fact, for the simple dual-chamber pacing system described in **FIGUREs 9** and 10, a code may be omitted because each leadless cardiac pacemaker can conclude that any detected pacing pulse which is not generated local to the pacemaker originates with the other co-implanted leadless cardiac pacemaker. After generating **1010** the right-ventricular pacing pulse, the right-ventricular leadless cardiac pacemaker starts timing **1012** a right-ventricular escape interval VV, and then returns to the wait state **1002.**

The right-ventricular leadless cardiac pacemaker can further be configured to detect 1006 a signal originating from a co-implanted atrial leadless cardiac pacemaker. The right-ventricular leadless cardiac pacemaker examines the elapsed amount of the ventricular-to-ventricular (VV) escape interval since a most recent right-ventricular heartbeat and determines **1014** whether the signal originating from the co-implanted atrial leadless cardiac pacemaker is premature. An atrial event is defined as premature if too early to trigger an atrio-ventricular delay to produce a right-ventricular heartbeat. In the presence of a premature signal 1016, the right-ventricular leadless cardiac pacemaker returns to the wait state **1002** with no further action. Thus, a premature atrial beat does not affect ventricular pacing. In the absence of a premature signal **1018,** the right-ventricular leadless cardiac pacemaker starts **1020** a right atrium to right ventricular (AV) escape interval that is representative of a typical time from an atrial beat to a right-ventricular beat in sinus rhythm. Thus a non-premature atrial event leads to starting **1020** an AV (right) atrium to right-ventricular escape interval that represents a typical time from an atrial beat to a right-ventricular beat in normally-conducted sinus rhythm. After starting **1020** the AV interval, the right-ventricular leadless cardiac pacemaker returns to the wait state **1002** so that a non-premature atrial beat can "trigger" the right-ventricular pacemaker after a physiological delay. The right-ventricular leadless cardiac pacemaker also responds to timeout of either the W escape interval and the AV escape interval **1008** by delivering **1010** a right ventricular pacing pulse, causing a right ventricular heartbeat. The right ventricular pacing pulse encodes paced type and right-ventricular location of a right ventricular heartbeat event.

Accordingly, co-implanted atrial and right-ventricular leadless cardiac pacemakers depicted in **FIGUREs 9** and **10** cooperate to form a dual-chamber pacing system.

Referring to **FIGURE 11****,** a state-mechanical representation illustrates the operation of a leadless cardiac pacemaker for implantation adjacent to left-ventricular cardiac muscle. The left-ventricular cardiac pacemaker can be used in combination with the dual-chamber pacemaker that includes atrial leadless cardiac pacemaker and the right-ventricular leadless cardiac pacemaker described in **FIGUREs 9** and **10** respectively to form a system for CRT-P. A leadless cardiac pacemaker, for example the left-ventricular cardiac pacemaker, can be configured for operation in a particular location and system either at manufacture or by an external programmer.

A cardiac pacing system, such as a CRT-P system, can include multiple leadless cardiac pacemakers including a left-ventricular leadless cardiac pacemaker implanted in electrical contact to a left-ventricular cardiac chamber. The left-ventricular leadless cardiac pacemaker can execute operations of an illustrative pacing method **1100.** In a wait state **1102,** the left-ventricular cardiac pacemaker waits **1102** at the left-ventricular leadless cardiac pacemaker for an earliest occurring event of multiple events including a sensed communication **1104** of a pacing pulse marking a heartbeat at an atrial leadless cardiac pacemaker and timeout **1106** of a left ventricular escape interval. Generally, the sensed communication **1104** can be the sensed communication of an event originating at another co-implanted leadless cardiac pacemaker, in the illustrative embodiment a pacing pulse marking a heartbeat at an atrial leadless cardiac pacemaker shown as sensed atrial pacing. The escape interval timeout **1106** can be timeout of an interval timed locally in the left-ventricular leadless cardiac pacemaker. In the wait state **1102** for the left-ventricular leadless cardiac pacemaker, operation is simplified and the left-ventricular pacemaker does not respond to left-ventricular heartbeats. Also, the left-ventricular cardiac pacemaker does not pace the left ventricle in the absence of a triggering signal from the atrial leadless cardiac pacemaker. The left-ventricular cardiac pacemaker responds to timeout **1106** of the left ventricular escape interval by delivering **1108** a left ventricular pacing pulse, causing a left ventricular heartbeat. The left ventricular pacing pulse encodes the type and location of a left ventricular heartbeat event. The left-ventricular pacing pulse can be coded in the manner shown in **FIGURE 5** to communicate signals to any and all other co-implanted leadless cardiac pacemakers that a left-ventricular heartbeat has occurred, although such encoding is not necessary in the simplified CRT-P system shown in the described embodiment because the other leadless cardiac pacemakers do not react to left-ventricular pacing. After generating **1108** the left-ventricular pacing pulse, the left-ventricular leadless cardiac pacemaker returns to the wait state **1102.**

The left-ventricular leadless cardiac pacemaker can be further configured detect a signal originating from a co-implanted atrial leadless cardiac pacemaker and examine the elapsed amount of the left ventricular escape interval since a most recent left-ventricular heartbeat. The left-ventricular cardiac pacemaker can determine **1110** whether the signal originating from the co-implanted atrial leadless cardiac pacemaker is premature. If the left-ventricular leadless cardiac pacemaker detects sensed atrial pacing, then the left-ventricular device determines whether the atrial event is premature, meaning too early to trigger an atrio-ventricular delay to produce a left-ventricular heartbeat. In the presence of a premature signal **1112,** the left-ventricular cardiac pacemaker reverts to the wait state **1102** and waits for an event with no effect on ventricular pacing so that a premature atrial beat does not affect ventricular pacing. In absence of a premature signal **1114,** the left-ventricular cardiac pacemaker starts **1116** a left atrium to left ventricular (AV) escape interval that is representative of a typical time from an atrial beat to a left ventricular beat in normally-conducted sinus rhythm. As shown in the depicted embodiment, the AV (left) escape interval can have a different value from the AV (right) escape interval. After starting **1116** the AV interval, the left-ventricular leadless cardiac pacemaker returns to wait state **1102.** Accordingly, a non-premature atrial beat can "trigger" the left-ventricular pacemaker after a physiological delay.

The left-ventricular cardiac pacemaker also responds to timeout **1106** of the AV escape interval by delivering **1108** a left ventricular pacing pulse, causing a left ventricular heartbeat. The left ventricular pacing pulse encodes paced type and left ventricular location of a left ventricular heartbeat event.

In various embodiments, the multiple leadless cardiac pacemakers can comprise a right ventricular leadless cardiac pacemaker and a left ventricular leadless cardiac pacemaker that are configured to operate with atrio-ventricular (AV) delays whereby a left ventricular pacing pulse can be delivered before, after, or substantially simultaneously with a right ventricular pacing pulse. For example, multiple co-implanted leadless cardiac pacemakers that function according to the state diagrams shown in **FIGUREs 9****,** **10****,** and **11** can support CRT-P with left-ventricular pacing delivered before, at the same time as, or after right-ventricular pacing.

In various embodiments, multiple co-implanted leadless cardiac pacemakers can be configured for multi-site pacing that synchronizes depolarization for tachyarrhythmia prevention.

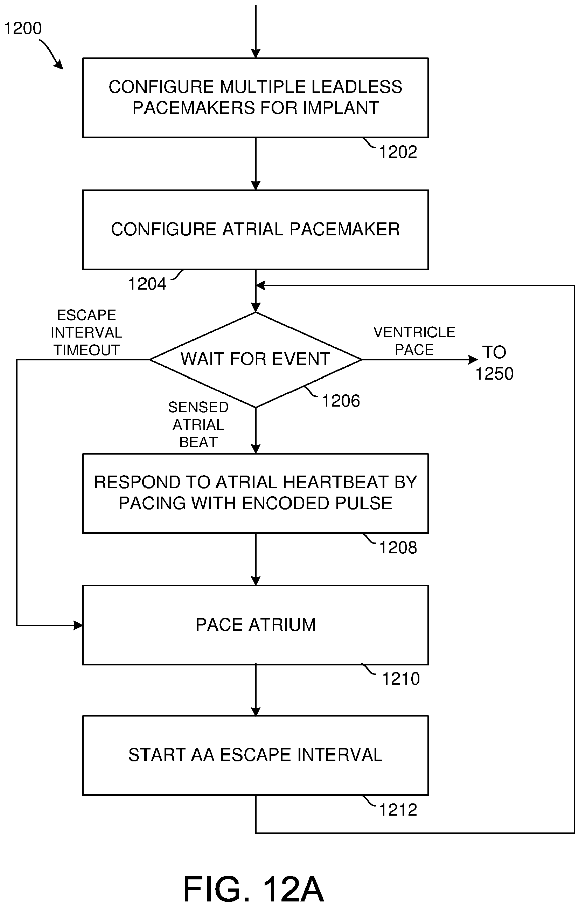

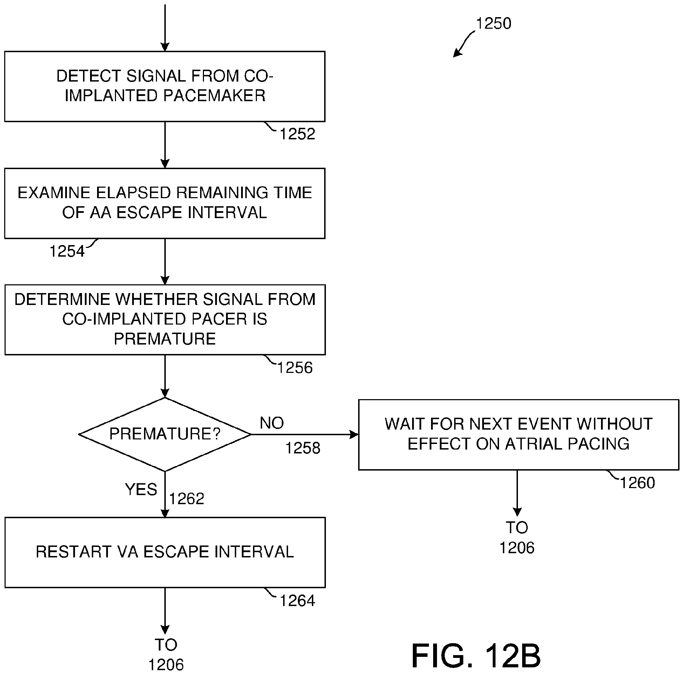

Referring to **FIGUREs 12A** and **12B****,** schematic flow charts illustrate an embodiment of a method for operating an atrial leadless cardiac pacemaker in multi-chamber cardiac pacing. **FIGURE 12A** depicts a method **1200** for multi-chamber cardiac pacing comprising configuring **1202** a multiple leadless cardiac pacemakers for implantation and configuring **1204** an atrial leadless cardiac pacemaker of the multiple leadless cardiac pacemakers for implantation in electrical contact to an atrial cardiac chamber. The atrial leadless cardiac pacemaker waits **1206** for an earliest occurring event of multiple events including a sensed atrial heartbeat, a communication of an event sensed on the at least two leadless electrodes encoding a pacing pulse marking a heartbeat at a ventricular leadless cardiac pacemaker, and timeout of an atrial-to-atrial (AA) escape interval. The atrial leadless cardiac pacemaker responds **1208** to the sensed atrial heartbeat by generating an atrial pacing pulse that signals to at least one pacemaker of the multiple leadless cardiac pacemakers that an atrial heartbeat has occurred and that encodes the atrial pacing pulse with a code signifying an atrial location and a sensed event type. After either a sensed atrial heartbeat or timeout of an escape interval, the atrial leadless cardiac pacemaker delivers **1210** an atrial pacing pulse, causing an atrial heartbeat and starts **1212** timing a predetermined length AA escape interval, then waiting **1206** for an event. The atrial pacing pulse identifies paced type and/or atrial location of an atrial heartbeat event.

In some embodiments, the atrial leadless cardiac pacemaker can encode an atrial pacing pulse that identifies synchronous pacing triggered by an atrial sensed event with a first code and encode an atrial pacing pulse that identifies atrial pacing following the AA escape interval with a second code distinct from the first code.

In some embodiments or conditions, the atrial leadless cardiac pacemaker can deliver the atrial pacing pulse in absence of encoding whereby, for dual-chamber cardiac pacing, a pacing pulse that is not generated in a first cardiac pacemaker that senses the pacing pulse is necessarily generated in a second cardiac pacemaker. Accordingly, neither the use of a code to identify the chamber corresponding to a pacing pulse, nor the use of a code to identify the type of pulse (whether paced or sensed) is a necessary step in a simple system such as a dual chamber pacing system disclosed in the specification.

The atrial leadless cardiac pacemaker can, upon delivery of an atrial pacing pulse, time an atrial-to-atrial (AA) escape interval.

**FIGURE 12B** is a flow chart showing another aspect of an embodiment of a method **1250** for operating an atrial leadless cardiac pacemaker. The atrial leadless cardiac pacemaker detects **1252** a signal originating from a co-implanted ventricular leadless cardiac pacemaker and examines **1254** an elapsed amount of the atrial-to-atrial (AA) escape interval since a most recent atrial heartbeat, determining **1256** whether the signal originating from the co-implanted ventricular leadless cardiac pacemaker is premature. In absence of a premature signal **1258,** the atrial leadless cardiac pacemaker waits **1260** for an event with no effect on atrial pacing, returning to wait state **1206.** In presence of a premature signal **1262,** the atrial leadless cardiac pacemaker restarts **1264** a ventricle-to-atrium (VA) escape interval that is shorter than the atrial-to-atrial (AA) escape interval and representative of a typical time from a ventricular beat to a next atrial beat in sinus rhythm, then returns to wait state **1206.**

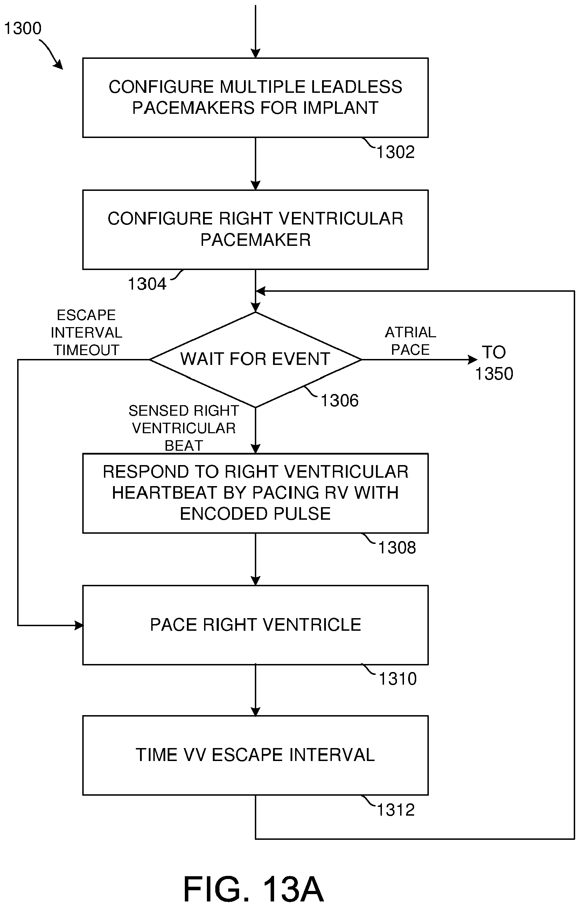

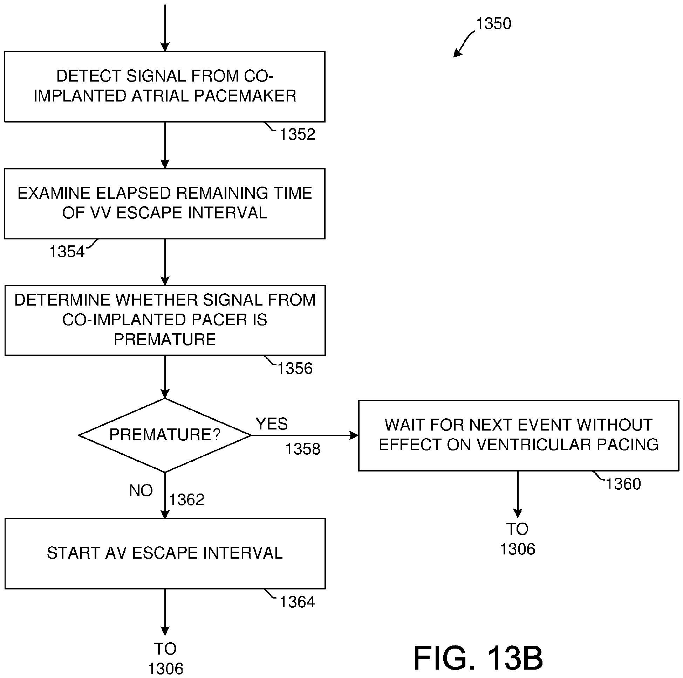

Referring to **FIGUREs 13A** and **13B****,** schematic flow charts illustrate an embodiment of a method for operating a right-ventricular leadless cardiac pacemaker in multi-chamber cardiac pacing. **FIGURE 13A** depicts a method **1300** for multi-chamber cardiac pacing comprising configuring **1302** a plurality of leadless cardiac pacemakers for implantation and configuring **1304** a right-ventricular leadless cardiac pacemaker of the multiple leadless cardiac pacemakers for implantation in electrical contact to a right-ventricular cardiac chamber. The right-ventricular leadless cardiac pacemaker waits **1306** for an earliest occurring event of a several events including a sensed right-ventricular heartbeat, a sensed communication of a pacing pulse marking a heartbeat at an atrial leadless cardiac pacemaker, and timeout of an escape interval. The right-ventricular leadless cardiac pacemaker responds **1308** to the sensed right-ventricular heartbeat by generating a right-ventricular pacing pulse that signals to at least one pacemaker of the leadless cardiac pacemakers that a right-ventricular heartbeat has occurred and that encodes the right-ventricular pacing pulse with a code signifying a right-ventricular location and a sensed event type. The right-ventricular leadless cardiac pacemaker responds **1310** to timeout of a first occurring escape interval by delivering a right ventricular pacing pulse, causing a right ventricular heartbeat, with the right ventricular pacing pulse encoding paced type and right ventricular location of a right ventricular heartbeat event, and times **1312** a predetermined ventricular-to-ventricular (VV) escape interval.

In some embodiments, the right-ventricular leadless cardiac pacemaker can encode a right-ventricular pacing pulse that identifies synchronous pacing triggered by a right-ventricular sensed event with a first code and encode a right-ventricular pacing pulse that identifies right-ventricular pacing following a ventricular-to-ventricular (VV) escape interval with a second code distinct from the first code.

In some embodiments, the right-ventricular leadless cardiac pacemaker, upon delivery of a right-ventricular pacing pulse, can time a ventricular-to-ventricular (VV) escape interval.

**FIGURE 13B** is a flow chart showing another aspect of an embodiment of a method **1350** for operating a right-ventricular leadless cardiac pacemaker. The right-ventricular leadless cardiac pacemaker detects **1352** a signal originating from a co-implanted atrial leadless cardiac pacemaker, examines **1354** the elapsed amount of the ventricular-to-ventricular (VV) escape interval since a most recent right-ventricular heartbeat, and determines **1356** whether the signal originating from the co-implanted atrial leadless cardiac pacemaker is premature. In presence **1358** of a premature signal, the right-ventricular leadless cardiac pacemaker waits **1360** for an event with no effect on ventricular pacing, returning to wait state **1306.** In absence **1362** of a premature signal, the right-ventricular leadless cardiac pacemaker starts **1364** a right atrium to right ventricular (AV) escape interval that is representative of a typical time from an atrial beat to a right-ventricular beat in sinus rhythm, and then returns to the wait state **1306.**

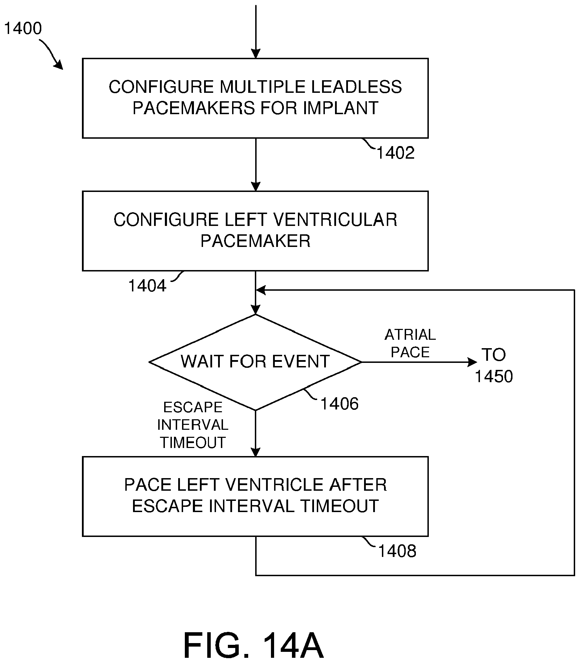

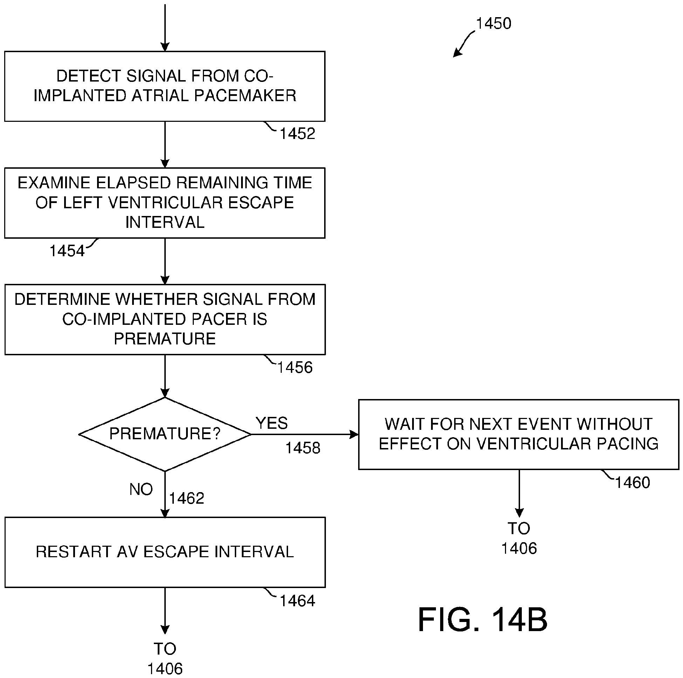

Referring to **FIGUREs 14A** and **14B****,** schematic flow charts illustrate embodiments of a method for operating a left-ventricular leadless cardiac pacemaker in multi-chamber cardiac pacing. **FIGURE 14A** depicts a method **1400** for multi-chamber cardiac pacing comprising configuring **1402** a plurality of leadless cardiac pacemakers for implantation and configuring **1404** a left-ventricular leadless cardiac pacemaker of the leadless cardiac pacemaker plurality for implantation in electrical contact to a left-ventricular cardiac chamber and for operation in cardiac resynchronization therapy (CRT-P). The left-ventricular cardiac pacemaker waits **1406** at the left-ventricular leadless cardiac pacemaker for an earliest occurring event of a plurality of events comprising a sensed communication of a pacing pulse marking a heartbeat at an atrial leadless cardiac pacemaker and timeout of a left ventricular escape interval. The left-ventricular cardiac pacemaker responds **1408** to timeout of the left ventricular escape interval by delivering a left ventricular pacing pulse, causing a left ventricular heartbeat, the left ventricular pacing pulse encoding type and location of a left ventricular heartbeat event.

In some embodiments, the left-ventricular cardiac pacemaker can configure the left-ventricular leadless cardiac pacemaker for operation in cardiac resynchronization therapy (CRT-P).

**FIGURE 14B** is a flow chart showing another aspect of an embodiment of a method 1450 for operating a left-ventricular leadless cardiac pacemaker. The left-ventricular leadless cardiac pacemaker detects **1452** a signal originating from a co-implanted atrial leadless cardiac pacemaker, examines **1454** the elapsed amount of the left ventricular escape interval since a most recent left-ventricular heartbeat, and determines **1456** whether the signal originating from the co-implanted atrial leadless cardiac pacemaker is premature. In the presence **1458** of a premature signal, the left-ventricular cardiac pacemaker waits **1460** for an event with no effect on ventricular pacing. In the absence **1462** of a premature signal, the left-ventricular cardiac pacemaker starts **1464** a left atrium to left ventricular (AV) escape interval that is representative of a typical time from an atrial beat to a left ventricular beat in sinus rhythm.

In some embodiments of an illustrative cardiac pacing system, one or more leadless cardiac pacemakers with low-power conducted communication can perform single-chamber pacing, dual-chamber pacing, CRT-D, or other pacing, co-implanted with an ICD, enabling functionality extending beyond what is possible or appropriate for conventional subcutaneous **ICDs.**

A system of leadless cardiac pacemakers enables pacing in conjunction with an implantable cardioverter-defibrillator (ICD) for usage in single-chamber, dual-chamber, CRT-D, and other multi-chamber cardiac pacing schemes.

Various embodiments of a system of an implantable cardioverter-defibrillator (ICD) and one or more leadless cardiac pacemakers are described. The individual leadless cardiac pacemakers can be substantially enclosed in a hermetic housing suitable for placement on or attachment to the inside or outside of a cardiac chamber. The pacemaker can have at least two electrodes located within, on, or near the housing, for delivering pacing pulses to and sensing electrical activity from the muscle of the cardiac chamber, and for bidirectional communication with at least one other co-implanted leadless cardiac pacemaker and optionally with another device outside the body. The housing can contain a primary battery to provide power for pacing, sensing, and communication. The housing can also contain circuits for sensing cardiac activity from the electrodes, receiving information from at least one other device via the electrodes, generating pacing pulses for delivery via the electrodes, transmitting information to at least one other device via the electrodes, monitoring device health, and controlling these operations in a predetermined manner.

A cardiac pacing system includes cardiac pacing in conjunction with an ICD and can supplement the functionality of ICDs with cardiac pacing functions, extending beyond functionality of conventional ICD-pacing arrangements.

The cardiac pacing system comprises a leadless cardiac pacemaker or pacemakers adapted to perform cardiac pacing functions with a co-implanted ICD, without a pacing electrode-lead separate from the leadless cardiac pacemaker, without a communication coil or antenna in the leadless cardiac pacemaker, and without an additional requirement on battery power in the leadless cardiac pacemaker for transmitted communication.

In some embodiments, a cardiac pacing system comprises an ICD with one or more leadless pacemakers for implantation adjacent to the inside or outside wall of a cardiac chamber, without the need for a connection between the leadless pulse generator and an electrode lead that can be connected or disconnected during implantation and repair procedures, and without the need for a lead body.

In some embodiments of a cardiac pacing system, communication between the implanted leadless cardiac pacemaker or pacemakers and other devices, including any co-implanted leadless cardiac pacemakers, the co-implanted ICD, and optionally a device external to the body, uses conducted communication via the same electrodes used for pacing, without the need for an antenna or telemetry coil.

Some embodiments and/or arrangements can implement communication between an implanted leadless cardiac pacemaker and other devices with power requirements similar to those for cardiac pacing, to enable optimization of battery performance. For example, transmission from the leadless cardiac pacemaker adds no power while reception adds a limited amount of power, such as about 25 microwatt.

A cardiac pacemaker or pacemakers are adapted for implantation in the human body. In a specific embodiment, one or more leadless cardiac pacemakers can be co-implanted with an implantable cardioverter-defibrillator (ICD). Each leadless cardiac pacemaker uses two or more electrodes located within, on, or within two centimeters of the housing of the pacemaker, for pacing and sensing at the cardiac chamber, for bidirectional communication with the ICD, optionally with at least one other leadless cardiac pacemaker, and optionally with at least one other device outside the body.

Referring to **FIGURE 1C**, a pictorial diagram shows an embodiment of a cardiac pacing system **100** including one or more leadless cardiac pacemakers **102** with conducted communication for performing cardiac pacing in conjunction with an implantable cardioverter-defibrillator (ICD) **106.** The system **100** can implement for example single-chamber pacing, dual-chamber pacing, or three-chamber pacing for cardiac resynchronization therapy, without requiring pacing lead connections to the defibrillator **106.** The illustrative cardiac pacing system **100** comprises at least one leadless cardiac pacemaker **102** configured for implantation in electrical contact with a cardiac chamber **104** and configured to perform cardiac pacing functions in combination with a co-implanted implantable cardioverter-defibrillator (ICD) **106.** One or more of the leadless cardiac pacemakers **102** can comprise at least two leadless electrodes **108** configured for delivering cardiac pacing pulses, sensing evoked and/or natural cardiac electrical signals, and uni-directionally or bi-directionally communicating with the co-implanted ICD **106.**

The leadless cardiac pacemakers **102** can communicate with one another and/or communicate with a non-implanted programmer and/or the implanted ICD **106** via the same electrodes **108** that are also used to deliver pacing pulses. Usage of the electrodes **108** for communication enables the one or more leadless cardiac pacemakers **102** for antenna-less and telemetry coil-less communication.

The leadless cardiac pacemakers **102** can be configured to communicate with one another and to communicate with a non-implanted programmer **106** via communication that has outgoing communication power requirements essentially met by power consumed in cardiac pacing.

In some embodiments, the individual leadless cardiac pacemaker **102** can comprise a hermetic housing **110** configured for placement on or attachment to the inside or outside of a cardiac chamber **104** and at least two leadless electrodes **108** proximal to the housing **110** and configured for bidirectional communication with at least one other device **106** within or outside the body.

The one or more leadless electrodes **108** can be configured to communicate bidirectionally among the multiple leadless cardiac pacemakers and/or the implanted ICD to coordinate pacing pulse delivery using messages that identify an event at an individual pacemaker originating the message and a pacemaker receiving the message react as directed by the message depending on the origin of the message. A pacemaker or pacemakers that receive the message react as directed by the message depending on the message origin or location. In some embodiments or conditions, the two or more leadless electrodes **108** can be configured to communicate bidirectionally among the one or more leadless cardiac pacemakers **102** and/or the ICD **106** and transmit data including designated codes for events detected or created by an individual pacemaker. Individual pacemakers can be configured to issue a unique code corresponding to an event type and a location of the sending pacemaker.

In some embodiments, information encoded on the leadless cardiac pacemakers can be used to enhance the sensitivity and specificity of the ICD such as, for example, a subcutaneous-only implantable defibrillator. Illustratively, a subcutaneously-only defibrillator senses only far-field signals, making difficult extraction of atrial information as well as uniquely identifying atrial depolarization from ventricular depolarization. When a subcutaneous-only defibrillator is used in combination with one or more leadless cardiac pacemakers, the information derived from the pacing pulse for each leadless pacemaker can be gathered and used to identify atrial and ventricular depolarization without ambiguity.

The leadless cardiac pacemaker **102** can communicate the information listed hereinabove with the implanted ICD **106,** or with a programmer outside the body, or both.

For example, in some embodiments an individual pacemaker **102** of the one or more leadless cardiac pacemakers can be configured to deliver a coded pacing pulse with a code assigned according to pacemaker location and configured to transmit a message to one or more other leadless cardiac pacemakers via the coded pacing pulse wherein the code identifies the individual pacemaker originating an event. The pacemaker or pacemakers receiving the message are adapted to respond to the message in a predetermined manner depending on type and location of the event.

In some embodiments or conditions, individual pacemakers **102** can deliver a coded pacing pulse with a code assigned according to pacemaker location and configured to transmit a message to at least one of the leadless cardiac pacemakers via the coded pacing pulse wherein the code identifies the individual pacemaker originating an event. The individual pacemakers can be further configured to deliver a pacing pulse in absence of encoding whereby, for dual-chamber cardiac pacing, a pacing pulse that is not generated in a first cardiac pacemaker that senses the pacing pulse is necessarily generated in a second cardiac pacemaker. Accordingly, neither the use of a code to identify the chamber corresponding to a pacing pulse, nor the use of a code to identify the type of pulse (whether paced or sensed) is a necessary step in a simple system such as a dual chamber pacing system disclosed in the specification.

Moreover, information communicated on the incoming channel can also include a message from another leadless cardiac pacemaker signifying that the other leadless cardiac pacemaker has sensed a heartbeat or has delivered a pacing pulse, and identifies the location of the other pacemaker. Similarly, information communicated on the outgoing channel can also include a message to another leadless cardiac pacemaker or pacemakers, or to the ICD, that the sending leadless cardiac pacemaker has sensed a heartbeat or has delivered a pacing pulse at the location of the sending pacemaker.

In some embodiments and in predetermined conditions, an individual pacemaker 102 of the one or more leadless cardiac pacemakers can be configured to communicate to one or more other implanted pacemakers indication of the occurrence of a sensed heartbeat at the individual pacemaker location via generation of a coded pacing pulse triggered by the sensed heartbeat in a natural refractory period following the sensed heartbeat.

Referring again to **FIGUREs 1C** and **1D****,** in various embodiments a cardiac pacing system **100** comprises at least one leadless cardiac pacemaker **102** that is configured for implantation in electrical contact with a cardiac chamber **104** and configured to perform cardiac pacing functions in combination with a co-implanted implantable cardioverter-defibrillator (ICD) **106.**

An embodiment of a cardiac pacing system **100** comprises an implantable cardioverter-defibrillator (ICD) **106** and at least one leadless cardiac pacemaker **102** configured for implantation in electrical contact with a cardiac chamber and for performing cardiac rhythm management functions in combination with the implantable ICD **106.** The implantable ICD **106** and the one or more leadless cardiac pacemakers **102** configured for leadless intercommunication by information conduction through body tissue.

In another embodiment, a cardiac pacing system **100** comprises one or more leadless cardiac pacemaker **102** or pacemakers configured for implantation in electrical contact with a cardiac chamber **104** and configured to perform cardiac pacing functions in combination with a co-implanted implantable cardioverter-defibrillator (ICD) **106.** The leadless cardiac pacemaker or pacemakers **102** are configured to intercommunicate and/or to communicate with a non-implanted programmer and/or the implanted ICD **106** via two or more electrodes **108** that are used for delivering pacing pulses. The pacemakers **102** are configured for antenna-less and telemetry coil-less communication.

In a further embodiment, a cardiac pacing system **100** comprises at least one leadless cardiac pacemaker **102** configured for implantation in electrical contact with a cardiac chamber **104** and configured to perform cardiac pacing functions in combination with a co-implanted implantable cardioverter-defibrillator (ICD) **106.** The leadless cardiac pacemaker or pacemakers **102** comprise at least two leadless electrodes **108** configured for delivering cardiac pacing pulses, sensing evoked and/or natural cardiac electrical signals, and transmitting information to the co-implanted ICD **106.**

In another example embodiment, a cardiac pacing system **100** comprises at least one leadless cardiac pacemaker **102** configured for implantation in electrical contact with a cardiac chamber **104** and configured to perform cardiac pacing functions in combination with a co-implanted implantable cardioverter-defibrillator (ICD) **106.** The leadless cardiac pacemaker or pacemakers **102** comprising at least two leadless electrodes **108** configured for delivering cardiac pacing pulses, sensing evoked and/or natural cardiac electrical signals, and receiving information from the co-implanted ICD **106.**

As shown in the illustrative embodiments, a leadless cardiac pacemaker **102** can comprise two or more leadless electrodes **108** configured for delivering cardiac pacing pulses, sensing evoked and/or natural cardiac electrical signals, and bidirectionally communicating with the co-implanted ICD **106.** A leadless cardiac pacemaker **102** can be configured to communicate with other pacemakers and/or communicate with a non-implanted programmer via communication that has communication power requirements essentially met by power consumed in cardiac pacing. For example, the leadless cardiac pacemaker **102** can be configured to communicate with other pacemakers and with a non-implanted programmer via communication that has negligible transmission power requirements in addition to power consumed in cardiac pacing.

Individual pacemakers of the one or more leadless cardiac pacemakers **102** can be configured for operation in a particular location and a particular functionality at manufacture and/or at programming by an external programmer. Bidirectional communication among the multiple leadless cardiac pacemakers can be arranged to communicate notification of a sensed heartbeat or delivered pacing pulse event and encoding type and location of the event to another implanted pacemaker or pacemakers. The pacemaker or pacemakers receiving the communication decode the information and respond depending on location of the receiving pacemaker and predetermined system functionality.

In some embodiments, individual pacemakers **102** of the one or more leadless cardiac pacemakers can be configured to receive conducted communication from a co-implanted cardioverter-defibrillator (ICD) **106** that configures the pacemakers **102** to deliver overdrive anti-tachycardia pacing in response to a detected tachyarrhythmia.

In some embodiments, the controller **112** in one leadless cardiac pacemaker **102** can access signals on the electrodes **108** and can examine output pulse duration from another pacemaker for usage as a signature for determining triggering information validity and, for a signature arriving within predetermined limits, activating delivery of a pacing pulse following a predetermined delay of zero or more milliseconds. The predetermined delay can be preset at manufacture, programmed via an external programmer, or determined by adaptive monitoring to facilitate recognition of the triggering signal and discriminating the triggering signal from noise. In some embodiments or in some conditions, the controller **112** can examine output pulse waveform from another leadless cardiac pacemaker for usage as a signature for determining triggering information validity and, for a signature arriving within predetermined limits, activating delivery of a pacing pulse following a predetermined delay of zero or more milliseconds.

In an illustrative application of the cardiac pacing system **100,** one or more leadless cardiac pacemakers **102** can be co-implanted with an ICD **106** in a single patient to provide a system for single-chamber pacing, dual-chamber pacing, CRT-D, or any other multi-chamber pacing application. Each leadless cardiac pacemaker in the system can use the illustrative communication structures to communicate the occurrence of a sensed heartbeat or a delivered pacing pulse at the location of sensing or delivery, and a communication code can be assigned to each combination of event type and location. Each leadless cardiac pacemaker can receive the transmitted information, and the code of the information can signify that a paced or sensed event has occurred at another location and indicate the location of occurrence. The receiving leadless cardiac pacemaker's processor **112** can decode the information and respond appropriately, depending on the location of the receiving pacemaker and the desired function of the system.

The implanted cardioverter-defibrillator (ICD) **106** can comprise a case and be fitted with a pair of electrodes mounted on or near the case. The ICD **106** can be configured to receive and transmit conducted communication using a pulse modulated or frequency modulated carrier signal whereby the ICD **106** can detect communication pulses from co-implanted leadless cardiac pacemakers **102** and transmit programming information to the co-implanted leadless cardiac pacemakers **102.** In some embodiments, an implanted cardioverter-defibrillator (ICD) **106** configured to receive conducted communication using two implantable electrodes.

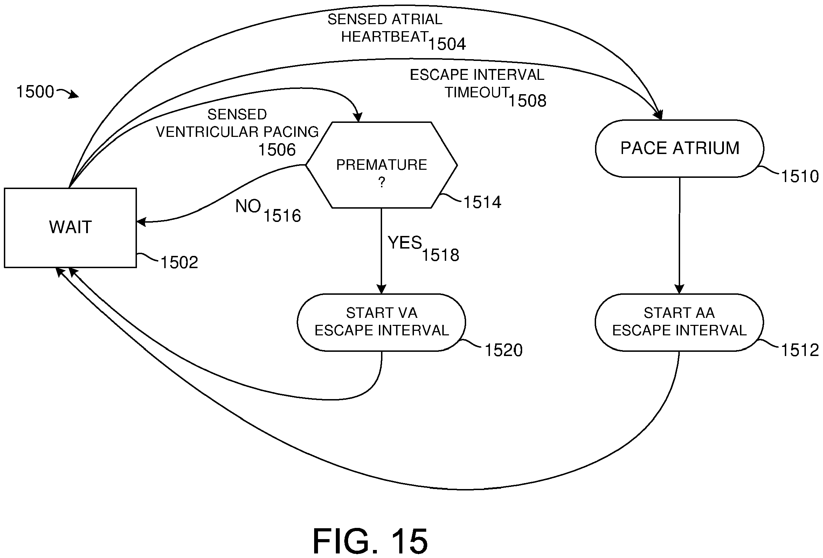

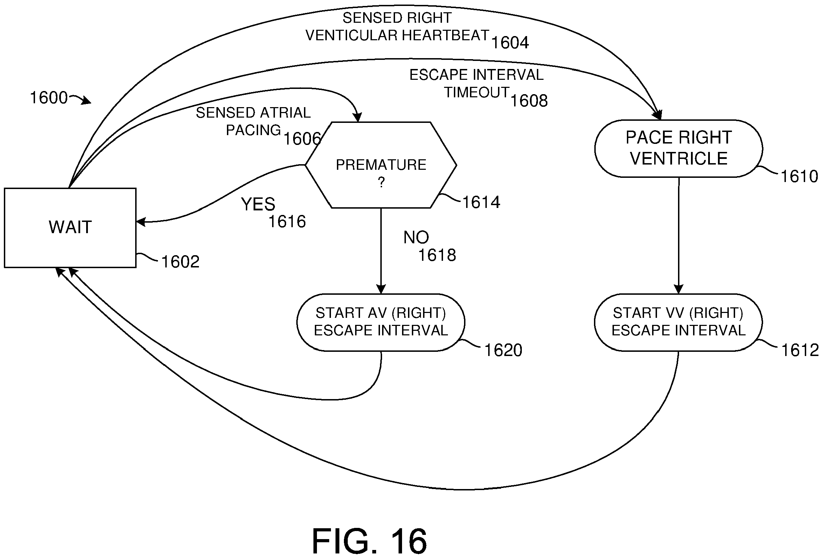

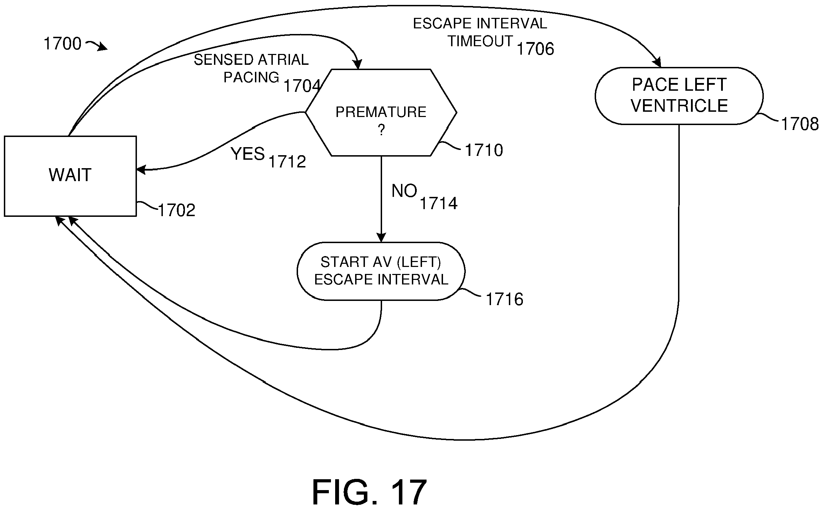

**FIGUREs 15** and **16** are state diagrams that illustrate application of illustrative combined control operations in an atrial and right-ventricular leadless cardiac pacemaker respectively, to implement a simple dual-chamber pacing system when co-implanted with an ICD **106.** **FIGURE 17** is a state diagram that illustrates inclusion of a left-ventricular leadless cardiac pacemaker to form a CRT-D system. In various embodiments, each leadless cardiac pacemaker may also broadcast other information destined for co-implanted leadless cardiac pacemakers and the co-implanted ICD, besides markers of paced or sensed events.

For clarity of illustration, descriptions of the atrial, right ventricular, and left-ventricular leadless cardiac pacemakers in respective **FIGUREs 15****,** **16****,** and **17** show only basic functions of each pacemaker. Other functions such as refractory periods, fallback mode switching, algorithms to prevent pacemaker-mediated tachycardia, and the like, can be added to the leadless cardiac pacemakers and to the system in combination. Also for clarity, functions for communication with an external programmer are not shown and are shown elsewhere herein.

Referring to **FIGURE 15****,** a state-mechanical representation shows operation of a leadless cardiac pacemaker for implantation adjacent to atrial cardiac muscle. As explained above, a leadless cardiac pacemaker can be configured for operation in a particular location and system either at manufacture or by an external programmer. Similarly, all individual pacemakers of the multiple pacemaker system can be configured for operation in a particular location and a particular functionality at manufacture and/or at programming by an external programmer wherein "configuring" means defining logic such as a state machine and pulse codes used by the leadless cardiac pacemaker.

In a cardiac pacing system, the multiple leadless cardiac pacemakers can comprise an atrial leadless cardiac pacemaker implanted in electrical contact to an atrial cardiac chamber. The atrial leadless cardiac pacemaker can be configured or programmed to perform several control operations **1500** in combination with one or more other pacemakers. In a wait state **1502** the atrial leadless cardiac pacemaker waits for an earliest occurring event of multiple events including a sensed atrial heartbeat **1504,** a communication of an event sensed on the at least two leadless electrodes encoding a pacing pulse marking a heartbeat **1506** at a ventricular leadless cardiac pacemaker, or timeout of an interval timed locally in the atrial leadless cardiac pacemaker shown as escape interval timeout **1508.** The atrial pacemaker responds to a sensed atrial heartbeat **1504** by generating **1510** an atrial pacing pulse that signals to one or more other pacemakers and optionally to the co-implanted ICD that an atrial heartbeat has occurred, encoding the atrial pacing pulse with a code signifying an atrial location and a sensed event type. The atrial pacing pulse can be encoded using the technique shown in **FIGURE 5** with a unique code signifying the location in the atrium. After pacing the atrium, the atrial cardiac pacemaker times **1512** a predetermined atrial-to-atrial (AA) escape interval. Accordingly, the atrial leadless cardiac pacemaker restarts timing **1512** for a predetermined escape interval, called the AA (atrial to atrial) escape interval, which is the time until the next atrial pacing pulse if no other event intervenes. The atrial leadless cardiac pacemaker then re-enters the Wait state **1502.** The atrial pacemaker also responds to timeout of a first occurring escape interval **1508** by delivering an atrial pacing pulse **1510,** causing an atrial heartbeat with the atrial pacing pulse encoding paced type and atrial location of an atrial heartbeat event. When the atrial escape interval times out, shown as transition **1508,** the atrial leadless cardiac pacemaker delivers an atrial pacing pulse. Because no other atrial heartbeat has occurred during the duration of the escape interval, the atrial pacing pulse does not fall in the atria's natural refractory period and therefore should effectively pace the atrium, causing an atrial heartbeat. The atrial pacing pulse, coded in the manner shown in **FIGURE 5****,** also signals to any and all other co-implanted leadless cardiac pacemakers and optionally to the co-implanted ICD that an atrial heartbeat has occurred. If functionality is enhanced for a more complex system, the atrial leadless cardiac pacemaker can use a different code to signify synchronous pacing triggered by an atrial sensed event in comparison to the code used to signify atrial pacing at the end of an escape interval. However, in the simple example shown in **FIGUREs 15** and **16****,** the same code can be used for all atrial pacing pulses. In fact, for the simple dual-chamber pacing system described in **FIGUREs 15** and **16** encoding may be omitted because each leadless cardiac pacemaker can conclude that any detected pacing pulse, which is not generated locally, must have originated with the other co-implanted leadless cardiac pacemaker. After generating the atrial pacing pulse **1510,** the atrial leadless cardiac pacemaker starts timing an atrial (AA) escape interval at action **1512,** and then returns to the wait state **1502.**

The atrial leadless cardiac pacemaker can further operate in response to another pacemaker. The atrial pacemaker can detect **1506** a signal originating from a co-implanted ventricular leadless cardiac pacemaker. The atrial pacemaker can examine the elapsed amount of the atrial-to-atrial (AA) escape time interval since a most recent atrial heartbeat and determine **1514** whether the signal originating from the co-implanted ventricular leadless cardiac pacemaker is premature. Thus, if the atrial leadless cardiac pacemaker detects a signal originating from a co-implanted ventricular leadless cardiac pacemaker, shown as sensed ventricular pacing **1506,** then the atrial device examines the amount of the escape interval elapsed since the last atrial heartbeat at decision point **1514** to determine whether the ventricular event is "premature", meaning too late to be physiologically associated with the last atrial heartbeat and in effect premature with respect to the next atrial heartbeat. In the absence **1516** of a premature signal, the atrial pacemaker waits **1502** for an event with no effect on atrial pacing. In contrast if the signal is premature **1518,** the pacemaker restarts **1520** a ventricle-to-atrium (VA) escape interval that is shorter than the atrial-to-atrial (AA) escape interval and is representative of a typical time from a ventricular beat to a next atrial beat in sinus rhythm, specifically the atrial interval minus the atrio-ventricular conduction time. After starting **1520** the VA interval, the atrial leadless cardiac pacemaker returns to wait state **1502,** whereby a ventricular premature beat can be said to "recycle" the atrial pacemaker. The pacemaker responds to timeout of the atrial-to-atrial (AA) escape interval **1508** by delivering an atrial pacing pulse **1510,** causing an atrial heartbeat. The atrial pacing pulse encodes the paced type and atrial location of an atrial heartbeat event.

The atrial leadless cardiac pacemaker can be further configured to time a prolonged post-ventricular atrial refractory period (PVARP) after recycling in presence of the premature signal, thereby preventing pacemaker-mediated tachycardia (PMT). Otherwise, if a received ventricular pacing signal evaluated at decision point **1514** is not found to be premature, then the atrial leadless cardiac pacemaker follows transition **1516** and re-enters the wait state **1502** without recycling, thus without any effect on the timing of the next atrial pacing pulse.

Referring to **FIGURE 16****,** a state-mechanical representation depicts operation of a leadless cardiac pacemaker for implantation adjacent to right-ventricular cardiac muscle. The leadless cardiac pacemaker can be configured for operation in a particular location and system either at manufacture or by an external programmer. A system comprising multiple leadless cardiac pacemakers can include a right-ventricular leadless cardiac pacemaker implanted in electrical contact to a right-ventricular cardiac chamber. The right-ventricular leadless cardiac pacemaker can be configured to perform actions **1600** for coordinated pacing in combination with the other pacemakers. The right-ventricular leadless cardiac pacemaker waits **1602** for the earliest occurring event of multiple events including a sensed right-ventricular heartbeat **1604,** a sensed communication of a pacing pulse **1606** marking a heartbeat at an atrial leadless cardiac pacemaker, and timeout **1608** of an escape interval. Generally, the sensed communication of a pacing pulse **1606** can be any suitable sensed communication of an event originating at another co-implanted leadless cardiac pacemaker, in the illustrative embodiment a pacing pulse marking a heartbeat at an atrial leadless cardiac pacemaker shown as sensed atrial pacing. The escape interval timeout **1608** can be any suitable timeout of an interval timed locally in the right-ventricular leadless cardiac pacemaker.

The right-ventricular leadless cardiac pacemaker responds to the sensed right-ventricular heartbeat **1604** by generating **1610** a right-ventricular pacing pulse that signals to at least one other pacemaker of the multiple cardiac pacemakers and optionally to the co-implanted ICD that a right-ventricular heartbeat has occurred. Thus, when a sensed right-ventricular heartbeat occurs **1604,** the right-ventricular leadless cardiac pacemaker generates **1610** a right-ventricular pacing pulse, not to pace the heart but rather to signal to another leadless cardiac pacemaker or pacemakers that a right-ventricular heartbeat has occurred. The right-ventricular pacing pulse can be encoded with a code signifying the right-ventricular location and a sensed event type. The right-ventricular pacing pulse is coded in the manner shown in **FIGURE 5** with a unique code signifying the location in the right ventricle. Upon right-ventricular pacing pulse generation **1610,** the right-ventricular leadless cardiac pacemaker can time **1612** a predetermined right ventricular-to-right ventricular (VV) escape interval. The right-ventricular leadless cardiac pacemaker restarts **1612** timing of a predetermined escape interval, called the VV (right) (right-ventricular to right-ventricular) escape interval, which is the time until the next right-ventricular pacing pulse if no other event intervenes.

The right-ventricular leadless cardiac pacemaker can further be configured to set the ventricular-to-ventricular (VV) escape interval longer than a predetermined atrial-to-atrial (AA) escape interval to enable backup ventricular pacing at a low rate corresponding to the VV escape interval in case of failure of a triggered signal from a co-implanted atrial leadless cardiac pacemaker. Typically, the VV (right) escape interval is longer than the AA interval depicted in **FIGURE 15****,** so that the system supports backup ventricular pacing at a relatively low rate in case of failure of the co-implanted atrial leadless cardiac pacemaker. In normal operation of the system, timeout of the VV interval never occurs. The right-ventricular leadless cardiac pacemaker then re-enters the Wait state **1602.**

The right-ventricular leadless cardiac pacemaker can respond to timeout of a first occurring escape interval **1608** by delivering **1610** a right ventricular pacing pulse, causing a right ventricular heartbeat. The right ventricular pacing pulse can encode information including paced type and right-ventricular location of a right ventricular heartbeat event.

When the right-ventricular escape interval times out **1608,** the right-ventricular leadless cardiac pacemaker delivers **1610** a right-ventricular pacing pulse. Because no other right-ventricular heartbeat has occurred during the duration of the VV escape interval, the pacing pulse **1610** does not fall in the ventricles' natural refractory period and therefore should effectively pace the ventricles, causing a ventricular heartbeat. The right-ventricular pacing pulse, coded in the manner shown in **FIGURE 5****,** also signals to any and all other co-implanted leadless cardiac pacemakers and optionally to the co-implanted ICD that a right-ventricular heartbeat has occurred. If useful for the function of a more complex system, the right-ventricular leadless cardiac pacemaker can use a different code to signify synchronous pacing triggered by a right-ventricular sensed event in comparison to the code used to signify right-ventricular pacing at the end of a VV escape interval. However, in the simple example shown in **FIGUREs 15** and **16****,** the same code can be used for all right-ventricular pacing pulses. In fact, for the simple dual-chamber pacing system described in **FIGUREs 15** and **16****,** a code may be omitted because each leadless cardiac pacemaker can conclude that any detected pacing pulse which is not generated local to the pacemaker originates with the other co-implanted leadless cardiac pacemaker. After generating **1610** the right-ventricular pacing pulse, the right-ventricular leadless cardiac pacemaker starts timing **1612** a right-ventricular escape interval VV, and then returns to the wait state **1602.**

The right-ventricular leadless cardiac pacemaker can further be configured to detect **1606** a signal originating from a co-implanted atrial leadless cardiac pacemaker. The right-ventricular leadless cardiac pacemaker examines the elapsed amount ofthe ventricular-to-ventricular (VV) escape interval since a most recent right-ventricular heartbeat and determines **1614** whether the signal originating from the co-implanted atrial leadless cardiac pacemaker is premature. An atrial event is defined as premature if too early to trigger an atrio-ventricular delay to produce a right-ventricular heartbeat. In the presence of a premature signal **1616,** the right-ventricular leadless cardiac pacemaker returns to the wait state **1602** with no further action. Thus, a premature atrial beat does not affect ventricular pacing. In the absence of a premature signal **1618,** the right-ventricular leadless cardiac pacemaker starts **1620** a right atrium to right ventricular (AV) escape interval that is representative of a typical time from an atrial beat to a right-ventricular beat in sinus rhythm. Thus a non-premature atrial event leads to starting **1620** an AV (right) atrium to right-ventricular escape interval that represents a typical time from an atrial beat to a right-ventricular beat in normally-conducted sinus rhythm. After starting **1620** the AV interval, the right-ventricular leadless cardiac pacemaker returns to the wait state **1602** so that a non-premature atrial beat can "trigger" the right-ventricular pacemaker after a physiological delay. The right-ventricular leadless cardiac pacemaker also responds to timeout of either the VV escape interval and the AV escape interval **1608** by delivering **1610** a right ventricular pacing pulse, causing a right ventricular heartbeat. The right ventricular pacing pulse encodes paced type and right-ventricular location of a right ventricular heartbeat event.

Accordingly, co-implanted atrial and right-ventricular leadless cardiac pacemakers depicted in **FIGUREs 15** and **16** cooperate to form a dual-chamber pacing system.

Referring to **FIGURE 17****,** a state-mechanical representation illustrates the operation of a leadless cardiac pacemaker for implantation adjacent to left-ventricular cardiac muscle. The left-ventricular cardiac pacemaker can be used in combination with the dual-chamber pacemaker that includes the atrial leadless cardiac pacemaker and the right-ventricular leadless cardiac pacemaker described in **FIGUREs 15** and **16** respectively to form a system for CRT-D. A leadless cardiac pacemaker, for example the left-ventricular cardiac pacemaker, can be configured for operation in a particular location and system either at manufacture or by an external programmer.

A cardiac pacing system, such as a CRT-D system, can include multiple leadless cardiac pacemakers including a left-ventricular leadless cardiac pacemaker implanted in electrical contact to a left-ventricular cardiac chamber. The left-ventricular leadless cardiac pacemaker can execute operations of an illustrative pacing method **1700.** In a wait state **1702,** the left-ventricular cardiac pacemaker waits **1702** at the left-ventricular leadless cardiac pacemaker for an earliest occurring event of multiple events including a sensed communication **1704** of a pacing pulse marking a heartbeat at an atrial leadless cardiac pacemaker and timeout **1706** of a left ventricular escape interval. Generally, the sensed communication **1704** can be the sensed communication of an event originating at another co-implanted leadless cardiac pacemaker, in the illustrative embodiment a pacing pulse marking a heartbeat at an atrial leadless cardiac pacemaker shown as sensed atrial pacing. The escape interval timeout **1706** can be timeout of an interval timed locally in the left-ventricular leadless cardiac pacemaker. In the wait state **1702** for the left-ventricular leadless cardiac pacemaker, operation is simplified and the left-ventricular pacemaker does not respond to left-ventricular heartbeats. Also, the left-ventricular cardiac pacemaker does not pace the left ventricle in the absence of a triggering signal from the atrial leadless cardiac pacemaker. The left-ventricular cardiac pacemaker responds to timeout **1706** of the left ventricular escape interval by delivering **1708** a left ventricular pacing pulse, causing a left ventricular heartbeat. The left ventricular pacing pulse encodes the type and location of a left ventricular heartbeat event. The left-ventricular pacing pulse can be coded in the manner shown in **FIGURE 5** to communicate signals to any and all other co-implanted leadless cardiac pacemakers and optionally to the co-implanted ICD that a left-ventricular heartbeat has occurred, although such encoding is not necessary in the simplified CRT-D system shown in the described embodiment because the other leadless cardiac pacemakers do not react to left-ventricular pacing. After generating **1708** the left-ventricular pacing pulse, the left-ventricular leadless cardiac pacemaker returns to the wait state **1702.**

The left-ventricular leadless cardiac pacemaker can be further configured detect a signal originating from a co-implanted atrial leadless cardiac pacemaker and examine the elapsed amount of the left ventricular escape interval since a most recent left-ventricular heartbeat. The left-ventricular cardiac pacemaker can determine **1710** whether the signal originating from the co-implanted atrial leadless cardiac pacemaker is premature. If the left-ventricular leadless cardiac pacemaker detects sensed atrial pacing, then the left-ventricular device determines whether the atrial event is premature, meaning too early to trigger an atrio-ventricular delay to produce a left-ventricular heartbeat. In the presence of a premature signal **1712,** the left-ventricular cardiac pacemaker reverts to the wait state **1702** and waits for an event with no effect on ventricular pacing so that a premature atrial beat does not affect ventricular pacing. In absence of a premature signal **1714,** the left-ventricular cardiac pacemaker starts **1716** a left atrium to left ventricular (AV) escape interval that is representative of a typical time from an atrial beat to a left ventricular beat in normally-conducted sinus rhythm. As shown in the depicted embodiment, the AV (left) escape interval can have a different value from the AV (right) escape interval. After starting **1716** the AV interval, the left-ventricular leadless cardiac pacemaker returns to wait state **1702.** Accordingly, a non-premature atrial beat can "trigger" the left-ventricular pacemaker after a physiological delay.

The left-ventricular cardiac pacemaker also responds to timeout **1706** of the AV escape interval by delivering **1708** a left ventricular pacing pulse, causing a left ventricular heartbeat. The left ventricular pacing pulse encodes paced type and left ventricular location of a left ventricular heartbeat event.

In various embodiments, the multiple leadless cardiac pacemakers can comprise a right ventricular leadless cardiac pacemaker and a left ventricular leadless cardiac pacemaker that are configured to operate with atrio-ventricular (AV) delays whereby a left ventricular pacing pulse can be delivered before, after, or substantially simultaneously with a right ventricular pacing pulse. For example, multiple co-implanted leadless cardiac pacemakers that function according to the state diagrams shown in **FIGUREs 15****,** **16****,** and **17** can support CRT-D with left-ventricular pacing delivered before, at the same time as, or after right-ventricular pacing.

The co-implanted ICD can configure the leadless cardiac pacemakers via conducted communication in a similar manner to an external programmer. In particular, the ICD can configure them to deliver overdrive anti-tachycardia pacing in response to detected tachyarrhythmias.

In various embodiments, multiple co-implanted leadless cardiac pacemakers can be configured for multi-site pacing that synchronizes depolarization for tachyarrhythmia prevention.

The illustrative system can be useful in conjunction with an ICD, and more particularly with a subcutaneous ICD, for such an ICD has no other means to provide bradycardia support, anti-tachycardia pacing, and CRT.

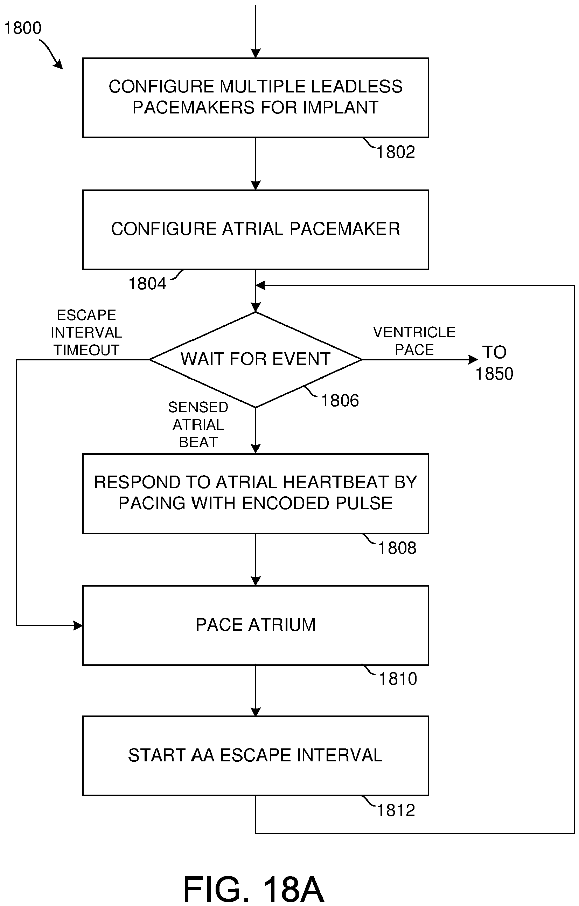

Referring to **FIGUREs 18A****,** **18B****,** **19A****,** **19B****,** **20A****,** and **20B****,** schematic flow charts illustrate an embodiment of a method for operating a cardiac pacing system that comprises an implantable cardioverter-defibrillator (ICD) and one or more leadless cardiac pacemakers configured for implantation in electrical contact with a cardiac chamber and configured for performing cardiac pacing functions in combination with the ICD. Pacing functions include delivering cardiac pacing pulses, sensing evoked and/or natural cardiac electrical signals, and bidirectionally communicating with a co-implanted ICD and/or at least one other pacemaker. The one or more leadless cardiac pacemakers are further configured to communicate a code that signifies occurrence of sensed cardiac electrical signals and/or delivered pacing pulses and identifies an event type and/or location.

Two or more electrodes are coupled to the ICD and configured to transmit and/or receive conducted communication using a pulse-modulated or frequency-modulated carrier signal. The ICD can be configured to detect communication pulses from at least one co-implanted leadless cardiac pacemaker and transmit programming information to the at least one co-implanted leadless cardiac pacemaker.

The leadless cardiac pacemakers can be configured to broadcast information to the co-implanted ICD and/or at least one other pacemaker. The leadless cardiac pacemakers can further be configured to receive the code and react based on the code, location of the receiving leadless cardiac pacemaker, and predetermined system functionality.

In various embodiments, configurations, and conditions, the leadless cardiac pacemakers can be adapted to perform one or more cardiac pacing functions such as single-chamber pacing, dual-chamber pacing, cardiac resynchronization therapy with cardioversion/defibrillation (CRT-D), single-chamber overdrive pacing for prevention of tachyarrhythmias, single-chamber overdrive pacing for conversion oftachyarrhythmias, multiple-chamber pacing for prevention oftachyarrhythmias, multiple-chamber pacing for conversion of tachyarrhythmias, and the like.

Multiple leadless cardiac pacemakers can be configured for co-implantation in a single patient and multiple-chamber pacing including CRT-D. Bidirectional communication among the multiple leadless cardiac pacemakers can be adapted to communicate notification of a sensed heartbeat or delivered pacing pulse event and encoding type and location of the event to at least one pacemaker of the leadless cardiac pacemaker plurality. The one or more pacemakers that receive the communication can decode the information and react depending on location of the receiving pacemaker and predetermined system functionality.

**FIGURE 18A** depicts a method **1800** for operating one or more leadless cardiac pacemakers including an atrial leadless cardiac pacemaker that is implanted in electrical contact to an atrial cardiac chamber and configured for dual-chamber pacing in combination with the co-implanted ICD. Cardiac pacing comprises configuring **1802** a multiple leadless cardiac pacemakers for implantation and configuring **1804** an atrial leadless cardiac pacemaker of the multiple leadless cardiac pacemakers for implantation in electrical contact to an atrial cardiac chamber. The atrial leadless cardiac pacemaker waits **1806** for an earliest occurring event of multiple events including a sensed atrial heartbeat, a communication of an event sensed on the at least two leadless electrodes encoding a pacing pulse marking a heartbeat at a ventricular leadless cardiac pacemaker, and timeout of an atrial-to-atrial (AA) escape interval. The atrial leadless cardiac pacemaker responds **1808** to the sensed atrial heartbeat by generating an atrial pacing pulse that signals to at least one pacemaker of the multiple leadless cardiac pacemakers and optionally to the co-implanted ICD that an atrial heartbeat has occurred and that encodes the atrial pacing pulse with a code signifying an atrial location and a sensed event type. After either a sensed atrial heartbeat or timeout of an escape interval, the atrial leadless cardiac pacemaker delivers **1810** an atrial pacing pulse, causing an atrial heartbeat and starts **1812** timing a predetermined length AA escape interval, then waiting **1806** for an event. The atrial pacing pulse identifies paced type and/or atrial location of an atrial heartbeat event.

In some embodiments, the atrial leadless cardiac pacemaker can encode an atrial pacing pulse that identifies synchronous pacing triggered by an atrial sensed event with a first code and encode an atrial pacing pulse that identifies atrial pacing following the AA escape interval with a second code distinct from the first code.

The atrial leadless cardiac pacemaker can, upon delivery of an atrial pacing pulse, time an atrial-to-atrial (AA) escape interval.

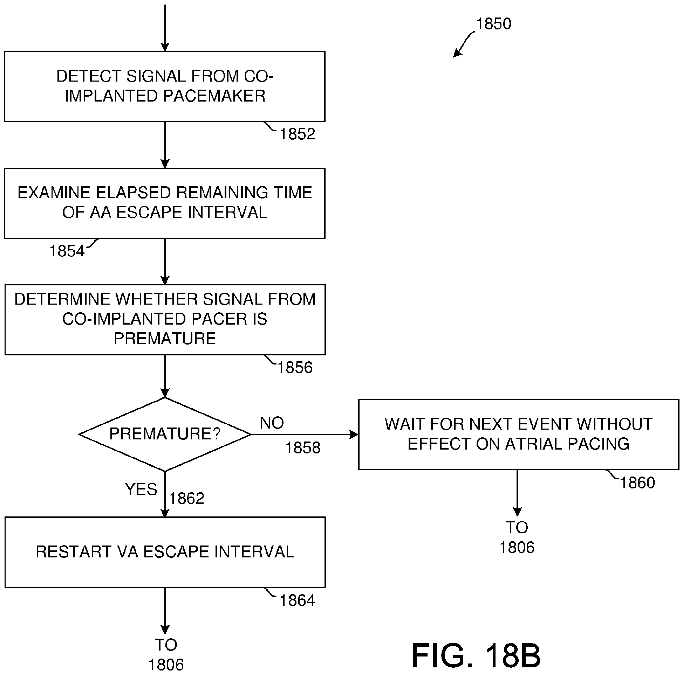

**FIGURE 18B** is a flow chart showing another aspect **1850** of the method embodiment for operating an atrial leadless cardiac pacemaker. The atrial leadless cardiac pacemaker detects **1852** a signal originating from a co-implanted ventricular leadless cardiac pacemaker and examines **1854** an elapsed amount of the atrial-to-atrial (AA) escape interval since a most recent atrial heartbeat, determining **1856** whether the signal originating from the co-implanted ventricular leadless cardiac pacemaker is premature. In absence of a premature signal **1858,** the atrial leadless cardiac pacemaker waits **1860** for an event with no effect on atrial pacing, returning to wait state **1806.** In presence of a premature signal **1862,** the atrial leadless cardiac pacemaker restarts **1864** a ventricle-to-atrium (VA) escape interval that is shorter than the atrial-to-atrial (AA) escape interval and representative of a typical time from a ventricular beat to a next atrial beat in sinus rhythm, then returns to wait state **1806.**

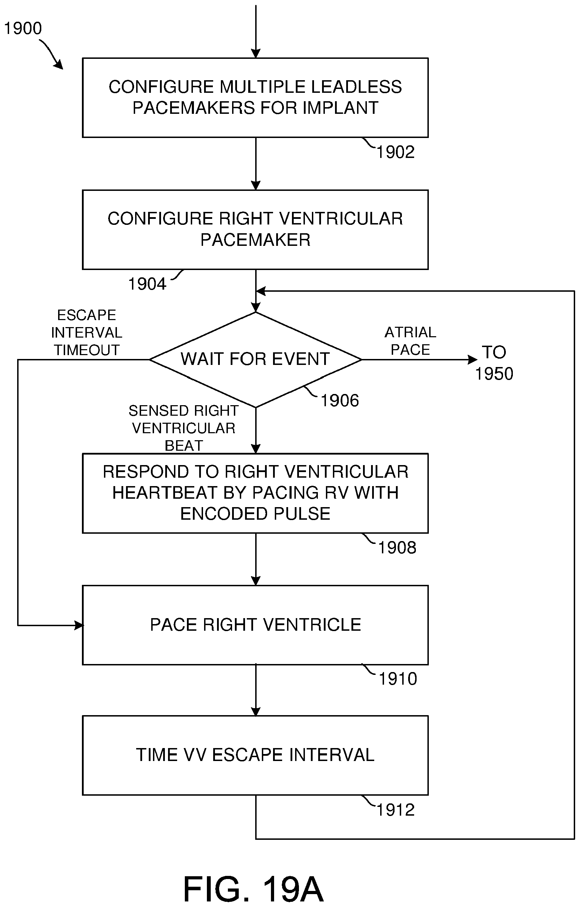

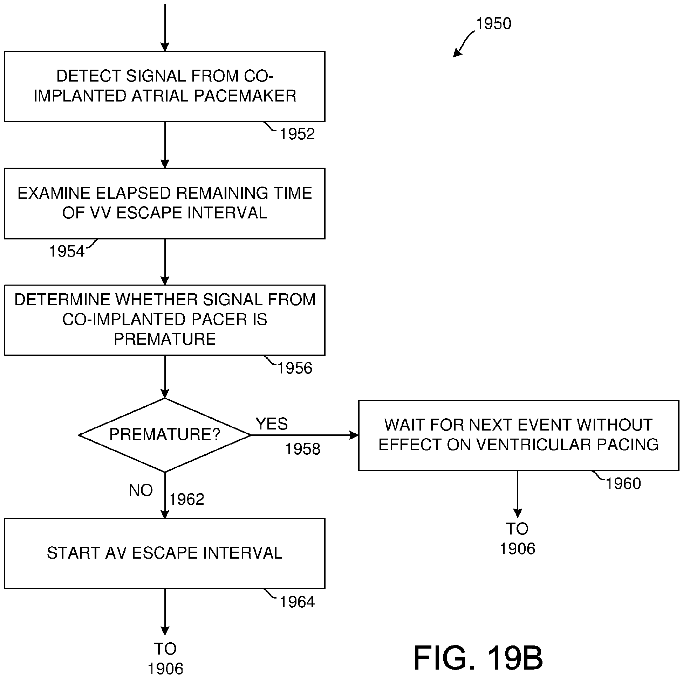

Referring to **FIGUREs 19A** and **19B****,** schematic flow charts illustrate an embodiment of a method for operating a right-ventricular leadless cardiac pacemaker in an illustrative multi-chamber cardiac pacing system. The right-ventricular leadless cardiac pacemaker is implanted in electrical contact to a right-ventricular cardiac chamber and configured for dual-chamber pacing in combination with the co-implanted ICD. **FIGURE 19A** depicts a method **1900** for cardiac pacing comprising configuring **1902** a plurality of leadless cardiac pacemakers for implantation and configuring **1904** a right-ventricular leadless cardiac pacemaker of the multiple leadless cardiac pacemakers for implantation in electrical contact to a right-ventricular cardiac chamber. The right-ventricular leadless cardiac pacemaker waits **1906** for an earliest occurring event of a several events including a sensed right-ventricular heartbeat, a sensed communication of a pacing pulse marking a heartbeat at an atrial leadless cardiac pacemaker, and timeout of an escape interval. The right-ventricular leadless cardiac pacemaker responds **1908** to the sensed right-ventricular heartbeat by generating a right-ventricular pacing pulse that signals to at least one pacemaker of the leadless cardiac pacemakers and optionally to the co-implanted ICD that a right-ventricular heartbeat has occurred and that encodes the right-ventricular pacing pulse with a code signifying a right-ventricular location and a sensed event type. The right-ventricular leadless cardiac pacemaker responds **1910** to timeout of a first-occurring escape interval by delivering a right ventricular pacing pulse, causing a right ventricular heartbeat, with the right ventricular pacing pulse encoding paced type and right ventricular location of a right ventricular heartbeat event, and times **1912** a predetermined ventricular-to-ventricular (VV) escape interval.

In some embodiments, the right-ventricular leadless cardiac pacemaker can encode a right-ventricular pacing pulse that identifies synchronous pacing triggered by a right-ventricular sensed event with a first code and encode a right-ventricular pacing pulse that identifies right-ventricular pacing following a ventricular-to-ventricular (VV) escape interval with a second code distinct from the first code.

In some embodiments, the right-ventricular leadless cardiac pacemaker, upon delivery of a right-ventricular pacing pulse, can time a ventricular-to-ventricular (VV) escape interval.

**FIGURE 19B** is a flow chart showing another aspect of an embodiment of a method 1950 for operating a right-ventricular leadless cardiac pacemaker. The right-ventricular leadless cardiac pacemaker detects **1952** a signal originating from a co-implanted atrial leadless cardiac pacemaker, examines **1954** the elapsed amount of the ventricular-to-ventricular (VV) escape interval since a most recent right-ventricular heartbeat, and determines **1956** whether the signal originating from the co-implanted atrial leadless cardiac pacemaker is premature. In presence **1958** of a premature signal, the right-ventricular leadless cardiac pacemaker waits **1960** for an event with no effect on ventricular pacing, returning to wait state **1906.** In absence **1962** of a premature signal, the right-ventricular leadless cardiac pacemaker starts **1964** a right atrium to right ventricular (AV) escape interval that is representative of a typical time from an atrial beat to a right-ventricular beat in sinus rhythm, and then returns to the wait state **1906.**

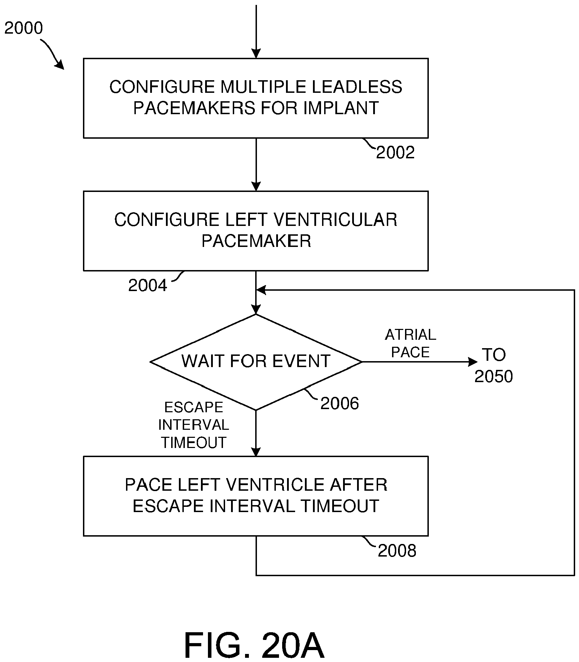

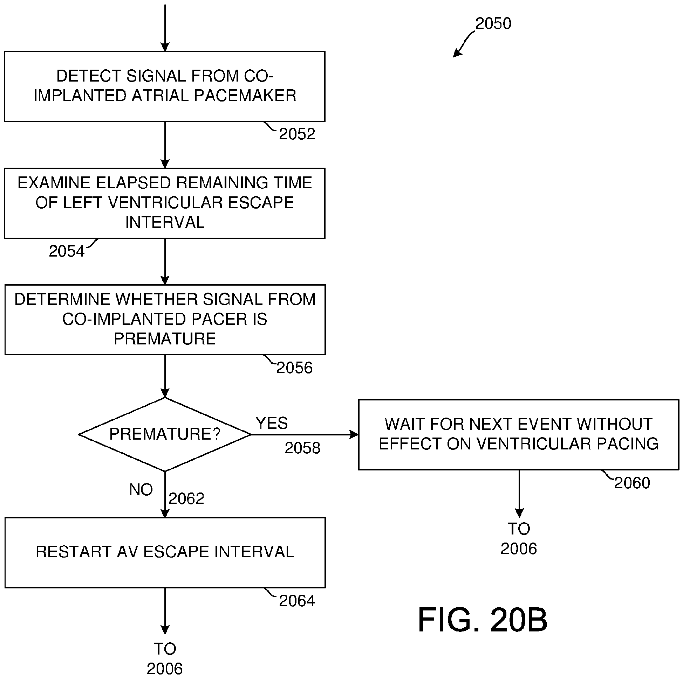

Referring to **FIGUREs 20A** and **20B****,** schematic flow charts illustrate embodiments of a method for operating a left-ventricular leadless cardiac pacemaker in multi-chamber cardiac pacing system. The left-ventricular leadless cardiac pacemaker is implanted in electrical contact to a left-ventricular cardiac chamber and configured for dual-chamber pacing in combination with the co-implanted ICD. **FIGURE 20A** depicts a method **2000** for cardiac pacing comprising configuring **2002** a plurality of leadless cardiac pacemakers for implantation and configuring **2004** a left-ventricular leadless cardiac pacemaker of the leadless cardiac pacemaker plurality for implantation in electrical contact to a left-ventricular cardiac chamber and for operation in cardiac resynchronization therapy (CRT-D). The left-ventricular cardiac pacemaker waits **2006** at the left-ventricular leadless cardiac pacemaker for an earliest occurring event of a plurality of events comprising a sensed communication of a pacing pulse marking a heartbeat at an atrial leadless cardiac pacemaker and timeout of a left ventricular escape interval. The left-ventricular cardiac pacemaker responds **2008** to timeout of the left ventricular escape interval by delivering a left ventricular pacing pulse, causing a left ventricular heartbeat, the left ventricular pacing pulse encoding type and location of a left ventricular heartbeat event.

In some embodiments, the left-ventricular cardiac pacemaker can configure the left-ventricular leadless cardiac pacemaker for operation in cardiac resynchronization therapy (CRT-D).

**FIGURE 20B** is a flow chart showing another embodiment of a method 2050 for operating a left-ventricular leadless cardiac pacemaker. The left-ventricular leadless cardiac pacemaker detects **2052** a signal originating from a co-implanted atrial leadless cardiac pacemaker, examines **2054** the elapsed amount of the left ventricular escape interval since a most recent left-ventricular heartbeat, and determines **2056** whether the signal originating from the co-implanted atrial leadless cardiac pacemaker is premature. In the presence **2058** of a premature signal, the left-ventricular cardiac pacemaker waits **2060** for an event with no effect on ventricular pacing. In the absence **2062** of a premature signal, the left-ventricular cardiac pacemaker starts **2064** a left atrium to left ventricular (AV) escape interval that is representative of a typical time from an atrial beat to a left ventricular beat in sinus rhythm.

In an illustrative system, a rate-responsive leadless cardiac pacemaker can be implanted adjacent to the inside or outside wall of a cardiac chamber.

In addition, a technique for rate-responsive pacing enables pacing control of the leadless cardiac pacemaker which is implanted adjacent to the inside or outside wall of a cardiac chamber.

A cardiac pacemaker for implantation in the human body, more specifically a leadless cardiac pacemaker for implantation adjacent to the inside or outside wall of a cardiac chamber, uses two or more electrodes located within, on, or within two centimeters of the housing of the pacemaker for pacing and sensing at the cardiac chamber and for bidirectional communication with at least one other device within or outside the body. The pacemaker contains an activity sensor, such as an accelerometer, temperature sensor, and/or a pressure transducer to measure patient activity, enabling rate-responsive pacing.

The illustrative system enables cardiac pacing without a pulse generator located in the pectoral region or abdomen, without an electrode-lead separate from the pulse generator, without a communication coil or antenna, and without an additional requirement on battery power for transmitted communication.

An illustrative rate-responsive leadless cardiac pacemaker can be substantially enclosed in a hermetic housing suitable for placement on or attachment to the inside or outside of a cardiac chamber. The pacemaker has at least two electrodes located within, on, or near the housing, for delivering pacing pulses to and sensing electrical activity from the muscle of the cardiac chamber, and for bidirectional communication with at least one other device within or outside the body. The housing contains a primary battery to provide power for pacing, sensing, and communication. The housing also contains circuits for sensing cardiac activity from the electrodes, receiving information from at least one other device via the electrodes, generating pacing pulses for delivery via the electrodes, transmitting information to at least one other device via the electrodes, monitoring device health, and controlling these operations in a predetermined manner.

A leadless pacemaker is configured for implantation adjacent to the inside or outside wall of a cardiac chamber, without the need for a connection between the pulse generator and electrode lead, and without the need for a lead body.

In some embodiments, the illustrative system enables communication between the implanted pulse generator and a device internal or external to the body, using conducted communication via the same electrodes used for pacing, without the need for an antenna or telemetry coil.

Still other embodiments enable communication between the implanted pulse generator and a device internal or external to the body, with power consumption similar to that for cardiac pacing, to allow optimization of battery performance.

Referring to **FIGUREs 1B** and **1E****,** a pictorial view which is not shown to scale and a schematic block diagram respectively depict an embodiment of a cardiac pacing system **100** that comprises a rate-responsive leadless cardiac pacemaker **102.** The rate-responsive leadless cardiac pacemaker **102** comprises a housing **110,** multiple electrodes **108** coupled to the housing **110,** a pulse delivery system **152** hermetically contained within the housing **110** and electrically coupled to the electrodes **108.** The pulse delivery system **152** configured for sourcing energy internal to the housing **110,** generating and delivering electrical pulses to the electrodes **108.** The rate-responsive leadless cardiac pacemaker **102** further comprises an activity sensor **154** which is hermetically contained within the housing **110** and adapted to sense activity. A processor **112** is also hermetically contained within the housing **110** as part of a pulse delivery system **152** and is communicatively coupled to the activity sensor **154,** and the electrodes **108.** The processor **112** can control electrical pulse delivery at least partly based on the sensed activity.

In various embodiments, the electrodes **108** can be coupled on, within, or within two centimeters of the housing **110.** In some arrangements, the electrodes **108** can be formed integrally to an outer surface of the housing **110.**

Referring to **FIGURE 1E****,** the rate-responsive leadless cardiac pacemaker 102 has functional elements substantially enclosed in a hermetic housing **110.** The pacemaker has at least two electrodes **108** located within, on, or near the housing **110,** for delivering pacing pulses to and sensing electrical activity from the muscle of the cardiac chamber, and for bidirectional communication with at least one other device within or outside the body. Hermetic feedthroughs **130, 131** conduct electrode signals through the housing **110.** The housing **110** contains a primary battery 114 to provide power for pacing, sensing, and communication. The housing **110** contains circuits 132 for sensing cardiac activity from the electrodes **108;** circuits **134** for receiving information from at least one other device via the electrodes **108;** and a pulse generator **116** for generating pacing pulses for delivery via the electrodes **108** and also for transmitting information to at least one other device via the electrodes **108.** The pacemaker **102** further contains circuits for monitoring device health, for example a battery current monitor **136** and a battery voltage monitor **138.** The pacemaker **102** further contains processor or controller circuits **112** for controlling these operations in a predetermined manner.

In accordance with another embodiment of a pacing system, a leadless cardiac pacemaker **102** comprises a housing **110,** multiple electrodes **108** coupled to the housing **108,** and a pulse generator **116** hermetically contained within the housing **110** and electrically coupled to the electrodes **108.** The pulse generator **116** is configured to generate and deliver electrical pulses to the electrodes **108** powered from a source **114** contained entirely within the housing **110.** An activity sensor **154** is hermetically contained within the housing **110** and adapted to sense activity. A logic **112,** for example a processor, controller, central processing unit, state machine, programmable logic array, and the like, which is hermetically contained within the housing **110** and communicatively coupled to the pulse generator **116,** the activity sensor **154,** and the electrodes **108.** The logic **112** is configured to control electrical pulse delivery at least partly based on the sensed activity.

In some embodiments, the logic **112** can be a processor that controls electrical pulse delivery and application of the activity sensor according to one or more programmable parameters with the processor programmable by communication signals transmitted via the electrodes **108.**

In accordance with another embodiment of a pacing system, a leadless cardiac pacemaker **102** comprises a housing **110,** multiple electrodes **108** coupled to the housing **110,** and a pulse generator **116** hermetically contained within the housing **110** and electrically coupled to the electrodes **108.** The pulse generator **116** generates and delivers electrical pulses to the electrodes **108,** causing cardiac contractions. The pulse generator **116** also conveys information to one or more devices **106** external to the pacemaker **102.** The pacemaker **102** further comprises at least one amplifier **132, 134** hermetically contained within the housing **110** and electrically coupled to the electrodes **108.** The amplifier or amplifiers **132, 134** are configured to amplify signals received from the electrodes **108** and to detect cardiac contractions, and further can receive information from the external device or devices **106.** The pacemaker **102** further comprises a power supply **114** hermetically contained within the housing **110** and coupled to the pulse generator **116.** The power supply **114** sources energy for the electrical pulses from internal to the housing **110.** The pacemaker **102** has an activity sensor **154** hermetically contained within the housing **110** that senses activity. A processor **112** is hermetically contained within the housing **110** and communicatively coupled to the pulse generator **116,** the amplifiers **132, 134,** the activity sensor **154,** and the electrodes **108.** The processor **112** configured to receive amplifier output signals from the amplifier or amplifiers **132, 134** and control electrical pulse delivery at least partly based on the sensed activity.

In an illustrative embodiment, the amplifiers comprise a cardiac sensing amplifier **132** that consumes no more than 5 microwatts, a communications amplifier **134** that consumes no more than 25 microwatts, and a rate-response sensor amplifier **156** that consumes no more than 10 microwatts.

In an example embodiment, the regulator **146** can be configured to consume electrical power of no more than 2 microwatts and configured to supply electrical power of no more than 74 microwatts in the illustrative system that includes a rate-response amplifier.

The processor **112** can be configured to consume electrical power of no more than 5 microwatts averaged over one cardiac cycle.

Current from the positive terminal **140** of primary battery **114** flows through a shunt **144** to a regulator circuit **146** to create a positive voltage supply **148** suitable for powering the remaining circuitry of the pacemaker **102.** The shunt **144** enables the battery current monitor **136** to provide the processor **112** with an indication of battery current drain and indirectly of device health.

The illustrative power supply can be a primary battery **114** such as a beta-voltaic converter that obtains electrical energy from radioactivity. In some embodiments, the power supply can be selected as a primary battery **114** that has a volume less than approximately 1 cubic centimeter.

In an illustrative embodiment, the primary battery **114** can be selected to source no more than 75-80 microwatts instantaneously since a higher consumption may cause the voltage across the battery terminals to collapse. Accordingly in one illustrative embodiment the circuits depicted in **FIGURE 1E** can be designed to consume no more than a total of 74 microwatts. The design avoids usage of a large filtering capacitor for the power supply or other accumulators such as a supercapacitor or rechargeable secondary cell to supply peak power exceeding the maximum instantaneous power capability of the battery, components that would add volume and cost.

In various embodiments, the activity sensor **154** is adapted for controlling rate-responsive pacing and may use any appropriate technology, for the example the activity sensor **154** may be an accelerometer, a temperature sensor, a pressure sensor, or any other suitable sensor.

In an illustrative embodiment, the activity sensor **154** can operate with a power requirement of no more than 10 microwatts.

**FIGURE 1E** shows a pacemaker embodiment wherein the activity sensor comprises an accelerometer **154** and an accelerometer amplifier **156** configured to detect patient activity for rate-responsive pacing. The accelerometer amplifier output terminal is connected to the processor **112.** Because the leadless cardiac pacemaker **102** is attached to cardiac muscle **104,** the accelerometer **154** measures some acceleration due to heartbeats in addition to the desired activity signal. Processor **112** performs sampling of the accelerometer output signal synchronously with the cardiac cycle as determined by the cardiac sensing amplifier **132** and the pulse generator **116.** Processor **112** then compares acceleration signals taken at the same relative time in multiple cardiac cycles to distinguish the part of the acceleration signal that results from activity and is not due to heart wall motion.

In other embodiments, the accelerometer **154** and accelerometer amplifier **156** shown in **FIGURE 1E** can be replaced with a temperature transducer such as a thermistor and a signal conditioning amplifier connected to processor **112.** In another embodiment, a pressure transducer and signal conditioning amplifier can be connected to processor **112.** Temperature is not sensitive to the cardiac cycle so that in such an activity sensor rate-responsive cardiac pacemaker embodiments, synchronous sampling with the cardiac cycle is superfluous. Although pressure varies in the cardiac cycle, easily measured features of the pressure wave, for example peak amplitude, peak-to-peak amplitude, peak rate of change (delta), and the like, can indicate the level of activity.

In accordance with another embodiment of a pacing system **100,** a pacemaker configured as a rate-responsive leadless cardiac pacemaker **102** comprising a housing **110,** and multiple electrodes **108** coupled to the housing **110.** A pulse generator **116** hermetically contained within the housing **110** and electrically coupled to the electrodes **108** and is configured for generating and delivering electrical pulses to the electrodes **108.** An activity sensor **154** is hermetically contained within the housing **110** and adapted to sense activity. A processor **112** is hermetically contained within the housing and communicatively coupled to the pulse generator **116,** the activity sensor **154,** and the electrodes **108.** The processor **112** controls electrical pulse delivery at least partly based on the sensed activity and communicates with one or more devices **106** external to the pacemaker **102** via signals conducted through the electrodes **108.**

In various embodiments, the processor **112** and pulse delivery system **152** transmits and/or receives information such as programmable parameter settings, event counts, power-supply voltage, power-supply current, rate-response control parameters adapted for converting an activity sensor signal to a rate-responsive pacing parameter.

Referring again to **FIGURE 1E****,** the circuit **132** for receiving communication via electrodes **108** receives the triggering information as described and can also optionally receive other communication information, either from the other implanted pulse generator **106** or from a programmer outside the body. This other communication could be coded with a pulse-position scheme as described in **FIGURE 5** or could otherwise be a pulse-modulated or frequency-modulated carrier signal, preferably from 10 kHz to 100 kHz.

With regard to operating power requirements in the leadless cardiac pacemaker **102,** for purposes of analysis, a pacing pulse of 5 volts and 5 milliamps amplitude with duration of 500 microseconds and a period of 500 milliseconds has a power requirement of 25 microwatts.

In an example embodiment of the leadless pacemaker **102,** the processor **112** typically includes a timer with a slow clock that times a period of approximately 10 milliseconds and an instruction-execution clock that times a period of approximately 1 microsecond. The processor **112** typically operates the instruction-execution clock only briefly in response to events originating with the timer, communication amplifier **134,** or cardiac sensing amplifier **132.** At other times, only the slow clock and timer operate so that the power requirement of the processor **112** is no more than 5 microwatts.

For a pacemaker that operates with the aforementioned slow clock, the instantaneous power consumption specification, even for a commercially-available micropower microprocessor, would exceed the battery's power capabilities and would require an additional filter capacitor across the battery to prevent a drop of battery voltage below the voltage necessary to operate the circuit. The filter capacitor would add avoidable cost, volume, and potentially lower reliability.

For example, a microprocessor consuming only 100 microamps would require a filter capacitor of 5 microfarads to maintain a voltage drop of less than 0.1 volt, even if the processor operates for only 5 milliseconds. To avoid the necessity for such a filter capacitor, an illustrative embodiment of a processor can operate from a lower frequency clock to avoid the high instantaneous power consumption, or the processor can be implemented using dedicated hardware state machines to supply a lower instantaneous peak power specification.

In a pacemaker, the cardiac sensing amplifier typically operates with no more than 5 microwatts.

An accelerometer amplifier, or other general purpose signal conditioning amplifier, operates with approximately 10 microwatts.

A communication amplifier at 100 kHz operates with no more than 25 microwatts. The battery ammeter and battery voltmeter operate with no more than 1 microwatt each.

A pulse generator typically includes an independent rate limiter with a power consumption of no more than 2 microwatts.

The total power consumption of the pacemaker is thus 74 microwatts, less than the disclosed 75-microwatt battery output.

Improvement attained by the illustrative cardiac pacing system **100** and leadless cardiac pacemaker 102 is apparent.

The illustrative cardiac pacing system **100** enables encoding optional outgoing communication in the pacing pulse, so that the outgoing communication power requirement does not exceed the pacing current requirement, approximately 25 microwatts.

The illustrative leadless cardiac pacemaker **102** can have sensing and processing circuitry that consumes no more than 10 microwatts as in conventional pacemakers.

The described leadless cardiac pacemaker **102** can have an incoming communication amplifier for receiving triggering signals and optionally other communication which consumes no more than 25 microwatts.

Furthermore, the leadless cardiac pacemaker **102** can have a primary battery that exhibits an energy density of at least 3 watt-hours per cubic centimeter (W·h/cc).

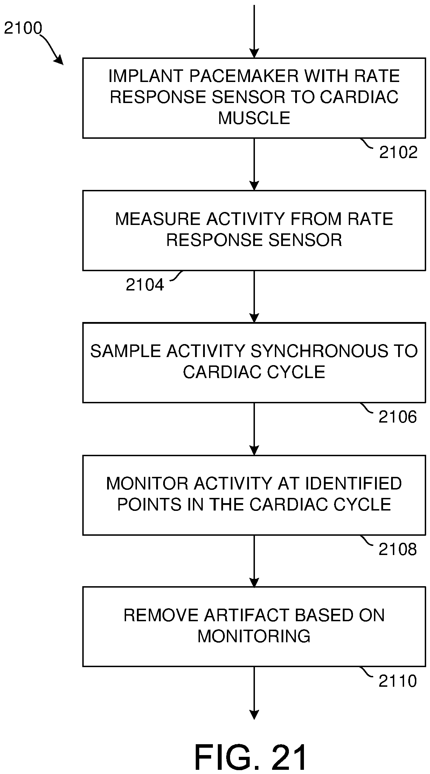

Referring to **FIGURE 21****,** a schematic flow chart depicts an embodiment of a method **2100** for operating an activity sensor in a rate-responsive cardiac pacemaker. A leadless cardiac pacemaker that includes a rate-response sensor is implanted **2102** in contact with cardiac muscle. An activity signal is measured **2104** using the rate-response sensor. The activity signal includes an artifact signal that results from cardiac muscle motion. The activity signal is sampled **2106** synchronously with a cardiac cycle. The activity signal is monitored **2108** at identified points in the cardiac cycle. The artifact signal is removed **2110** from the activity signal based on the monitoring. In various embodiments, the activity signal can be measured using an accelerator, thermistor, or pressure sensor.

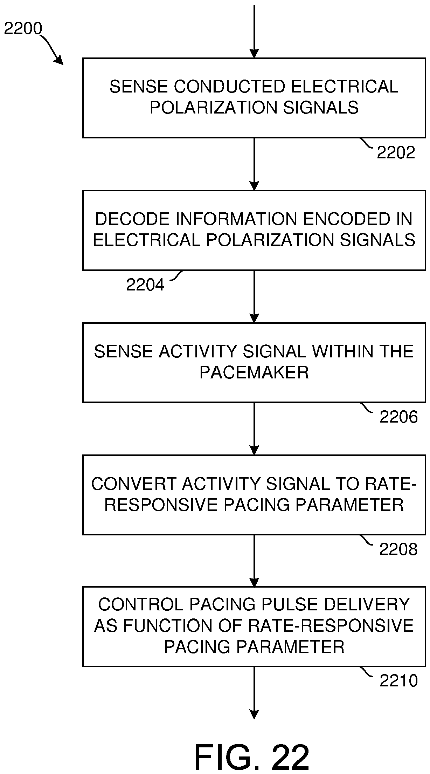

Referring to **FIGURE 22****,** a schematic flow chart depicts an embodiment of a method **2200** for setting operating parameters in a rate-responsive cardiac pacemaker. The method **2200** comprises sensing **2202** electrical signals conducted through a patient's body, decoding **2204** information encoded in the electrical signals, and storing the result. An activity signal is sensed **2206** within the pacemaker. The activity sensor signal is converted **2208** to a rate-responsive pacing parameter as a function of the stored information encoded in the electrical signals. Pacing pulse delivery is controlled **2210** as a function of the rate-responsive pacing parameter.

In some embodiments, information is encoded, for example, as a binary code in one or more notches interrupting a stimulation pulse. Information can otherwise or also be encoded in selected or designated codes as variations in pacing pulse width of a stimulation pulse. Information can also be conveyed as electrical energy in a stimulation pulse in designated codes encoding the information in modulation of off-time between pacing pulses.

A delivery system can deploy leadless cardiac pacemakers equipped with either an active or passive fixation device. A first sheathed catheter system protects the fixation device and provides counter rotation to disengage the leadless cardiac pacemaker from the catheter. A second sheath-less catheter system includes a dissolvable, protective capsule covering the fixation device and a lumen providing counter-rotational force to disengage the leadless cardiac pacemaker from the catheter.

A delivery system is depicted which can be used with a biostimulation device, such as a leadless cardiac pacemaker.

A delivery system can be constructed that reduces the number of concentric elements forming a catheter.

For example, an embodiment of a delivery system can be used for implanting a leadless cardiac pacemaker inside the cardiac chamber in the human body. Embodiments include two delivery systems, for example a sheath and sheath-less approach, to safely deliver leadless cardiac pacemakers with active or passive fixation devices to the cardiovascular system.

In some embodiments, a delivery system can support rotational counter traction to enable disengagement of a stylet using a sheathed approach for protecting the implantable biostimulation device.

A delivery system that implements a sheathed technique for implantable device protection can be configured to enable retraction of the sheath for a pre-defined distance to expose pacemaker electrodes and permit threshold testing without completely disengaging a delivery catheter.

In other illustrative embodiments, a delivery system can employ rotational counter traction to enable disengagement of a stylet using a sheath-less approach for protecting the device. For example, a biocompatible, soluble, protective cover for the fixation devices can be used to prevent damage to the cardiovascular system.

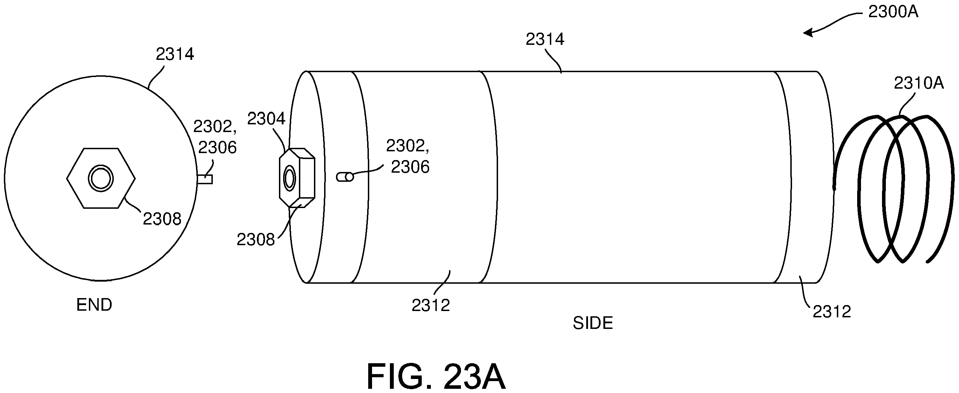

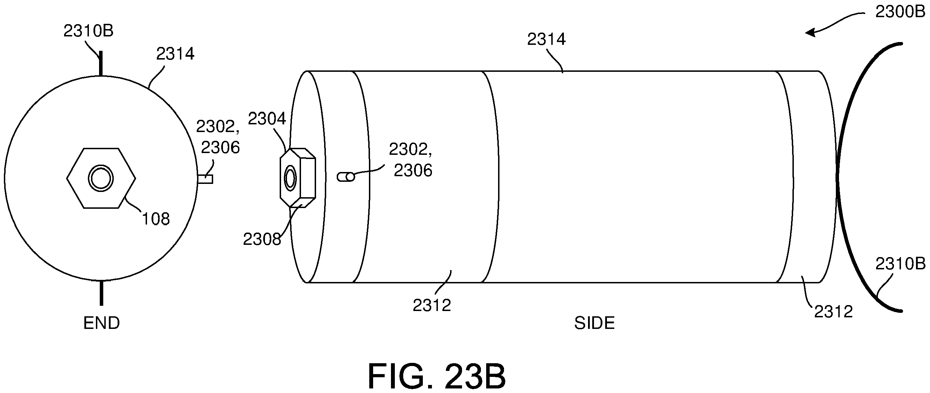

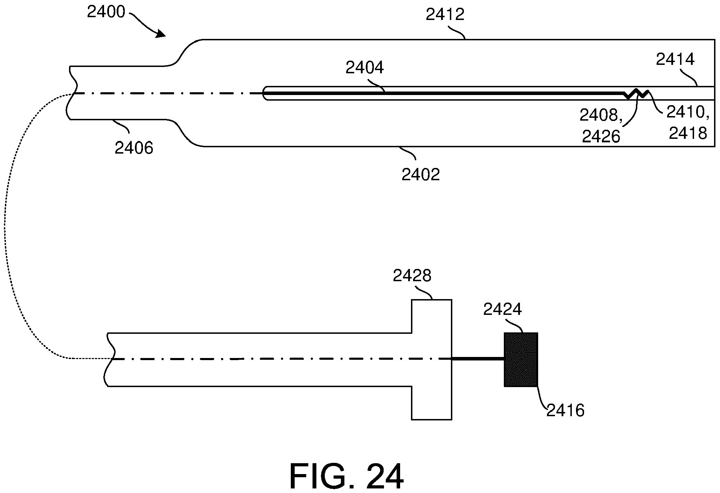

Referring to **FIGUREs 23A** and **23B****,** pictorial end and side views show embodiment of a leadless cardiac pacemaker **2300A, 2300B** that can be delivered using a delivery apparatus. Referring to **FIGURE 24****,** a pictorial view illustrates an embodiment of a delivery apparatus **2400** for delivering and implanting an implantable device, for example a leadless cardiac pacemaker **2300A, 2300B.** The illustrative delivery apparatus **2400** comprises a bi-concentric-axial element catheter **2402** configured for engaging to and disengaging from the leadless cardiac pacemaker via relative motion of an internal stylet element **2404** to an externally circumferential tube element **2406.**

As shown in **FIGURE 24** in combination with **FIGUREs 23A** and **23B****,** the tube element **2406** has a feature **2408** that engages a corresponding feature **2302** on the leadless cardiac pacemaker **2300A, 2300B** whereby the stylet element **2404** can be rotated relative to the tube element **2406** for disengagement of a threaded end **2410** of the stylet **2404** from a threaded end **2304** of the leadless cardiac pacemaker **2300A, 2300B.**

**FIGURE 23A** depicts a leadless cardiac pacemaker **2300A** with an active fixation device **2310A.** The leadless cardiac pacemaker **2300A** is contained within a cylindrical hermetic housing **2314** and includes annular electrodes **2312** at housing extremities. In one example of an active fixation device, a helix **2310A** provides active fixation when screwed into cardiac muscle. A stylet hex nut **2308** enables the delivery catheter **2402** to attach to the leadless cardiac pacemaker **2300A** during deployment. One or more alignment pins **2306** are attached to the hermetic housing **2314.**

**FIGURE 23B** illustrates a leadless cardiac pacemaker **2300B** with a passive fixation device **2310B,** depicted with tines **2310B** shown expanded and facilitate lodging of the leadless cardiac pacemaker **2300B** inside a cardiac vessel during the first few weeks after implantation. The tines **2310B** are commonly made from a biocompatible polymer such as polyurethane or medical grade silicone and may have many various shapes and forms.

**FIGURE 24** illustrates a sheathed embodiment of a delivery apparatus **2400.** The tube element **2406** can comprise a sliding sheath **2412** configured to axially slide over the stylet **2404** and engaged leadless cardiac pacemaker **2300A, 2300B** and configured to protect patient tissue from damage during insertion of the leadless cardiac pacemaker. The sliding sheath **2412** has a feature **2414** that engages a corresponding feature **2306** on the leadless cardiac pacemaker **2300A, 2300B** whereby the stylet **2404** can be rotated relative to the sliding sheath 2412 for disengagement of the threaded end **2410** of the stylet **2404** from the threaded end 2304 of the leadless cardiac pacemaker **2300A, 2300B.**

The sheath/stylet delivery catheter **2402** shown in **FIGURE 24** is an assembly comprising a sheath **2412** and a stylet **2404.** At the distal end 2418 of stylet **2404** is screw **2426.** At the proximal end **2416** of the stylet wire **2404** is a knob **2424** which enables the screw **2426** to be inserted into a stylet hex nut **2308** shown in **FIGUREs 23A****,** **23B** and **24****.** The stylet **2404** can be formed from a biocompatible metal such as stainless steel. The stylet knob **2424** can be formed from a rigid plastic. The sheath **2412** can be formed from extruded Teflon, polytetrafluoroethylene, polyolefin, polyvinyl chloride, or polyurethane and contains one or more slots **2414.** A sheath knob **2428** can be formed from rigid plastic. Elements and components of the sheath/stylet catheter **2402** can be constructed from any suitable material in addition to the materials specifically described herein.

Accordingly, **FIGUREs 23A****,** **23B****,** and **24** describe a leadless cardiac pacemaker (LCP) delivery system used to deploy a LCP with active **2300A** or passive **2300B** fixation using a sheath catheter **2406.** The LCP **2300A, 2300B** connects to a catheter **2406** containing a stylet **2404** and sheath assembly **2412.** The stylet **2404** screws into a hex nut **2308** attached to the end of the LCP **2300A, 2300B.** The sheath **2412** includes a feature **2414** that, when aligned with a guide pin **2306** on the LCP **2300A, 2300B,** enables application of a counter-rotational force for disengaging the locking stylet **2404.** The sheath **2412** protects the cardiovascular system from damage during insertion from the fixation devices **2310A, 2310B** and includes a feature **2414** enabling the catheter **2406** to rotate the LCP **2300A, 2300B.** Once positioned the catheter assembly **2406** can be rotated to affix the active fixation screw **2310A** in the case of active fixation. The sheath **2412** is then partially withdrawn to allow pacing and sensing threshold measurements. After confirming the LCP position, the stylet **2404** can be unscrewed and withdrawn, followed by the sheath **2412,** leaving the LCP **2300A, 2300B** in the selected position.

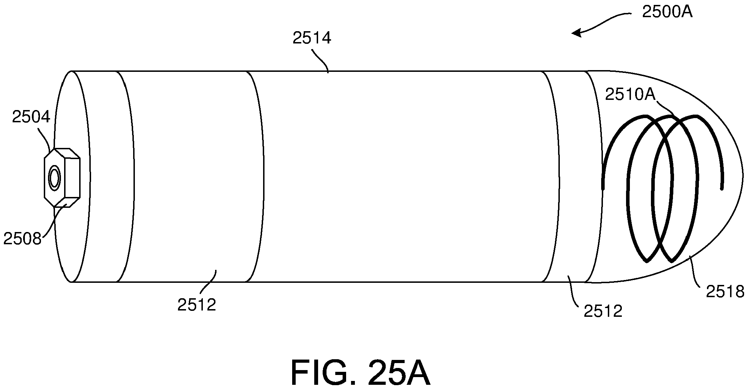

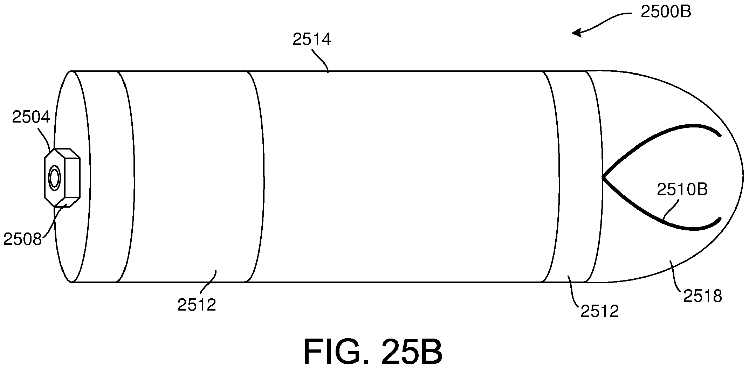

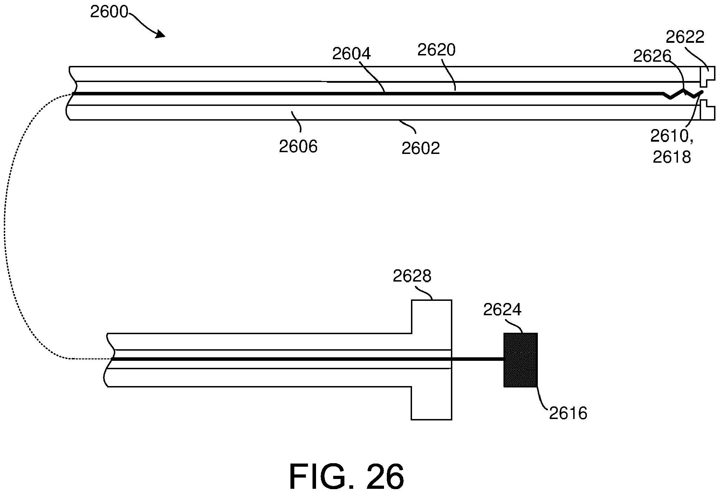

Referring to **FIGUREs 25A** and **25B****,** pictorial side views show embodiment of a leadless cardiac pacemaker **2500A, 2500B** that can be delivered using a sheath-less delivery apparatus. Referring to **FIGURE 26****,** a pictorial view illustrates an embodiment of a sheath-less delivery apparatus **2600** adapted to deliver and implant an implantable device such as a leadless cardiac pacemaker **2500A, 2500B.** The illustrative sheath-less delivery apparatus **2600** has a bi-concentric-axial element catheter **2602** that engages and disengages from the leadless cardiac pacemaker by motion of an internal stylet element **2604** relative to an externally circumferential tube element **2606.** The tube element **2606** comprises a sheath-less catheter **2602** that extends axially from a knob end **2616** to a socket end **2618** and encloses an internal lumen **2620.** The sheath-less catheter **2602** is configured to axially slide over the stylet **2604** and engage the leadless cardiac pacemaker **2500A, 2500B.** The sheath-less catheter **2602** comprises a socket **2622** at the socket end **2618** that engages a corresponding nut **2508** on the leadless cardiac pacemaker **2500A, 2500B** so that the stylet **2604** can be rotated relative to the sheath-less catheter **2602** for disengagement of the threaded end **2610** of the stylet **2604** from the threaded end **2504** of the leadless cardiac pacemaker **2500A, 2500B.** A biocompatible soluble protective covering **2518** can be adapted to cover a fixation member **2510A, 2510B** coupled to the leadless cardiac pacemaker **2500A, 2500B** during insertion of the leadless cardiac pacemaker **2500A, 2500B** into a patient's body whereby the patient's body tissue is protected.

Another embodiment of a delivery system **2600** is sheath-less and includes a tube element **2606** with internal lumen **2620** and stylet catheter **2602** to deliver the LCP **2500A, 2500B** to a selected site. The fixation mechanism **2510A, 2510B** on the LCP **2500A, 2500B** is protected using a biocompatible, soluble coating **2518** during deployment. **FIGUREs 25A** and **25B** depict the leadless cardiac pacemaker with an active fixation **2500A** and passive fixation **2500B** device respectively protected using mannitol or other sugar derivates. Other materials may also or otherwise used that can form a protective capsule at room temperature, dissolve when implanted yet have no toxic side-effects.

The lumen/stylet delivery catheter **2600** is shown in **FIGURE 26****.** The assembly **2600** comprises of a tube element **2606** enclosing a lumen **2620,** a stylet **2604,** a stylet knob **2628,** and lumen assembly knob **2624.** The distal end **2618** of the stylet **2604** contains a threaded screw **2626** for insertion into the stylet hex nut **2508.** Also shown is hex nut socket **2622** which is bonded to the distal end **2618** of the lumen assembly **2606** and adapted to receive the external features of the stylet hex nut **2508** attached to the LCP **2500A, 2500B.** The hex nut socket **2622** prevents counter rotation of the LCP **2500A, 2500B** and lumen assembly **2606** when engaged. The tube element **2606** enclosing the lumen **2620** can be extruded from polyurethane or silicone. The lumen assembly knob **2628** is typically made from polyurethane or rigid plastic. The stylet **2604** can be made from stainless steel. The stylet knob **2424** is typically made from rigid plastic. The diameter of the lumen **2620** is typically the same or inferior to the diameter of the LCP **2500A, 2500B.** Other suitable materials may be substituted for the material specifically disclosed herein.

**FIGURE 26** depicts an LCP delivery system **2600** that can be used to deploy a LCP with active **2500A** or passive **2500B** fixation using a sheath-less catheter **2602** wherein the LCP **2500A, 2500B** connects to a catheter **2602** containing a stylet **2604** and lumen assembly **2620.** The stylet **2604** screws into a hardware element such as a hexagonal or other polygonal sided nut **2508** attached to the end of the LCP **2500A, 2500B.** Counter-rotation can be enabled via a plastic locking polygonal-sided socket, for example a hex socket **2622,** attaches to the lumen assembly **2620** and engages the LCP polygonal-sided nut, for example hex nut **2508.** To protect the cardiovascular system from damage during insertion the fixation devices **2510A, 2510B** are coated with a biocompatible soluble protective covering **2518** such as mannitol. Once positioned, the protective covering **2518** dissolves and the catheter assembly **2602** can be rotated to affix the active fixation screen **2510A** in the case of active fixation. Pacing and sensing threshold measurement can then be performed. After the LCP **2500A, 2500B** position is confirmed, the stylet **2604** can be unscrewed and withdrawn, followed by the lumen assembly **2620,** leaving the LCP **2500A, 2500B** in a selected position.

**FIGUREs 24** and **26** depict two leadless cardiac pacemaker delivery systems **2400, 2600.** The first system **2400** uses a sheath **2412** and stylet catheter **2402.** The second system **2600** uses a lumen assembly **2620** and stylet catheter **2602** in combination with a biocompatible, soluble, protective coating **2518** applied to the leadless cardiac pacemaker **2500A, 2500B.** Either approach can be used with an active fixation or passive fixation element to anchor the leadless cardiac pacemaker to the desired site.

In addition, both delivery systems **2400, 2600** can be combined with other tools and techniques commonly used to obtain access to the cardiovascular venous system, such as introducers, guide-wires, dilators and other tools to gain access to locations commonly used to provide cardiac pacing therapy.

Referring again to **FIGURE 24** in combination with **FIGUREs 23A** and/or **23B,** an embodiment of a delivery system **2400** is depicted which is configured for implanting a biostimulation device **2300A, 2300B.** The delivery system **2400** comprises a stylet **2404** and a catheter tube **2406.** The stylet **2404** extends along an axis from knob end **2414** to a threaded end **2416** and is configured to engage an internally threaded nut **2308** of the biostimulation device **2300A, 2300B.**

The catheter tube **2406** is configured to axially contain the stylet **2404** and comprises a feature **2408** that engages a corresponding feature **2302** on the biostimulation device **2300A, 2300B** whereby the stylet **2404** can be rotated relative to the catheter tube **2406** for disengagement of the threaded end **2408** of the stylet **2404** from the threaded end **2304** of the biostimulation device **2300A, 2300B.**

As shown in the embodiment depicted in **FIGURE 24****,** a sliding sheath assembly **2412** is configured to axially slide over the stylet **2404** and the engaged biostimulation device **2300A, 2300B.** The sliding sheath assembly **2412** comprising a feature **2412** that engages a corresponding feature **2306** on the biostimulation device **2300A, 2300B** whereby the stylet **2404** can be rotated relative to the sliding sheath **2412** for disengagement of the threaded end **2408** of the stylet **2404** from the threaded end **2304** of the biostimulation device **2300A, 2300B.** The sliding sheath **2412** is configured to protect patient tissue from damage during insertion of the biostimulation device **2300A, 2300B.** The sliding sheath **2412** further comprises a feature **2412** configured to engage and rotate the biostimulation device **2306** and to affix a fixation member **2310A, 2310B** coupled to the biostimulation device **2300A, 2300B** into patient tissue. The engaging feature **2412** of the sliding sheath assembly **2412** is configured to enable rotational counter traction for disengagement of the stylet **2404.**

In some embodiments, the sliding sheath assembly **2412** can be configured to axially retract from the stylet **2404** a predetermined distance so that electrodes **2312** of the biostimulation device **2300A, 2300B** are exposed, enabling threshold testing while the biostimulation device **2300A, 2300B** remains engaged to the stylet **2404.**

In the illustrative embodiment, the catheter **2402** comprises a tube element **2406** and a sheath **2412.** The sheath **2412** is configured with a sheath slot **2414** configured to align with an alignment pin **2306** of the biostimulation device **2300A, 2300B** that prevents rotation of the biostimulation device **2300A, 2300B** with respect to the sheath **2412.** The stylet **2404** extends along the axis from a knob **2424** coupled to a proximal end **2416** to a screw **2426** coupled to the distal end **2418.** The stylet **2404** is configured so that for the knob **2424** to be fully retracted, the biostimulation device **2300A, 2300B** is fully contained within the sheath **2412** and a fixation member **2310A, 2310B** coupled to the biostimulation device **2300A, 2300B** is protected. The stylet **2404** is further configured so that for a condition that the knob **2424** is fully depressed, the alignment pin **2306** is contained within the sheath **2412** and electrodes **2312** of the biostimulation device **2300A, 2300B** are fully exposed, enabling threshold testing before disengagement.

Referring to **FIGURE 26** in combination with **FIGUREs 25A** and/or **25B,** a catheter tube assembly **2602** extends from a knob end **2616** to a socket end **2618** and encloses an internal lumen **2620** configured to axially slide over the stylet **2604** and engage the biostimulation device **2500A, 2500B.** The catheter tube assembly **2602** comprises a socket **2622** at the socket end **2618** that engages a corresponding nut **2508** on the biostimulation device **2500A, 2500B** whereby the stylet **2604** can be rotated relative to the catheter tube assembly **2602** for disengagement of the stylet threaded end **2610** from the biostimulation device threaded end **2504.** The stylet **2604** can be configured to screw into a multiple-sided nut **2508** attached to the biostimulation device **2300A, 2300B** and the socket **2622** can be configured as a locking hex socket that engages the multiple-sided nut **2508** on the biostimulation device **2500A, 2500B** whereby the stylet **2604** and socket **2622** are configured to enable counter-rotation of the stylet 2604 relative to the biostimulation device **2500A, 2500B.**

In the embodiment depicted in **FIGURE 26** in combination with **FIGUREs 25A** and **25B****,** the catheter tube assembly **2602** is a sheath-less catheter and the biostimulation device **2500A, 2500B** can be a leadless cardiac pacemaker with the delivery system **2600** configured to deploy the leadless cardiac pacemaker with active **2510A** or passive **2510B** fixation using the sheath-less catheter **2602.**

A sheath-less catheter socket **2622** can comprise a socket adapted to enable rotational counter traction for stylet disengagement. A biocompatible soluble protective covering **2518** configured to cover a fixation member **2510A, 2510B** can be coupled to the biostimulation device **2500A, 2500B** during insertion of the biostimulation device into a patient's body whereby the patient's body tissue is protected. In various embodiments, the biocompatible soluble protective covering **2518** can comprise mannitol, polyvinylpyrrolidone, a protective salt, or other suitable material.

The biocompatible soluble protective covering **2518** is most suitably selected to comprise a material that forms a protective capsule at room temperature, dissolves when implanted, and has no toxic side effects. For example, the biocompatible soluble protective covering **2518** can be selected to dissolve in a selected time after which a fixation device **2510A, 2510B** coupled to the biostimulation device **2500A, 2500B** is exposed. The fixation device **2510A, 2510B** is typically advanced by rotating the catheter tube assembly **2602** and the stylet **2604** until the biostimulation device **2500A, 2500B** is anchored.

In the illustrative embodiment, the catheter tube assembly **2602** circumferentially encloses an inner lumen **2620** and the stylet **2604** extending through the lumen **2620.** The catheter tube assembly **2602** comprises a catheter knob **2624** at a proximal end **2616** and a multiple-sided nut socket **2622** at a distal end **2618** of the catheter tube assembly **2602.** The multiple-sided nut socket **2622** is configured to receive external features of a multiple-sided nut 2508 coupled to the biostimulation device **2500A, 2500B.** The multiple-sided nut socket **2622** and biostimulation device's multiple-sided nut **2508** are formed to prevent counter-rotation of the biostimulation device **2500A, 2500B** and the catheter tube assembly **2602** when engaged. The stylet **2604** comprising a stylet knob **2624** at a proximal end **2616** and a threaded screw **2626** at a distal end **2618** of the stylet **2604.** The threaded screw **2626** is configured for engaging the internally threaded nut **2508** of the biostimulation device **2500A, 2500B.**

In a particular sample embodiment, the catheter tube assembly **2602** can be extruded from polyurethane, polytetrafluoroethylene, polyolefin, polyvinyl chloride or silicone. The diameter of the catheter tube assembly **2602** can be smaller than or equal to the biostimulation device diameter. For example, the catheter knob **2624** can be constructed from rigid plastic or metal and the stylet **2604** constructed from stainless steel. Typically, the stylet knob **2624** can be constructed from rigid plastic or metal. Elements and components of the catheter tube assembly **2602** can be constructed from any suitable material in addition to the materials specifically identified herein.

The biostimulation device **2500A, 2500B** can be configured as a leadless cardiac pacemaker.

In various embodiments, for example the structures depicted in **FIGUREs 24** and **26****,** the stylet **2404, 2604** and the catheter tube **2406, 2606** comprise the delivery system **2400, 2600** whereon two concentric members alone form a catheter **2402, 2602.** The stylet **2404, 2604** and the catheter tube **2406, 2606** are configured for implanting the biostimulation device which is adapted for either active or passive fixation to patient tissue. In some embodiments, a radio-opaque marker can be adapted for coupling to the stylet **2404, 2604,** the catheter tube **2406, 2606,** and/or the biostimulation device for identification under fluoroscopy and facilitation of positioning.

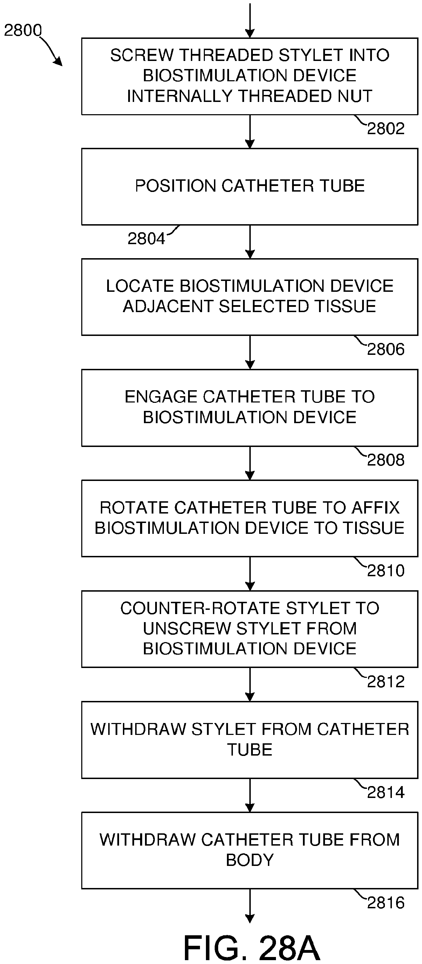

Referring to **FIGURE 28A** in combination in the context of **FIGUREs 23** through **27****,** a flow chart depicts an embodiment of a method **2800** for implanting a biostimulation device **2300A, 2300B, 2500A, 2500B** in patient body tissue. The method **2800** comprises screwing **2802** a threaded end of a stylet into an internally threaded nut of the biostimulation device and positioning **2804** the catheter tube to locate **2806** the biostimulation device adjacent a selected patient body tissue. A feature of the catheter tube is engaged **2808** against a corresponding feature on the biostimulation device so that rotation of the catheter tube rotates the biostimulation device relative to the patient body tissue. The catheter tube is rotated **2810** to affix the fixation device on the biostimulation device to the patient body tissue. The stylet is counter-rotated **2812** relative to the catheter tube to unscrew the threaded end of a stylet from the internally threaded nut of the biostimulation device.

The stylet can be withdrawn **2814** from the catheter tube and the catheter tube withdrawn **2816** from the patient's body. In a typical embodiment, the biostimulation device can be a leadless cardiac pacemaker and the patient body tissue for implanting the pacemaker is cardiac tissue.

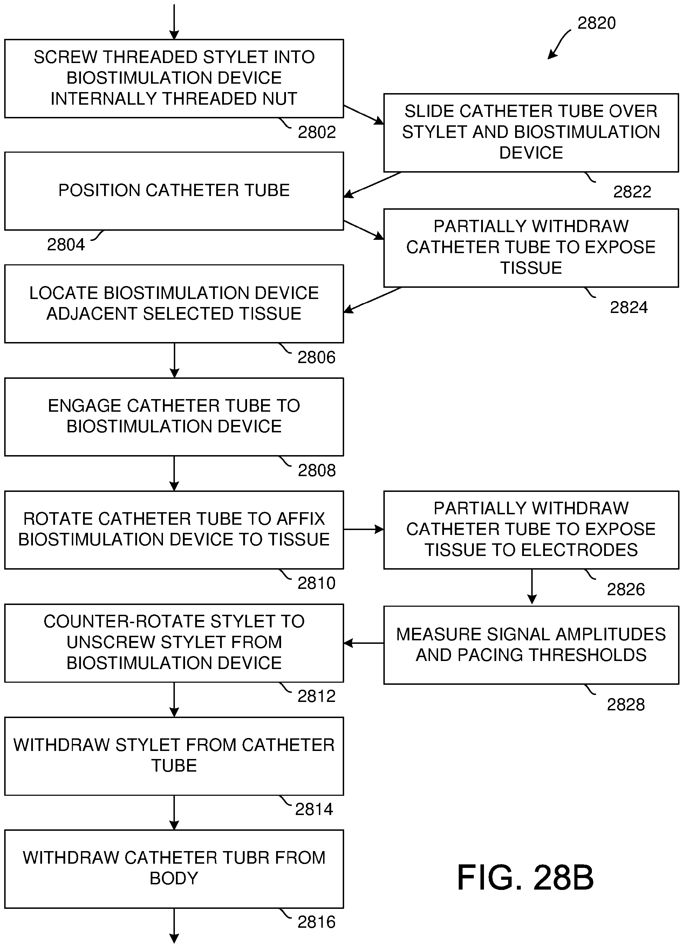

Referring to **FIGURE 28B****,** in some embodiments a method **2820** can further comprise, prior to positioning **2804** the catheter tube, axially sliding **2822** a catheter tube over the stylet and the biostimulation device so that the stylet and biostimulation device are internally contained within the catheter tube. Also in various embodiments, prior to locating **2806** the biostimulation device adjacent the selected patient body tissue, the catheter tube can be partially withdrawn **2824** the catheter tube to expose patient body tissue to the fixation device on the biostimulation device for affixation. Prior to unscrewing **2812** the stylet from the biostimulation device, the catheter tube can be partially withdrawn **2826** to expose patient body tissue to biostimulation device electrodes so that signal amplitudes and pacing thresholds of the biostimulation device can be measured **2828.** According to the measurements, the biostimulation device can be repositioned and parameters retested until measurements attain predetermined levels.

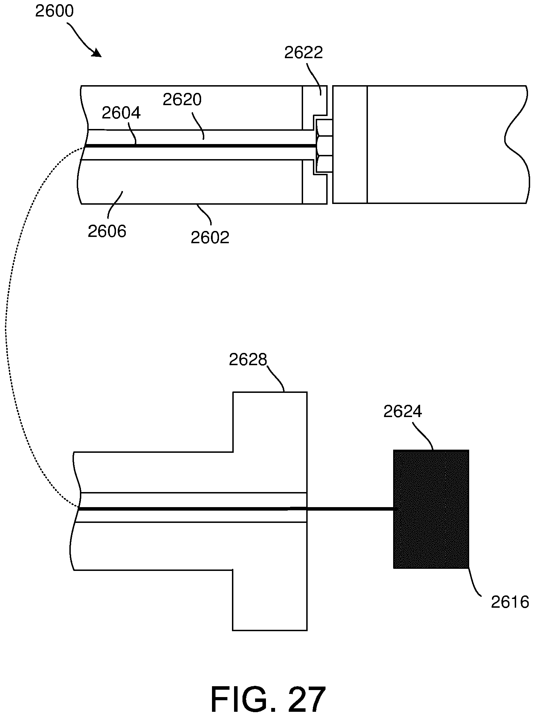

The lumen/stylet catheter **2602** depicted in **FIGUREs 26** or **27** can be used to place a LCP **2500A, 2500B** in the cardiovascular system. Once located, in the case of an LCP **2500A** with active fixation, the LCP is advanced to the desired location and time is allowed for the protective coating to dissolve thus exposing the helix **2510A.** Once exposed, the helix **2510A** is advanced by rotating both the lumen assembly knob **2628** and stylet knob **2624** until the LCP **2500A** is anchored. Unlike the sheath/stylet catheter **2400,** catheter pacing and sensing tests can be performed immediately.

As with both active and passive fixation LCPs **2500A, 2500B,** the lumen/stylet catheter **2600** can be disengaged by holding the lumen assembly knob **2628** and rotating the stylet knob **2624** to unscrew the stylet **2604** from the stylet hex nut **2508.** After the screw **2626** and nut **2508** are apart, the lumen/stylet catheter **2602** can be retracted.

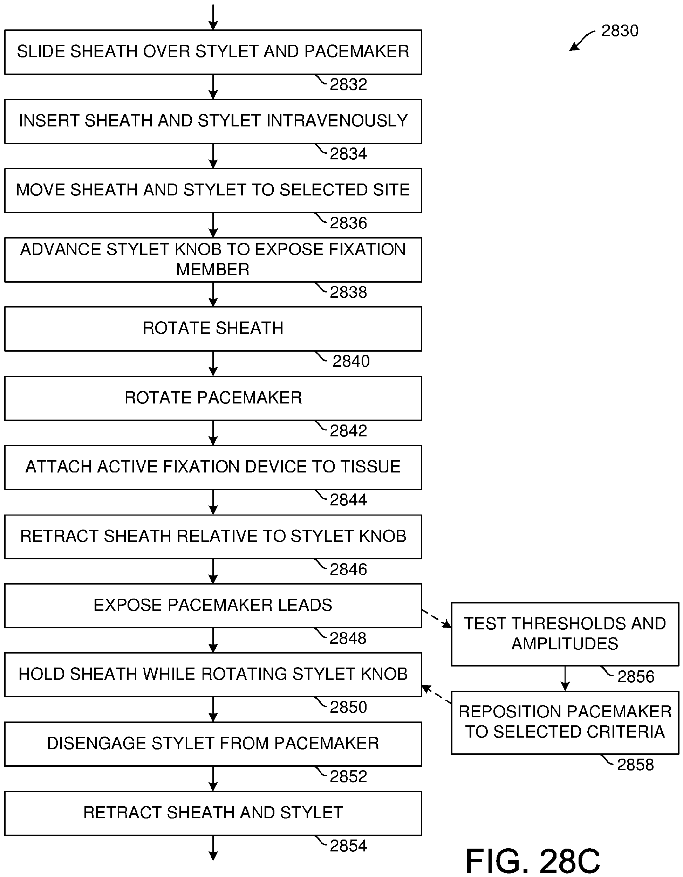

Referring to **FIGURE 28C** in combination in the context of **FIGUREs 23A** and **24****,** a flow chart depicts an embodiment of a method **2830** for implanting a biostimulation device **2300A** in patient body tissue using a sheathed delivery system and active fixation.

In typical usage of the sheath/stylet catheter system **2400** for use with a LCP **2300A** configured with active fixation **2310A,** initially the stylet hex nut **2308** connects to the stylet screw **2426** shown in **FIGUREs 23A** and **24****,** respectively. When the stylet knob **2416** is fully retracted, the LCP **2300A** is fully contained within the sheath **2412** protecting the helix **2310A.** The LCP **2300A** alignment pin **2306** aligns with the sheath slot **2414** to prevent rotation of the LCP **2300A** with respect to the sheath **2412.** When the stylet knob **2416** is fully depressed the alignment pins **2306** on the LCP **2300A** remain within the sheath **2412,** however both electrodes **2312** are fully exposed, enabling testing of the LCP **2300A** before disengagement.

The sheath/stylet catheter system **2400** and LCP **2300A** can be inserted intravenously either in the cephalic, subclavian, or femoral vein and moved progressively towards the heart until the distal end of the sheath **2412** reaches the selected site. An LCP **2300A** with active fixation is typically implanted in either the right atrium or ventricle. When the selected position is obtained, the stylet knob **2424** is advanced gently to expose the helix screw **2426.** The sheath knob **2428** is then used to rotate the entire assembly **2400** enabling the helix **2310A** to attach to the cardiac muscle.

When the LCP **2300A** is sufficiently anchored, the sheath knob **2428** is pulled back relative to the stylet knob position to expose both electrodes **2312.** Pacemaker testing can be performed with the implanted LCP **2300A** while still connected to the delivery system **2400.** When adequate pacing thresholds and signal amplitudes have been verified, the catheter system **2402** can be disengaged from the LCP **2300A** by holding the sheath knob **2428** and rotating the stylet knob **2424** to unscrew the stylet **2404** from the stylet hex nut **2308.** Once the screw **2426** and nut **2308** are apart, the sheath/stylet catheter **2402** can be retracted.

The method **2830** comprises axially sliding **2832** a sheath over the stylet and a leadless cardiac pacemaker so that the stylet and leadless cardiac pacemaker with active fixation member are internally contained within the sheath. The sheath and stylet combination are inserted **2834** intravenously either in a cephalic, subclavian, or femoral vein of a patient. The sheath and stylet combination are progressively moved **2836** towards patient cardiac tissue until a distal end of the sheath reaches a selected site of the cardiac tissue. At the selected site, a stylet knob is gently advanced **2838** into the sheath to expose the active fixation member coupled to the leadless cardiac pacemaker. The sheath is rotated **2840,** thereby rotating **2842** the leadless cardiac pacemaker and attaching **2844** the active fixation member to the cardiac tissue. Upon sufficient attachment of the active fixation member, the sheath is retracted **2846** relative to the stylet knob, exposing **2848** electrodes of the leadless cardiac pacemaker. The sheath is held **2850** while rotating the stylet knob, unscrewing and disengaging **2852** the stylet from the leadless cardiac pacemaker. The sheath and stylet combination is retracted **2854** from the patient's cephalic, subclavian, or femoral vein.

After exposure **2848** of leadless cardiac pacemaker leads, implanted leadless cardiac pacemaker pacing thresholds and signal amplitudes can be tested **2856** and the leadless cardiac pacemaker repositioned **2858** until the pacing thresholds and signal amplitudes meet selected criteria.

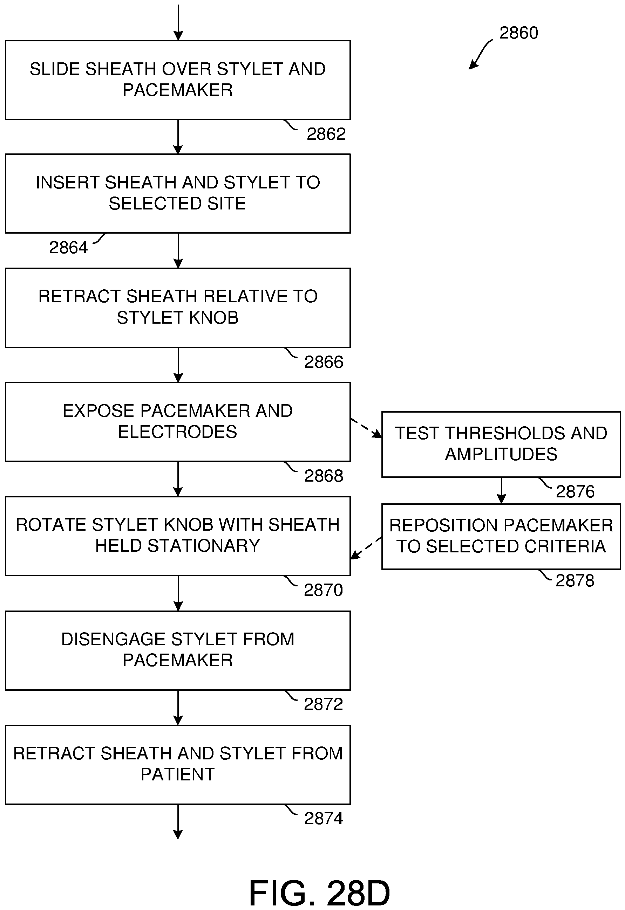

Referring to **FIGURE 28D** in combination in the context of **FIGUREs 23B** and **24****,** a flow chart depicts an embodiment of a method **2860** for implanting a biostimulation device **2300B** in patient body tissue using a sheathed delivery system and passive fixation.

In typical usage of the sheath/stylet catheter system **2400** for use with a LCP **2300B** configured with active fixation **2310B,** the LCP **2300B** and tines **2310B** are fully contained within the sheath **2412** when the stylet knob **2424** is fully retracted. Because LCP **2300B** with passive fixation devices **2310B** are typically located in the coronary sinus, the sheath/stylet catheter system **2400** is typically used with a coronary sinus introducer system in which a guide wire is first inserted and positioned under fluoroscopy to the desired location. A dilator and introducer can be advanced over the guide wire. Once fully inserted, the dilator and guide wire are removed, leaving the introducer. The sheath/stylet catheter assembly **2400** including the LCP **2300B** can then be advanced to the selected position. The LCP **2300B** advances ahead of the introducer to enable exposure of the LCP electrodes **2312** to tissue once the sheath **2412** is retracted. To expose the LCP **2300B** and expose both electrodes **2312** the sheath knob **2428** are pulled back relative to the stylet knob position. After pacemaker testing confirms the correct placement of the LCP **2300B,** the stylet **2404** can be disengaged from the LCP **2300B** by holding the sheath knob **2428** and rotating the stylet knob **2424.** After the screw **2426** and nut **2308** are apart, both the introducer and sheath/stylet catheter **2402** can be retracted.

The method **2860** comprises axially sliding **2862** a sheath over the stylet and a leadless cardiac pacemaker so that the stylet and leadless cardiac pacemaker with passive fixation member are internally contained within the sheath. The sheath and stylet are inserted **2864** in combination to a selected location of a patient's body tissue and, at the selected site, the sheath is gently retracted **2866** relative to the stylet knob position, exposing **2868** the leadless cardiac pacemaker and the electrodes. The stylet knob is rotated **2870** while holding the sheath stationary, disengaging **2872** the stylet from the leadless cardiac pacemaker and retracting **2874** the sheath and stylet combination from the patient.

After exposure **2868** of leadless cardiac pacemaker leads, implanted leadless cardiac pacemaker pacing thresholds and signal amplitudes can be tested **2876** and the leadless cardiac pacemaker repositioned **2878** until the pacing thresholds and signal amplitudes meet selected criteria.

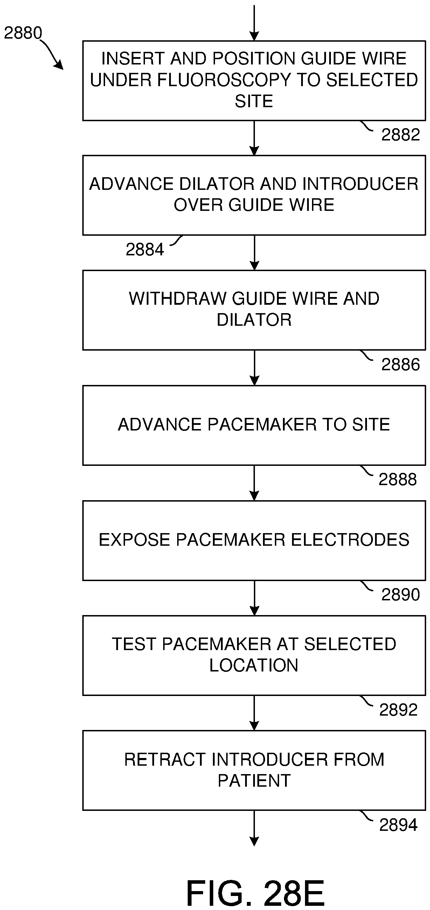

Referring to **FIGURE 28E** in combination in the context of **FIGUREs 23B** and **24****,** a flow chart depicts another embodiment of a method **2880** for implanting a biostimulation device **2300B** in patient body tissue using a sheathed delivery system and passive fixation. The method **2880** comprises inserting and positioning **2882** a guide wire under fluoroscopy imaging to a selected location in a patient's coronary sinus using a coronary sinus introducer system and advancing **2884** a dilator and introducer over the guide wire to the selected location. The guide wire can be withdrawn **2886** followed by the dilator so that the introducer remains positioned at the selected location. The leadless cardiac pacemaker is advanced **2888** into the coronary sinus for positioning at the selected location, exposing **2890** leadless cardiac pacemaker electrodes. The leadless cardiac pacemaker can be tested **2892** at the selected location before retracting **2894** the introducer from the patient.

The LCP **2300A, 2300B, 2500A, 2500B** and/or either catheter system **2400, 2600** can contain radio-opaque markers for identification under fluoroscopy to aid in positioning.

An external programmer can be used with a system of one or more leadless cardiac pacemakers. Individual leadless cardiac pacemakers can be implanted adjacent to the inside or outside wall of a cardiac chamber. The programmer uses a minimum of two electrodes in electrical contact with the skin to communicate with each pacemaker through conduction. Information is passed from programmer to implant through a modulation technique designed to avoid stimulation of skeletal muscles. Communication from implant to programmer is performed by encoding information on the pacing pulses.

The programmer includes a user interface to display status and settings information for one or more individual implantable pacemakers, and enables the user to change programmable parameters on an individual implantable pacemaker. The programmer also can display the electrocardiogram sensed from the same two external electrodes on the skin. The programmer can perform tasks including electrocardiogram sensing, retrieving status information from implantable pacemakers, and changing configuration parameters of the implantable pacemakers simultaneously through the same set of electrodes.

Use of conducted communication of information improves over standard methods of communication in several aspects. For example, the illustrative conductive techniques enable communication without requiring a programmer head to be held undesirably close to the patient or to be held in a precise position relative to the implant site for an extended period of time. The illustrative conductive communication also enables power consumption to be reduced due to substantially lower current requirements and eliminating peak power demands currently imposed by existing inductive and radio frequency (RF) systems. Also, the conductive communication technique uses elements generally already existing in the implanted pulse generator, such as the therapeutic electrodes that function as an input-output device, enabling elimination of a coil or antenna that are conventionally used for communication and reducing complexity and component count significantly.

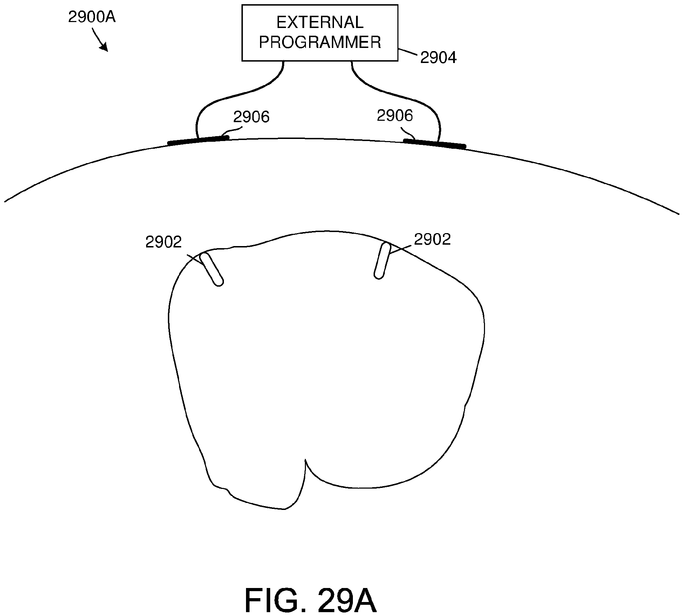

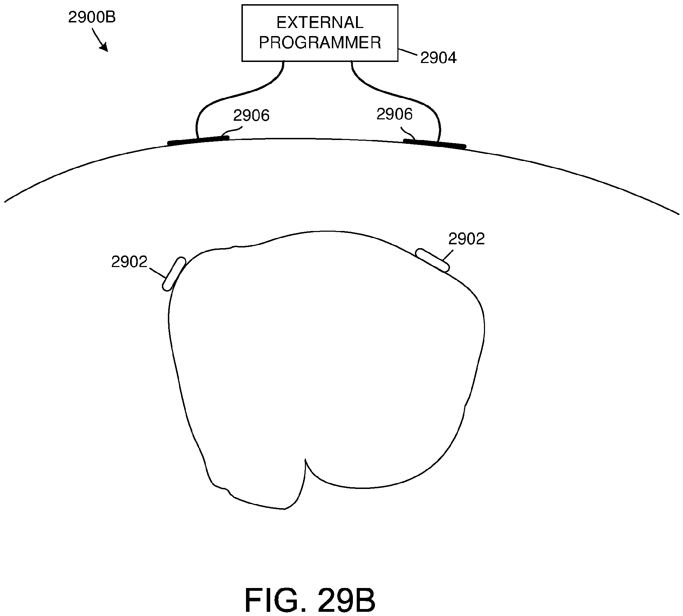

Referring to **FIGUREs 29A** and **29B****,** schematic pictorial views depict embodiments of biostimulator systems **2900A, 2900B** that communicate via conductive communication. The biostimulator systems **2900A, 2900B** comprise one or more implantable devices **2902** and an external programmer **2904** configured for communicating with the one or more implantable devices **2902** via bidirectional communication pathways comprising a receiving pathway that decodes information encoded on stimulation pulses generated by one or more of the implantable devices **2902** and conducted through body tissue to the external programmer **2904.**

According to the illustrative arrangement, the bidirectional communication pathways can be configured for communication with multiple leadless cardiac pacemakers **2902** via two or more electrodes **2906** and conduction through body tissue.

In accordance with various biostimulator system embodiments, an external device or module **2904** is connected by a communication transmission channel and has transmitting and receiving functional elements for a bidirectional exchange of information with one or more implanted medical devices **2902.** The communication channel includes two or more electrodes **2906** which can be affixed or secured to the surface of the skin. From the point of the skin, the communication transmission channel is wireless, includes the ion medium of the intra- and extracellular body liquids, and enables electrolytic-galvanic coupling between the surface electrodes and the implantable modules **2904.**

In the biostimulator systems **2900A, 2900B,** the bidirectional communication pathways can further comprise a transmitting pathway that passes information from the external programmer **2904** to one or more of the implantable devices **2902** by direct conduction through the body tissue by modulation that avoids skeletal muscle stimulation using modulated signals at a frequency in a range from approximately 10 kHz to 100 kHz.

Information transmitted from the external programmer **2904** to the implanted devices **2902** is conveyed by modulated signals at the approximate range of 10 kHz to 100 kHz which is a medium-high frequency. The signals are passed through the communication transmission channel by direct conduction. A modulated signal in the frequency range has a sufficiently high frequency to avoid any depolarization within the living body which would lead to activation of the skeletal muscles and discomfort to the patient. The frequency is also low enough to avoid causing problems with radiation, crosstalk, and excessive attenuation by body tissue. Thus, information may be communicated at any time, without regard to the heart cycle or other bodily processes. No restriction is imposed regarding location of electrode placement on the body because low signal attenuation enables the signal to travel throughout the body and to be received by the implanted devices **2902.**

In some embodiments, the bidirectional communication pathways can further comprise a receiving pathway including a low-pass filter adapted to separate the electrocardiogram from the information signals. The same surface electrodes **2906** that are used to transmit the information through the communication channel may also be used to detect a patient's electrocardiogram. Electrocardiogram frequencies are generally between 1 and 100 Hz, far lower than the 10 kHz to 100 kHz range of frequencies used to transmit information through the communication transmission channel. Therefore, the electrocardiogram can be separated from the information signal by a low-pass filter and can optionally be displayed by the programmer **2904.** In addition to low-pass filtering, blanking techniques that are typical in processing of cardiac signals can be used when the communication channel is active to prevent noise or erroneous signals from the communication channel affecting the electrocardiogram channel.

Because a plurality of implantable devices **2902** can be present, communication of information from the programmer is detected by all devices, enabling information to be sent to each implanted device without sending the same information multiple times.

In various embodiments and applications, the bidirectional communication pathways can further comprise a transmitting pathway that passes information from the programmer **2904** to the one or more implantable devices **2902** in a common communication event whereby information is sent to one or more target devices of the implantable devices **2902** using a selected technique. For example, information specific to a single implantable device or a subset of implantable devices having a unique address can be assigned to the single implantable device or the subset of implantable devices and encoded in the information. In another technique, information can designate a specific function that is executed by a particular implantable device or a particular subset of implantable devices. The information is passed to one or more implantable devices without sending individual address information for activating execution by the particular implantable device or the particular subset of implantable devices alone. In another technique, information can designate a specific function that is executed by a particular implantable device or a particular subset of implantable devices that have programming specific to the function adapted to recognize the received information is relevant to the function.

Specifically, information that is specific to a single implanted device or a subset of devices can be sent. A unique address can be assigned to each device or subset. The address can be encoded in the information sent to the plurality of devices, and any individual device can make use only of information that matches either the address or the address of the subset to which the particular device belongs.

In another technique, if each implanted device **2902** or subset of devices **2902** serves a specific function, which is different from other implanted devices, then information may be passed to the specific device or subset without the additional overhead of a group or individual address. For example, the device or subset can be responsible for only a specific function. When the programmer transmits information to the entire group, but the information is relevant to only the device or subset of that group, then any devices that cannot make use of the information may ignore the information. Each device has unique programming specific to a particular function and can recognize whether received information is relevant to the function. Devices operative in conjunction with the technique can be non-generic and perform specific functions, or can be generic devices with general functionality that can be made more specific by programming. Accordingly, functionality of a device can be defined at manufacture or may be defined at implantation or thereafter. The function of each device can be defined at the time of manufacture and the devices labeled or marked such that the associated function can be known upon inspection.

In some embodiments, the one or more implantable devices **2902** can comprise one or more leadless cardiac pacemakers that generate cardiac pacing pulses and encode information onto the generated cardiac pacing pulses by selective alteration of pacing pulse morphology that is benign to therapeutic effect and energy cost of the pacing pulse. The cardiac pacing pulses conduct into body tissue via the electrodes for antenna-less and telemetry coil-less communication. For information transmitted from the implanted leadless cardiac pacemaker 2902 to the external programmer **2904,** a communication scheme can be used in which the information is encoded on the pacing pulse. The pulse morphology is altered to contain the encoded information without altering the therapeutic benefits of the pacing pulse. The energy delivered by the pacing pulse remains essentially the same after the information is encoded. The external programmer **2904** receives the pacing pulses through the associated surface electrodes **2906.** Encoded information is drawn from the pacing pulses and can contain state information ofthe implantable leadless cardiac pacemaker, such as battery voltage, lead impedance, sensed electrocardiogram amplitude, pacemaker current drain, programmed parameters, or other parameters.

The leadless cardiac pacemaker or pacemakers **2902** can be configured to detect a natural cardiac depolarization, time a selected delay interval, and deliver an information-encoded pulse during a refractory period following the natural cardiac depolarization. By encoding information in a pacing pulse, power consumed for transmitting information is not significantly greater than the power used for pacing. Information can be transmitted through the communication channel with no separate antenna or telemetry coil. Communication bandwidth is low with only a small number of bits encoded on each pulse.

In some embodiments, information can be encoded using a technique of gating the pacing pulse for very short periods of time at specific points in the pacing pulse. During the gated sections of the pulse, no current flows through the electrodes of a leadless cardiac pacemaker. Timing of the gated sections can be used to encode information. The specific length of a gated segment depends on the programmer's ability to detect the gated section. A certain amount of smoothing or low-pass filtering of the signal can be expected from capacitance inherent in the electrode/skin interface of the programmer as well as the electrode/tissue interface of the leadless cardiac pacemaker. A gated segment is set sufficiently long in duration to enable accurate detection by the programmer **2904,** limiting the amount of information that can be transmitted during a single pacing pulse. Accordingly, a technique for communication can comprise generating stimulation pulses on stimulating electrodes of an implanted biostimulator and encoding information onto generated stimulation pulses. Encoding information onto the pulses can comprise gating the stimulation pulses for selected durations at selected timed sections in the stimulation pulses whereby gating removes current flow through the stimulating electrodes and timing of the gated sections encodes the information.

Another method of encoding information on pacing pulses involves varying the timing between consecutive pacing pulses in a pulse sequence. Pacing pulses, unless inhibited or triggered, occur at predetermined intervals. The interval between any two pulses can be varied slightly to impart information on the pulse series. The amount of information, in bits, is determined by the time resolution of the pulse shift. The steps of pulse shifting are generally on the order of microseconds. Shifting pulses by up to several milliseconds does not have an effect on the pacing therapy and cannot be sensed by the patient, yet significant information can be transmitted by varying pulse intervals within the microsecond range. The method of encoding information in variation of pulses is less effective if many of the pulses are inhibited or triggered. Accordingly, a technique for communication can comprise generating stimulation pulses on stimulating electrodes of an implanted biostimulator and encoding information onto generated stimulation pulses comprising selectively varying timing between consecutive stimulation pulses.

Alternatively or in addition to encoding information in gated sections and/or pulse interval, overall pacing pulse width can be used to encode information.

The three described methods of encoding information on pacing pulses can use the programmer **2904** to distinguish pacing pulses from the patient's normal electrocardiogram, for example by recognition of the specific morphology of the pacing pulse compared to the R-wave generated during the cardiac cycle. For example, the external programmer **2904** can be adapted to distinguish a generated cardiac pacing pulse from a natural cardiac depolarization in an electrocardiogram by performing comparative pattern recognition of a pacing pulse and an R-wave produced during a cardiac cycle.

The illustrative external programmer **2904** and associated operating methods or techniques enable presentation to the user of information gathered from the implanted biostimulator or leadless cardiac pacemakers **2902** using conductive communication. Some of the information to be presented may include battery voltage, lead impedance, electrocardiogram amplitude, or current drain ofthe device. The information can be presented in addition to other information such as parameters to be set and programmed into the leadless cardiac pacemaker. The information can be presented to a user on a display screen. Some embodiments or configurations of an external programmer **2904** can include a secondary link, for example either wireless or through a cable, to another display device, such as a handheld computer or terminal. The secondary link can also include communication over a local area network or the internet for display at a remote terminal.

**FIGURE 29A** depicts a sample configuration involving the external programmer **2904** and two endocardially implanted leadless cardiac pacemakers **2902.** The external programmer **2904** is physically connected to the skin surface via two electrodes **2906,** which serve three functions. First, the electrodes **2906** transmit encoded information from the programmer **2904** to the implanted leadless cardiac pacemakers **2902** using a modulated signal at a medium frequency 10 kHz to 100 kHz. Second, the electrodes **2906** receive information from individual leadless cardiac pacemakers **2902** by detecting encoded information in the pacing pulses ofthe leadless cardiac pacemakers **2902.** Third, the electrodes **2906** receive surface electrocardiogram for display and analysis by the programmer **2904.**

In **FIGURE 29A****,** the two leadless cardiac pacemakers **2902** are implanted endocardially. Thus, in a biostimulator system **2900A** or **2900B** an implantable device **2902** may comprise one or more leadless cardiac pacemakers that can be implanted adjacent to an inside or an outside wall of a cardiac chamber. Referring to **FIGURE 29B****,** a similar system is represented with a difference that the two leadless cardiac pacemakers **2902** are implanted by affixing to the exterior surface of the heart. The electrodes **2906** and programmer **2904** function similarly in arrangements shown in **FIGUREs 29A** and **29B** whether the leadless cardiac pacemakers **2902** are implanted endocardially or epicardially (on the external heart surface). No restriction is imposed that the leadless cardiac pacemakers are all implanted inside or all implanted outside the heart. One or more may be implanted endocardially along with others implanted on the outer surface of the heart. The functioning of the programmer **2904** is substantially the same. Although two electrodes **2906** are shown in **FIGUREs 29A** and **29B****,** two is generally the minimum number for adequate conductive communication. More electrodes can be used, enabling an electrocardiogram (ECG) to be sensed at multiple vectors for better analysis. More than two electrodes also enable a choice of vectors for conducted communication with the leadless cardiac pacemakers, thereby maximizing the signal to noise ratio of the system. **FIGUREs 29A** and **29B** each depict two leadless cardiac pacemakers **2902.** One, two, or more leadless cardiac pacemakers can be implanted, depending on the number of pacemakers appropriate for effective therapy.

In various embodiments, the external programmer **2904** can be configured to perform one or more operations such as electrocardiogram sensing, retrieving status information from implanted pacemakers, modifying configuration parameters of multiple implanted pacemakers simultaneously in information passed through a common electrode set, displaying electrocardiograms, displaying information received from the at least one implantable device, and others.

In various embodiments, a pacemaker **2902** can manage power consumption to draw limited power from an internal battery, thereby reducing device volume. Each circuit in the pacemaker can be designed to avoid large peak currents. For example, cardiac pacing can be achieved by discharging a tank capacitor (not shown) across the pacing electrodes. Recharging of the tank capacitor is typically controlled by a charge pump circuit. In a particular embodiment, the charge pump circuit can be throttled to recharge the tank capacitor at constant power from the battery. The one or more leadless cardiac pacemakers can be configured to charge the tank capacitor in preparation for stimulation pulse generation, time one or more windows between pulse generation, disable charging of the tank capacitor during the one or more timed windows, and enable a receive amplifier in the implanted biostimulator while the tank capacitor is disabled.

In some embodiments, the external programmer **2904** can detect a stimulation pulse from a leadless cardiac pacemaker **2902** and transmit data after a selected delay to coincide with a window that the leadless cardiac pacemaker's receiving amplifier is enabled.

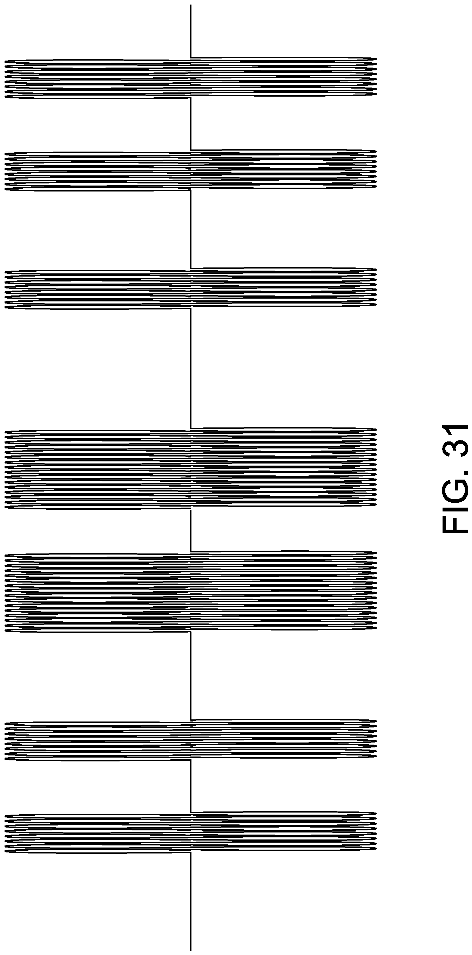

The implantable devices **2902** can encode and/or decode information using various techniques such as encoding the information using pacing pulse width, binary-coded notches in a pacing pulse, modulation of off-time between pacing pulses, or other suitable encoding techniques. The external programmer **2904** can encode and/or decode information using on-off keying encoding and modulation techniques depicted in **FIGURE 31****.** However, any other appropriate method can be used whereby a modulated bit-stream can be generated at a medium high frequency, for example frequency-shift keying, frequency modulation, or amplitude shift keying.

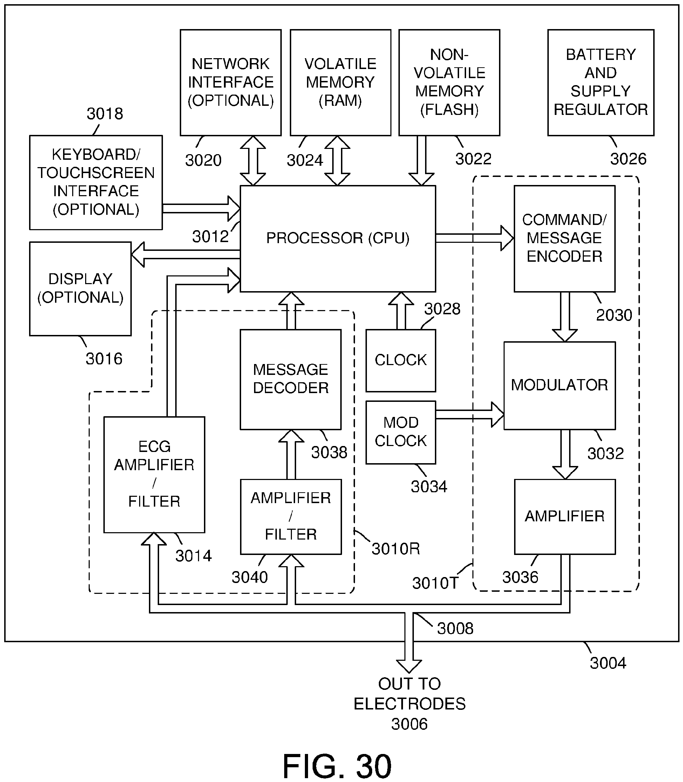

Referring to **FIGURE 30****,** a schematic block diagram shows an embodiment of an external programmer **3004** adapted for communicating with an implanted biostimulator system using conducted communication. The external programmer **3004** comprises an interface **3008** configured for coupling to at least two electrodes **3006** that make electrical contact with body skin for communicating with one or more implanted biostimulators. The external programmer **3004** further comprises bidirectional communication pathways **3010R** and **3010T** coupled to the interface **3008** and configured for bidirectional communication with the one or more implanted biostimulators. The communication pathways comprise a receiving pathway **3010R** that decodes information encoded on stimulation pulses generated by the one or more implanted biostimulators and conducted through body tissue.

The bidirectional communication pathways **3010R** and **3010T** are configured for communication with one or more leadless cardiac pacemakers via the electrodes **3006** and conduction through body tissue.

The external programmer **3004** can have bidirectional communication pathways **3010R** and **3010T** that further comprise a transmitting pathway **3010T** that passes information from the programmer **3004** to one or more implanted biostimulators by conduction through the body tissue using modulation that avoids skeletal muscle stimulation.

In some arrangements, the bidirectional communication pathways **3010R** and **3010T** can be further specified to comprise a transmitting pathway that passes information from the programmer **3004** to the one or more implanted biostimulators by direct conduction using modulated signals at a frequency in a range from approximately 10 kHz to 100 kHz. Also in some arrangements, the two or more electrodes **3006** and the bidirectional communication pathways **3010R** and **3010T** can be configured for bidirectional information signal communication and for sensing an electrocardiogram.

Also in some embodiments, the bidirectional communication pathways **3010R** and **3010T** can further comprise a transmitting pathway **3010T** that passes information from the programmer **3004** to multiple implanted devices in a common communication event. In some embodiments or selected operating conditions, the transmitting pathway **3010T** can be arranged to pass information from the programmer **3004** to multiple implanted devices in a common communication event whereby information specific to a single implanted device or a subset of implanted devices have a unique address assigned to the single implanted device or the subset of implanted devices and encoded in the information. The transmitting pathway **3010T** can also be arranged to pass information from the programmer **3004** to multiple implanted devices in a common communication event whereby information designates a specific function that is executed by a particular implanted device or a particular subset of implanted devices. The information is passed to the multiple implanted devices without individual address information for activating execution by the particular implanted device or the particular subset of implanted devices alone. The transmitting pathway **3010T** can also be arranged, either alone or in combination with other techniques, to pass information from the programmer **3004** to multiple implanted devices in a common communication event whereby information designates a specific function that is executed by a particular implanted device or a particular subset of implanted devices that comprise programming specific to the function adapted to recognize the received information is relevant to the function.

In the illustrative embodiment, the bidirectional communication pathways **3010R** and **3010T** comprise the two or more electrodes **3006** forming a conductive communication path between the programmer **3004** and the skin surface, and a transmitting pathway **3010T.** The transmitting pathway **3010T** comprises a processor **3012,** a command/message encoder **3030,** a modulator **3032,** and an amplifier **3036.** The processor **3012** is configured to communicate information to one or more implanted leadless cardiac pacemakers. The command/message encoder **3030** is coupled to the processor **3012** via a parallel interface and configured to encode and serialize data into a bit stream. Information encoding can be selected from encoding techniques such as on-off keying, frequency-shift keying, frequency modulation, and amplitude shift keying. The modulator **3032** is coupled to the command/message encoder **3030** and receives and modulates the serialized data using a frequency in a range from approximately 10 kHz to approximately 100 kHz. The amplifier **3036** is coupled to the modulator **3032** and increases signal amplitude to a level suitable for robust conducted communication.

The bidirectional communication pathways **3010R** and **3010T** further comprise a receiving pathway **3010R** including a low-pass filter **3014** adapted to separate the electrocardiogram from the information signals.

In various embodiments and arrangements, the bidirectional communication pathways **3010R** and **3010T** further comprise a receiving pathway **3010R** that receives information at the programmer **3004** from the one or more implanted biostimulators by conduction through the body tissue. The receiving pathway **3010R** can decode information, for example by decoding data that is encoded by the biostimulators using pacing pulse width, using binary-coded notches in a pacing pulse, using modulation of off-time between pacing pulses, or other suitable techniques for encoding data in the biostimulators.

In the illustrative embodiment, the bidirectional communication pathways **3010R** and **3010T** couple to the two or more electrodes **3006** forming a conductive communication path between the programmer **3004** and the skin surface, and a receiving pathway **3010R.** The receiving pathway **3010R** comprises an electrocardiogram (ECG) amplifier/filter **3014,** an analog-to-digital converter (ADC) which is not shown in **FIGURE 30****,** and the processor **3012.** The electrocardiogram (ECG) amplifier/filter **3014** includes a differential band-pass amplifier configured to select and amplify signals in a frequency range from approximately 1 Hz to approximately 100 Hz. The analog-to-digital converter (ADC) is configured to digitize the filtered and amplified signal. The processor **3012** is coupled to the ADC and configured to receive and optionally display ECG data, and configured to decode information encoded into cardiac pacing pulses.

The programmer **3004** may further comprise a processor **3012** coupled to the bidirectional communication pathways and configured to manage communication with one or more biostimulators, for example leadless cardiac pacemakers. Leadless cardiac pacemakers can be implanted adjacent to an inside or an outside wall of a cardiac chamber as depicted in **FIGUREs 29A** and **29B****.**

As depicted in **FIGURE 30****,** external electrodes **3006** enable a conductive communication path between the programmer **3004** and the skin surface. Electrocardiogram (ECG) signals enter an ECG amplifier/filter **3014,** which can include a differential band-pass amplifier. In general, an ECG signal has spectral components in a range between 1 Hz and 100 Hz. Band-pass filter poles for the ECG amplifier/filter **3014** can be selected such that sufficient signal energy is passed within the 1 Hz to 100 Hz range, while filtering other signals that are not associated with cardiac activity. The ECG signal can be amplified and digitized using an analog-to-digital converter (ADC). Once digitized, the signal is passed to the processor, for example central processing unit (CPU) **3012.**

In some embodiments, the electrodes **3006** can be implemented with more than two electrodes to enable an electrocardiogram (ECG) to be sensed at multiple vectors and further to enable selection from among the multiple vectors for conducted communication with implanted leadless cardiac pacemakers so that system signal-to-noise ratio can be improved or maximized.

The CPU **3012** receives and optionally displays ECG data using a display interface 3016 and can also display other data acquired from the implanted leadless cardiac pacemaker acquired through the encoded pacing pulses, such as battery voltage, lead impedance, sensed cardiac signal amplitude, or other system status information. The CPU **3012** also can accept input from a user via a keyboard and/or touch-screen interface **3018.** Some examples of user input are selected pacing rate or pacing pulse amplitude for implanted leadless cardiac pacemakers. The CPU **3012** can also communicate over a network interface **3020** to other data entry or display units, such as a handheld computer or laptop/desktop unit. The network interface **3020** can be cabled or wireless and can also enable communication to a local area network or the internet for greater connectivity.

The processor **3012** is coupled to the bidirectional communication pathways and configured to perform one or more of various operations such as electrocardiogram sensing, retrieving status information from implanted pacemakers, modifying configuration parameters of multiple implanted pacemakers within a single or multiple cardiac cycles in information passed through a common electrode set, and other operations. A display interface **3016** coupled to the processor **3012** can be configured to display an electrocardiogram sensed from the electrodes **3006.** In some arrangements or embodiments, a secondary link **3020** can be coupled to the processor **3012** and configured for unidirectional or bidirectional wireless or cable transmission to and/or from a remote display and/or data-entry device to display an electrocardiogram sensed from the at least two electrodes, and/or to control the programmer and/or at least one implanted biostimulator.

The CPU **3012** can execute operations based on firmware stored in non-volatile memory (Flash) **3022.** The non-volatile memory **3022** can also be used to store parameters or values that are to be maintained when power is removed. The CPU **3012** uses volatile memory or random access memory (RAM) **3024** as general storage for information such as ECG data, status information, swap memory, and other data. A battery and supply regulator **3026** gives a constant voltage supply to the programmer **3004** during normal operation. A clock module **3028** generates a system clock signal used by the CPU **3012** and by interface blocks for timing.

The CPU **3012,** during operation to communicate information to one or more implanted leadless cardiac pacemakers, sends the information over a parallel interface to a command/message encoder **3030,** which serializes the data into a bit stream. Serialized data is sent to a modulator **3032.** The serialized bit-stream is modulated, for example using a frequency between 10 kHz and 100 kHz. An optional separate modulator clock **3034** supplies a timing signal at a selected carrier frequency that may be used by the modulator **3032.** An amplifier **3036** sets signal amplitude to a level that enables robust conducted communication. A sample of a modulated bit-steam is shown in **FIGURE 31** wherein logic high is shown as a medium high frequency sine wave. An encoding and modulation technique depicted in **FIGURE 31** is on-off keying. However, any other appropriate method whereby a modulated bit-stream can be generated at a medium high frequency may be used, for example frequency shift keying, frequency modulation, or amplitude shift keying.

Because multiple biostimulator devices can be implanted, communication of information from the programmer **3004** can be detected by all devices, enabling information to be sent to each implanted device without sending the same information multiple times.

If information for communication is specific to a single implanted device or a subset of devices, a unique address can be assigned to each device or subset. The address is encoded in the information sent to the plurality of devices, and any individual device can have the option to make use of information that either matches the address or the address of the subset to which the particular device belongs.

If each implanted device or a subset of devices performs a specific function which is different from other implanted devices, then information can be passed to the specific device or subset without the additional overhead of a group or individual address. For example, when the device or subset is responsible for only a specific function. When the programmer **3004** transmits information to the entire group, but the information is relevant to only the device or subset of that group, then any devices that cannot make use of the information may ignore the information as superfluous. The technique presumes that each device have unique programming specific to the associated function, and each device have capability to recognize whether or not received information is relevant to the function. Devices using the illustrative technique are not generic. The function of each device can be defined at the time of manufacture or at the time of implant or thereafter. The devices are labeled or marked such that the associated function can be known upon inspection.

To reduce the peak current for operation of the leadless cardiac pacemakers, a technique can be used in which a window or multiple windows occur between subsequent pacing pulses during which the leadless cardiac pacemaker does not charge pacing tank capacitor in preparation for the next pacing pulse. Instead the pacemaker enables an internal receiving amplifier. Because the programmer **3004** can sense pacing pulses from the implanted devices, the programmer **3004** can time data transmission to coincide with the pre-defined synchronous window or windows. A reduced peak current capability occurs because the charger and receiving amplifier, both power intensive elements, never have to be operated together. Because the data transmission is generally very short compared to the period between pacing pulses, the window technique should not significantly lower the ability of the leadless cardiac pacemaker to charge the pacing tank capacitor effectively between pacing pulses.

Referring again to **FIGURE 30****,** data acquired by the programmer **3004** from a specific implanted leadless cardiac pacemaker is received at the surface electrodes **3006** and passes to an amplifier/filter **3040,** which functions to remove noise from the incoming signal. Any filtering performed by the amplifier/filter **3040** is designed to leave encoded pulses intact as much as possible. A message decoder **3038** determines whether the received signal is actually a pacing pulse or another signal, such as a cardiac R-wave.

The method of encoding data using modulation of off-time between pacing pulses is less effective if pulses are inhibited, since data can be transmitted using only pacing pulses generated by the pacemaker. When data are encoded in binary-coded notches in the pacing pulse or by varying pacing pulse width, if a therapeutic pacing pulse is inhibited, then the leadless cardiac pacemaker can still generate a non-therapeutic pulse during the refractory period of the heart after the sensed beat, although the pacing pulse has the sole purpose of transmitting data to the programmer or optionally to at least one other implanted biostimulator.

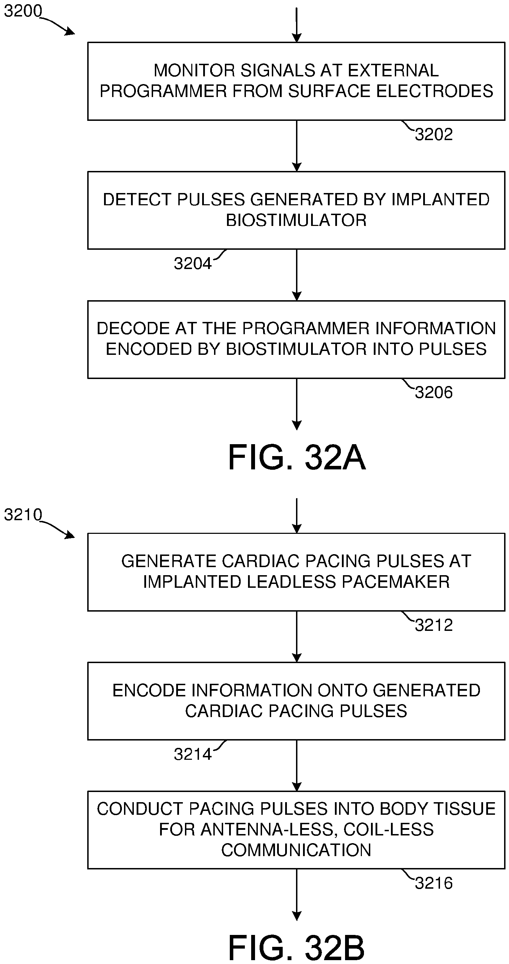

Referring to **FIGUREs 32A** through **32E****,** schematic flow charts depict techniques that can be used in various embodiments of methods for communicating in an implantable biostimulator system. According to **FIGURE 32A****,** an illustrative method **3200** comprises monitoring **3202,** at an external programmer, electrical signals conducted through body tissue to body surface electrodes and detecting **3204** pulses generated by a body-implanted biostimulator. The external pacemaker decodes **3206** information encoded into the generated pulse by the body-implanted biostimulator.

Referring to **FIGURE 32B****,** a method **3210** can further comprise generating **3212** cardiac pacing pulses at an implanted leadless cardiac pacemaker. Information is encoded **3214** onto generated cardiac pacing pulses at the implanted leadless cardiac pacemaker by selective alteration of pacing pulse morphology that is benign to therapeutic effect and energy cost of the pacing pulse. In various embodiments, the implanted leadless cardiac pacemaker can encode the information using one or more techniques such as encoding using pacing pulse width, using binary-coded notches in a pacing pulse, and using modulation of off-time between pacing pulses. The cardiac pacing pulses are conducted **3216** into body tissue via electrodes for antenna-less and telemetry coil-less communication. The information encoded onto generated cardiac pacing pulses can include pacemaker state information, battery voltage, lead impedance, sensed cardiac signal amplitude, pacemaker current drain, programmed parameters, and the like.

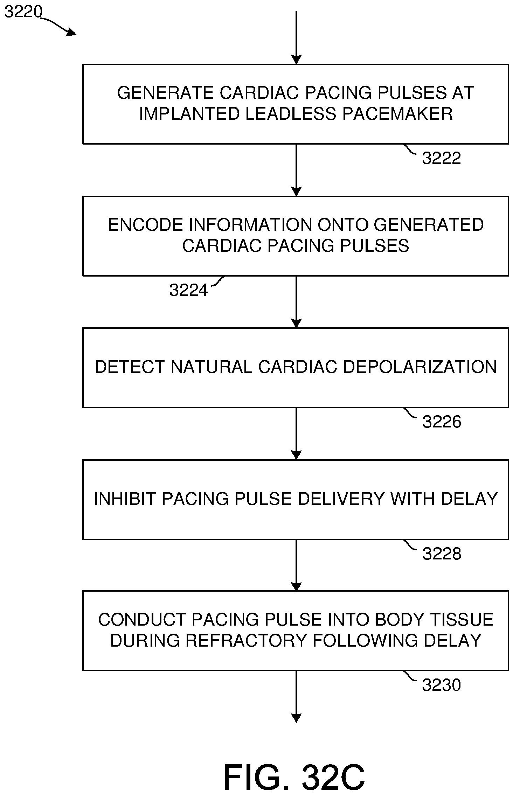

Referring to **FIGURE 32C****,** a method **3220** can further comprise generating **3222** cardiac pacing pulses at an implanted leadless cardiac pacemaker and encoding **3224** information onto generated cardiac pacing pulses at the implanted leadless cardiac pacemaker by selective alteration of pacing pulse morphology that is benign to therapeutic effect and energy cost of the pacing pulse. The implanted leadless cardiac pacemaker detects **3226** a natural cardiac depolarization and inhibits **3228** cardiac pacing pulse delivery with delay for delivery during a refractory period following the natural cardiac depolarization. The cardiac pacing pulses are conducted **3230** into body tissue via electrodes for antenna-less and telemetry coil-less communication.

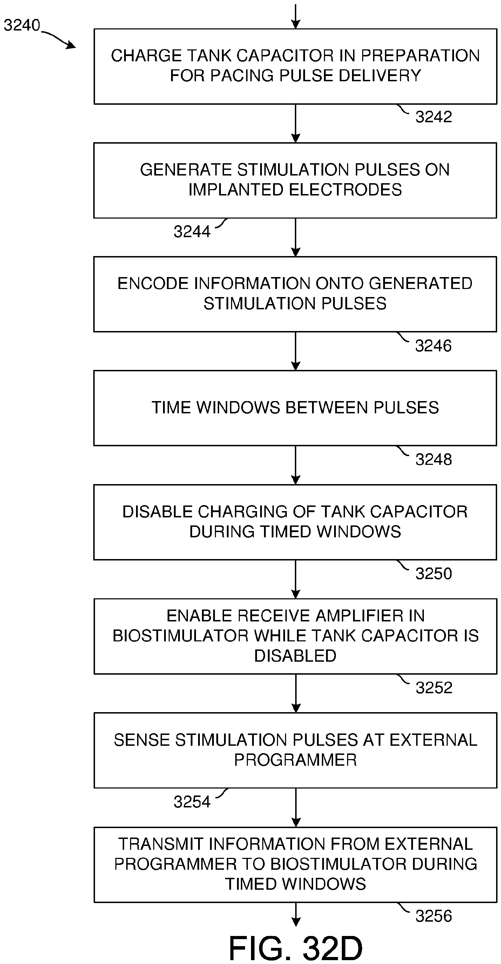

Referring to **FIGURE 32D****,** various embodiments of a method **3240** can comprise charging **3242** a tank capacitor in preparation for stimulation pulse generation. Stimulation pulses are generated **3244** on stimulating electrodes of an implanted biostimulator and information encoded **3246** onto generated stimulation pulses. One or more windows can be timed **3248** between pulse generations. Charging of the tank capacitor is disabled **3250** during the one or more timed windows and a receiving amplifier in the implanted biostimulator is enabled **3252** while the tank capacitor is disabled. The external programmer senses **3254** the stimulation pulses generated by the implanted biostimulator and transmits **3256** information from the external programmer to the implanted biostimulator to coincide with the one or more timed windows. For example, the external programmer can detect a stimulation pulse from the implanted biostimulator, time a selected delay interval, and transmit data after the selected delay to coincide with a window that the implanted biostimulator's receive amplifier is enabled.

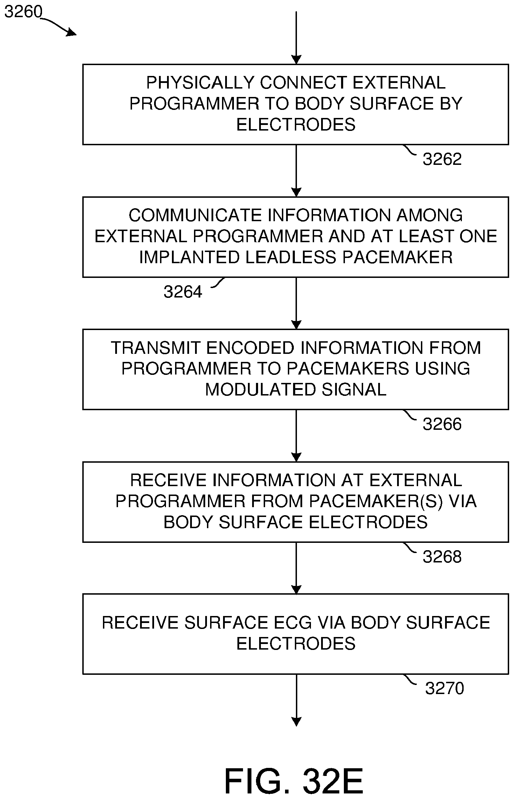

Referring to **FIGURE 32E****,** various embodiments of a method **3260** can comprise physically connecting **3262** the external programmer to a body surface via two or more body surface electrodes and communicating **3264** information among the external programmer and one or more implanted leadless cardiac pacemakers. Encoded information is transmitted **3266** from the external programmer to the implanted leadless cardiac pacemakers via the body surface electrodes using a modulated signal at a frequency in a range of approximately 10 kHz to approximately 100 kHz. The external programmer receives **3268** the information via the body surface electrodes from one or more of the implanted leadless cardiac pacemakers by detecting information encoded into generated pacing pulses. The external programmer can also receive **3270** a surface electrocardiogram via the body surface electrodes for display and analysis.

Terms "substantially", "essentially", or "approximately", that may be used herein, relate to an industry-accepted tolerance to the corresponding term. Such an industry-accepted tolerance ranges from less than one percent to twenty percent and corresponds to, but is not limited to, component values, integrated circuit process variations, temperature variations, rise and fall times, and/or thermal noise. The term "coupled", as may be used herein, includes direct coupling and indirect coupling via another component, element, circuit, or module where, for indirect coupling, the intervening component, element, circuit, or module does not modify the information of a signal but may adjust its current level, voltage level, and/or power level. Inferred coupling, for example where one element is coupled to another element by inference, includes direct and indirect coupling between two elements in the same manner as "coupled".

While the present disclosure describes various embodiments, these embodiments are to be understood as illustrative and do not limit the claim scope. Many variations, modifications, additions and improvements of the described embodiments are possible. For example, those having ordinary skill in the art will readily implement the steps necessary to provide the structures and methods disclosed herein, and will understand that the process parameters, materials, and dimensions are given by way of example only. The parameters, materials, and dimensions can be varied to achieve the desired structure as well as modifications, which are within the scope of the claims. Variations and modifications of the embodiments disclosed herein may also be made while remaining within the scope of the following claims. For example, although the description has some focus on the pacemaker, system, structures, and techniques can otherwise be applicable to other uses. Phraseology and terminology employed herein are for the purpose of the description and should not be regarded as limiting. With respect to the description, optimum dimensional relationships for the component parts are to include variations in size, materials, shape, form, function and manner of operation, assembly and use that are deemed readily apparent and obvious to one of ordinary skill in the art and all equivalent relationships to those illustrated in the drawings and described in the specification are intended to be encompassed by the present description. Therefore, the foregoing is considered as illustrative only of the principles of structure and operation. Numerous modifications and changes will readily occur to those of ordinary skill in the art whereby the scope is not limited to the exact construction and operation shown and described, and accordingly, all suitable modifications and equivalents may be included.

## Claims

1. A delivery system configured for implanting a biostimulation device comprising:
a stylet comprising a knob end and a threaded end configured to engage a threaded end of the biostimulation device; and
a sheath catheter configured to axially contain the stylet, the sheath catheter comprising a feature configured to engage a corresponding feature on the biostimulation device to provide rotational counter traction when the stylet is rotated relative to the sheath catheter for disengagement of the stylet threaded end from the biostimulation device threaded end, the sheath catheter being further configured to axially slide over the stylet and engaged biostimulation device and to axially retract from the stylet a predetermined distance whereby electrodes of the biostimulation device are exposed, enabling threshold testing while the biostimulation device remains engaged to the stylet.

2. The delivery system to claim 1 wherein the sheath cathether is configured to protect patient tissue from damage during insertion of the biostimulation device.

3. The delivery system of claim 1 wherein the sheath catheter comprises a feature that engages a corresponding feature on the biostimulation device whereby the stylet can be rotated relative to the sliding sheath for disengagement of the stylet threaded end from the biostimulation device threaded end.

4. The delivery system to claim 3 wherein the sheath catheter further comprises a feature configured to engage and rotate the biostimulation device and affix a fixation member coupled to the biostimulation device into patient tissue.

5. The delivery system of claim 3 wherein the sheath catheter engaging feature is configured to enable rotational counter traction for stylet disengagement.

6. The delivery system of claim 1 wherein the sheath catheter comprise a sheath slot that aligns with an alignment pin of the biostimulation device that prevents rotation of the biostimulation device with respect to the sheath catheter; and
the stylet extending along the axis from a knob coupled to a proximal end to a screw coupled to the distal end, the stylet configured whereby for the knob fully retracted the biostimulation device is fully contained within the sheath catheter and a fixation member coupled to the biostimulation device is protected, and whereby for the knob fully depressed the alignment pin is contained within the sheath catheter and electrodes ofthe biostimulation device are fully exposed, enabling threshold testing before disengagement.

7. The delivery system of claim 1 wherein the sheath catheter extends from a knob end to a socket end and encloses an internal lumen configured to axially slide over the stylet and engage the biostimulation device, the sheath catheter comprising a socket at the socket end that engages a corresponding nut on the biostimulation device whereby the stylet can be rotated relative to the sheath catheter for disengagement of the stylet threaded end from the biostimulation device threaded end.

8. The delivery system of claim 7 wherein the stylet screws into a multiple-sided nut attached to the biostimulation device; and
the socket is configured as a locking hex socket that engages the multiple-sided nut on the biostimulation device whereby the stylet and socket are configured to enable counter-rotation of the stylet relative to the biostimulation device.

9. The delivery system of claim 1 further comprising a biocompatible soluble protective covering that covers a fixation member coupled to the biostimulation device during insertion of the biostimulation device into a patient's body whereby the patient's body tissue is protected, the biocompatible soluble protective covering comprising a material that forms a protective capsule at room temperature, dissolves when implanted in a patient, and no toxic side effects.

10. The delivery system of claim 9 wherein the biocompatible soluble protective covering comprises mannitol, polyvinylpyrrolidone, or a protective salt.

11. The delivery system of claim 1 wherein the stylet and the sheath catheter comprise the delivery system with only two concentric members forming a catheter.
